# EUROPEAN PATENT APPLICATION

(11) **EP 4 745 575 A1**
(43) Date of publication of application: **20.05.2026**
(21) Application number: 24383240.9
(22) Date of filing: 14.11.2024
(51) Int. Cl.: G01N 33/569, G01N 33/68

(54) **LEVERAGING CIRCULATING ORAL MICROBIOME VESICLE-ASSOCIATED PROTEINS FOR THE DIAGNOSIS AND PROGNOSIS OF MIXED DEMENTIAS**

(71) Applicant: Institut de Recerca Biomèdica de Lleida Fundació Dr Pifarré, 25198 Lleida (ES); Universitat de Lleida, 25003 Lleida (ES)
(72) Inventor: SERRA MAQUEDA, Aida, 25003 Lleida (ES); GALLART PALAU, Xavier, 25198 Lleida (ES); MULET, María, 25003 Lleida (ES); SÁNCHEZ MILÁN, Jose Antonio, 25198 Lleida (ES)
(74) Representative: Hoffmann Eitle

(57) **Abstract**

The present invention refers to the use of an oral microbiome-derived extracellular vesicle associated protein (OMEVP) in the diagnosis or prognosis of mixed dementias in a biological sample obtained from a subject, wherein the OMEVP is the Chaperone protein DnaK or a fragment thereof. Furthermore, the present inventions refers to the method of diagnosis or prognosis of mixed dementia in a subject comprising assessing the level of an OMEVP in a biological sample obtained from a subject.

## Description

### Field of the invention

The present invention refers to the field of medicine. In particular, to neurology in the diagnosis and prognosis of mixed dementias in a subject.

### Background of the invention

The oral microbiome (OM) is formed by the billions of bacteria living in the mouth, and is also the second most diverse in the human body.

Oral microbiome is influenced by different aspects of lifestyle, is very dynamic and composed of several types of microorganisms, such as bacteria, fungi and archaea. Some of the microbes associated with the oral cavity have been found in very distant organs, showing an enormous capacity of interaction and communication within the body.

There is an emerging and consistent link between the OM and different health conditions, including mental health; Indeed, its proximity to the brain has led to growing interest in their relationship. Many research has been done in this regard since 1998 when reported signs of bacterial presence (*Chlamydia pneumoniae*) in the brain of patients with Alzheimer's disease (AD) for the first time. Since then, other possible agents of the OM have been found potentially implicated in the appearance and progression of neurodegenerative diseases, in particular AD (Sureda A, et al. "Oral microbiota and Alzheimer's disease: Do all roads lead to Rome?", Parmacol. Res, 2020, 151:104582. doi: 10.1016/j.phrs.2019.104582.)

Dementia is a syndrome involving cognitive decline in which abnormal protein deposition that typically coexists with neurovascular damage in different stages. Vascular diseases are highly involved in dementia too; In fact, vascular dementia (VaD) is responsible of approximately 15% of all diseases coursing with dementia worldwide. Mixed-etiology dementias, also known as mixed dementias, correspond to the condition in which more than one neuropathological signs of dementia coexist in Central Nervous System (CNS) tissues. The coexistence of VaD and AD signs is the most common form of mixed dementias in elders. VaD is generally also associated to deposition of senile plaque in the brain, in this case it is considered mixed dementias.

Mixed dementia is typically diagnosed using positron emission tomography (PET) of tracer molecules and an analysis of cerebrospinal fluid (CSF) proteins like tau or Aβ protein. Recently, blood and CSF biomarkers are also being used to assess health and disease status in elder patients. Magnetic resonance imaging can also be used for structural and functional analysis of patients because of the profound damage that dementia causes in the structure of the brain. Nonetheless, palliative care to alleviate symptoms are usually the only way for treating the related diseases, since the neuropathological signs related to the apparition and progression of mixed dementias start almost a decade earlier than the symptoms and makes it difficult for an early detection.

Besides the use of classical protein biomarkers such as tau or Aβ, other human proteins have been pointed out as potential biomarkers for neurodegenerative diseases that cause dementia. The application CN 1258748 A describes the Thioredoxin system (Trx system) and Thioredoxin reductase (TR) of human origin, proposing that the TR can be used in the treatment and diagnosis of neurodegenerative diseases. No mention is made to a bacterial TR of external origin. It is important to notice that, although proteins of the thioredoxin system are ubiquitous from Archaea to man and share functionality, they are different molecules in their amino acid sequence and structure. In bacteria, fungi and plants, TR is a specific dimeric 70-kDa flavoprotein with a redox active site disulfide/dithiol; In contrast, thioredoxin reductases of higher eukaryotes are 112-130 kDa selenium-dependent dimeric flavoproteins. It cannot be settled a *priori* that a bacterial protein remains functional in a different environment as the human body and, in any case, could be foreseen as an eventual biomarker of neurodegenerative diseases in a human.

Similarly, KR 20120041823 A describes a list of protein biomarkers present in blood for early diagnosis of AD. Among others, they protect the human protein ATP synthase beta subunit. Nonetheless, the publication again refers only to the human protein omitting suggesting a similar function for the bacterial protein associated its presence within the CNS to extracellular vesicles.

On the other side, specific bacteria have also been associated to neurodegererative diseases. Dominy gave evidence of that *Porphyromonas gingivalis* and gingipains, which are trypsin-like cysteine proteinases produced by *P. gingivalis,* in the brain play a role in the pathogenesis of AD (Dominy, S. et al., "Porphyromonas gingivalis in Alzheimer's disease brains: Evidence for disease causation and treatment with small-molecule inhibitors", 2019, Jan 23;5(1):eaau3333. doi: 10.1126/sciadv.aau3333.) The authors demonstrate the presence of P. *gingivalis* DNA and gingipain antigens in AD brains and propose a treatment consisting of the administration of small-molecule gingipain inhibitors that blocks gingipain-induced neurodegeneration, reduces P. *gingivalis* load in the mouse brain, and decreases the host Aβ1-42 response to P. *gingivalis* brain infection. However, the presence of these molecules of bacterial origin linked to any mechanism of nanotransportation has not been contemplated in this study, neither has been studied in mixed dementias.

*Anti-Chlamydia pneumoniae* antibodies have managed to detect by inmunohistochemistry analysis both typical intracellular and atypical extracellular C. *pneumoniae* antigens in frontal and temporal cortices of the AD brain (Hammond et al. "Immunohistological detection of Chlamydia pneumoniae in the Alzheimer's disease brain", 2010, Sep 23;11:121. doi: 10.1186/1471-2202-11-121). This document finds evidence of that a *C*. *pneumoniae* infection is present in brain tissues in areas of amyloid pathology, thereby suggesting an interrelationship between these entities in the pathogenesis of sporadic late-onset AD. No mention or suggestion is made, however, to a possible mechanism for that, omitting referring to proteins eventually secreted by the microorganism, any mechanism that may help these to penetrate the blood brain barrier, and their eventual effect or abilities as biomarker of the disease, much less to Elongation-factor Tu.

OM disbiosis, refering to an inbalance of the microbial composition, is associated to different oral cavity diseases including mild and serious gum diseases such as gingivitis and periodontitis. In a related manner, gingivitis and periodontitis, both involving the damage of the tissue that supports the teeth, have been associated epidemiologically with apparition of age-related dementia (Guo H, Chang S, Pi X, Hua F, Jiang H, Liu C, Du M. The Effect of Periodontitis on Dementia and Cognitive Impairment: A Meta-Analysis. Int J Environ Res Public Health. 2021 Jun 25;18(13):6823. doi: 10.3390/ijerph18136823.) The present meta-analysis thoroughly examines the current body of literature and suggests a potential link between periodontitis and dementia. However, the nature of this relationship remains uncertain, as there is limited evidence to support any known molecular mechanism that could sustain it. Furthermore, no specific molecular indicator has been identified to establish a concrete connection between periodontitis and dementia as detailed in this recent meta-analysis study.

About the way of travelling of the proteins to the brain, in recent years it has been demonstrated that extracellular vesicles (EVs) display capacity to mediate an intercellular communication mechanism and are potentially implicated in the development of mutiple diseases including dementia.

EVs are small vesicles (100-2000 nm diameter) composed of a lipid bilayer containing transmembrane proteins and enclosing myriad of proteins, small molecules and RNA. EVs are ubiquitous, as they are secreted by cells of muticellular organisms and by unicellular organisms including bacteria, proteists and yeast. Once EVs reach their target cell, they are able to either induce a signal by receptor-ligand interaction or they are phagocytosed releasing their contents into the recipient cell. They are not only involved in simple communication, as it has been proposed that these EVs have both a pathogenic function, when transporting some pathogens such as beta-amyloid protein or phosphorylated tau, as well as a neuroprotective function in AD. EVs, by its composition, are able to circulate through the body in humans, being present in biological fluids and products such as blood, urine, saliva, tear fluid, cerebrospinal fluid, semen, uterine and menstrual fluids, mucus and gingival plaque. Interestingly, brain-derived EVs are also able to cross the blood brain barrier in a bi-directional manner and therefore can be detected by skilled investigators and clinicians in biological fluids (Hornung S, Dutta S, Bitan G. CNS-Derived Blood Exosomes as a Promising Source of Biomarkers: Opportunities and Challenges. Front Mol Neurosci. 2020 Mar 19;13:38. doi: 10.3389/fnmol.2020.00038.). Although it is widely known that EVs produced by the cells in the human body are a good source of circulating biomarkers for human diseases, it has not been conceived that EVs can also be produced by the microbiomes in our systems as holobionts, and that these microorganisms also influence the molecular compositions of the circulating extracellular vesicles that circulate and show ability cross the blood brain barrier as we specifically indicate in this invention.

The art has extensively taught about EVs carrying proteins that revealed useful for the diagnosis of certain diseases that cause age-associated dementia (e.g. AD) and has proposed several ways of treatment, although to the best of the knowledge of the inventors the art has never described EVs derived from the OM, identified in CNS tissues and related to the diagnosis or treatment of mixed dementias.

WO 2022228516 A1 discloses a nasal drop preparation containing engineered cell-derived vesicles for the treatment of AD, since stem cell-derived EVs contain miRNAs that are related to tissue repair and anti-inflammatory effects. The ingredients of the preparation penetrate the blood barrier and would be taken up by the microglia and nerve cells. No any mention is made about OM or suggestion to the content that oral-microbiome EVs may transport molecules to and from the CNS and to any potential effect of these.

US 2021277443 A describes the detection of DNA of bacterial origin in EVs extracted from the blood of patients as a method for diagnosing AD, and establishes cut-off values of phenoconversion for the different grades of evolution of the disease. The document describes the detected bacteria showing different levels between healthy and diseased subjects. No mention or suggestion is made to EVs containig proteins that reach the brain any potential role in the evolution of AD, much less to the OM producing these proteins and any effects of these in mixed dementias.

The need to be solved in the art can be formulated as a requirement to obtain alternative molecular agents able to assist on the prognosis, diagnosis of mixed dementias. The solution proposed by the present invention is the detection and potential interventions on OM-derived bacterial proteins associated with blood and brain EVs, that are able to circulate in biological fluids and that can be isolated from human biological samples.

### Brief description of the invention

The involvement of the OM (OM) in the physiopathology of Alzheimer's disease (AD) and vascular dementia (VaD) has been epidemiologically recognized, but the identification of feasible molecular pathways remains elusive. In the present invention, unbiased meta-proteomics verified the presence of OM-derived proteins (OMdP) in brain extracellular vesicles (bEVs) from post-mortem AD and VaD subjects. OMdP circulation in blood EVs was verified in prodromal and clinical AD. Advanced bioinformatics explored interactions between OMdP and dementia-related proteins. Peptidome-wide correlations analyzed the exchange abilities and composition(s) of OMdP in bEVs. The obtained data reveals that specific OMdP are present in bEVs, with their levels and presence fluctuating in tune with the clinical progression of AD and VaD. OMdP in bEVs were also found to transfer to CNS tissues, to differentially circulate in blood plasma, and to interact with key dementia-related proteins in the brain.

The molecular involvement of the OM in the physiopathology of dementia is thus here revealed through its association with circulating EVs, which opens new promising avenues for clinical diagnostics, prognostics, and therapeutics.

### Description of the invention

### Definitions

The inventors have found for the first time EVs carrying bacterial originated proteins from the OM, dubbed as OM-derived EV associated proteins (OMEVP), that may play a role in the pathogenesis of mixed dementias and be useful in the diagnosis and prognosis of the disease. This means identifying a possible source of disease development agents at the molecular levelable to cross the blood brain barrier and of transferring proteins to CNS cells.

In the context of the present invention as it has previously disclosed in the present document are the EVs which are small vesicles with a 100-2000 nm diameter composed of a lipid bilayer containing transmembrane proteins and enclosing a myriad of proteins, small molecules and RNA. EVs are ubiquitous, as they are secreted by cells of muticellular organisms and by unicellular organisms including bacteria, proteists and yeast.

In the present invention, the term mixed dementias designates a disease that causes progressive age-related dementia, a syndrome that impairs the cognitive and motor skills of the person to perform essential daily tasks, and that is product of brain damage originated not by a single predominant neuropathological sign. In a preferred embodiment of the invention the mixed dementias are selected from the group consisting of proteinopathy, senile plaque, neurofibrilaly tangles, microinfarcts, leukaraiosis, AD and a combination thereof. These mixed dementias are AD neuropathology combined with any kind of brain hypoperfusion processes, being more preferably VaD signs and neurovascular unit impairment at biochemical and/or physical levels.

The term "AD subjects" is referred for those subjects that suffers from AD at any of the stages of the disease.

The term "VaD subjects" is referred for those subjects that suffers from VaD at any stage of the disease.

In the present invention, "phenoconversion of mixed dementia " is defined as signs of a disease related to VaD and proteinopathy characteristic in the CNS of aging-associated cortical dementias.

The term "microbiome" is defined as an ecological community of commensal, symbiotic and/or pathogenic microorganisms found in and on all multicellular organisms from plants to animals. The associated microbiota includes bacteria, protists, fungi and viruses.

The term "oral microbiome" refers to the microbiota present in the oral cavity of a subject.

In the present invention, the term "diagnosis of mixed dementia" refers to the detection of mixed dementia in a subject that shows at least two symptoms of this disease.

The term "prognosis of mixed dementia" refers to the likelihood or expected development of mixed dementia, including whether the signs and symptoms will improve or worsen and how quickly or remain stable over time in a subject.

The terms "protein-aggregation disease", "proteopathy", "proteinopathy" or "protein misfolding diseases" refers to any disease in which certain proteins become structurally abnormal and thereby disrupt the function of cells, tissues and organs of the body. Often the proteins fail to fold into their normal configuration; in this misfolded state, the proteins can become toxic in some way or they can lose their normal function. Non-limiting examples of protein-aggregation diseases include systemic AL amyloidosis, AD, Diabetes mellitus type 2, Parkinson's disease, Transmissible spongiform encephalopathy e.g. Bovine spongiform encephalopathy, Fatal Familial Insomnia, Huntington's Disease, Medullary carcinoma of the thyroid, Cardiac arrhythmias, Atherosclerosis, Rheumatoid arthritis, Aortic medial amyloid, Prolactinomas, Familial amyloid polyneuropathy, Hereditary non-neuropathic systemic amyloidosis, Dialysis related amyloidosis, Finnish amyloidosis, Lattice corneal dystrophy, Cerebral amyloid angiopathy, Cerebral amyloid angiopathy (Icelandic type), Sporadic Inclusion Body Myositis, Amyotrophic lateral sclerosis, Prion-related or Spongiform encephalopathies, such as Creutzfeld-Jacob, Dementia with Lewy bodies, Frontotemporal dementia with Parkinsonism, Spinocerebellar ataxias, Spinocerebellar ataxia, Spinal and bulbar muscular atrophy, Hereditary dentatorubral-pallidoluysian atrophy, Familial British dementia, Familial Danish dementia, Non- neuropathic localized diseases, such as in Type II diabetes mellitus, Medullary carcinoma of the thyroid, Atrial amyloidosis, Hereditary cerebral haemorrhage with amyloidosis, Pituitary prolactinoma, Injection-localized amyloidosis, Aortic medial amyloidosis, Hereditary lattice corneal dystrophy, Corneal amyloidosis associated with trichiasis, Cataract, Calcifying epithelial odontogenic tumors, Pulmonary alveolar proteinosis, Inclusion-body myositis, Cutaneous lichen amyloidosis, and Non-neuropathic systemic amyloidosis, such as AL amyloidosis, AA amyloidosis, Familial Mediterranean fever, Senile systemic amyloidosis, Familial amyloidotic polyneuropathy, Hemodialysis-related amyloidosis, ApoAl amyloidosis, ApoAII amyloidosis, ApoAIV amyloidosis, Finnish hereditary amyloidosis, Lysozyme amyloidosis, Fibrinogen amyloidosis, Icelandic hereditary cerebral amyloid angiopathy, familial amyloidosis, and systemic amyloidosis which occurs in multiple tissues, such as light-chain amyloidosis, and other various neurodegenerative disorders.

### DESCRIPTION OF EMBODIMENTS

In recent years, there has been a heightened interest in investigating the impact of the microbiome on human health, and alterations of the gut and OM have been specifically associated with neuropathological hallmarks. Nonetheless, it is crucial to deepen our comprehension of the potential role(s) of these microorganisms and their products in neurological diseases, particularly in relation to dementia-like conditions such as AD. This invention aims to provide new insights into the multifactorial understanding of AD by presenting, to the best of our knowledge, the findings on the means by which the products of certain OM-linked microorganisms reach the brain and their potential involvement in the pathophysiology of the disease.

Numerous research groups have previously reported the existence of poorer oral health and diseases such as periodontitis linked to the epidemiological emergence and progression of AD. Our findings indicate that OMdP possess the ability to permeate brain tissues, engage with bEVs, and be detectable within brain parenchyma fractions that do not include bEVs in all of the individuals analyzed. The concentrations and presence of these bacterial proteins, though, in bEVs, experience particular transformations that are associated with the clinical development of both the most prevalent age-related dementias, namely AD and VaD. Furthermore, it appears that the changes observed in bEVs may parallel certain disruptions in the OM, which seem to be prevalent in individuals with dementia.

In the course of this invention, particular attention was paid to the distinct and discriminatory sensitivities of OMdP in bEVs levels across the diverse phases of AD and VaD that were assessed, which allowed for the classification of OMdP in bEVs into four distinct categories. These categories include OMdP in bEVs associated with AD, such as AHCY (adenosylhomocysteinase) and acnA (aconitate hydratase A), which are related to the disease with clinical symptoms; OMdP in bEVs related to VaD, such as ATPD (ATP synthase subunit beta), DnaK1, DnaK4, Tuf (elongation factor), and trxA ((nerve growth factor-like); OMdP in bEVs associated with early AD, such as DnaK3, SDR (short-chain dehydrogenase), Tuf, trxA (nerve growth factor-like), and others; and OMdP in bEVs related to advanced AD, such as DnaK1-3, SDR, PstS (Phosphate ABC transporter substrate-binding protein), FHS (formate--tetrahydrofolate ligase), and ACAD (dehydrogenase). In the context of the specific ecosystem where the microorganisms capable of producing the identified disease-specific modifications in this invention reside, our research demonstrates that a greater proportion of these microorganisms originate from the OM. Additionally, our findings suggest that individuals diagnosed with dementia exhibit a higher concentration of OM-derived pathogenic and commensal microorganisms.

Furthermore, our findings from a clinical cohort of subjects with MCI and AD indicate that OMdP in bEVs not only have the ability to circulate in blood plasma but also to distinguish between clinical groups affected by cognitive decline and dementias.

The first aspect of the present invention is a use of an OMEVP in the diagnosis or prognosis of proteotoxicity in a protein-aggregation disease in a biological sample obtained from a subject. Preferably the present invention refers to the use of an OMEVP in the diagnosis or prognosis of vascular mixed dementias in a biological sample obtained from a subject, preferably in the diagnosis or prognosis of AD or VaD.

These OMEVP have been found, by the skilled inventors, associated to brain EVs in mixed dementias. Therefore, in the present document it is described the use of these proteins associated to brain EVs in the diagnosis and/or prognosis of mixed dementias. To the best knowledge of the inventors, there are no previous descriptions of OMEVP linked to the apparition and progression of this age dementia-linked disease in humans.

In a preferred embodiment, the OMEVP is selected from the group consisting of Chaperone protein DnaK, Adenosylhomocysteinase, 2-keto-3-deoxy-L-fuconate dehydrogenase, Formate-tetrahydrofolate ligase, Thioredoxin 1, Phosphate ABC transporter substrate-binding protein (from hereinafter Phosphate-binding protein), 2-methylcitrate dehydratase, Acyl-CoA dehydrogenase, Elongation factor Tu, and ATP synthase subunit beta and combinations thereof.

Another aspect of the invention is the use of these OMEVP considered in groups of two or more. In fact, some combinations have revealed increase sensitivity as biomarkers for neurovascular specific dysfunction in mixed dementias.

Another aspect of the invention relates to the *in vitro* use of a combination of the OMEVPs Adenosylhomocysteinase, Formate-tetrahydrofolate ligase and Thioredoxin 1, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK, 2-keto-3-deoxy-L-fuconate dehydrogenase and Formate-tetrahydrofolate ligasein, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK, Adenosylhomocysteinase and Formate-tetrahydrofolate ligase, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK and 2-keto-3-deoxy-L-fuconate dehydrogenase, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs 2-methylcitrate dehydratase and Acyl-CoA dehydrogenase in the prognosis or diagnosis of, or in the determination of a risk of suffering from, mixed dementia, preferably in the diagnosis or prognosis of, or in determination of a risk of suffereing from, AD or VaD.

Another aspect of the invention is the use of OMEVP is a combination of 2-methylcitrate dehydratase and Phosphate-binding protein, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK and Adenosylhomocysteinase, in the prognosis or diagnosis of, or in the determination of a risk of suffering from, mixed dementia, preferably in the diagnosis or prognosis of, or in determination of a risk of suffereing from, AD or VaD. Another aspect of the invention relates to the *in vitro* use of a combination of the OMEVPs Adenosylhomocysteinase and Elongation factor Tu, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK and ATP synthase subunit beta, in the prognosis or diagnosis of, or in the determination of a risk of suffering from, mixed dementia, preferably in the diagnosis or prognosis of, or in determination of a risk of suffereing from, AD or VaD.

More preferably, the OMEVP is selected from the group consisting of:
a) Chaperone protein DnaK of SEQ ID NO 1 to 23 or any fragments thereof, in particular fragments of SEQ ID NO 51 to 142 or fragments having at least 90% sequence identity with any one of SEQ ID NO 51 to 142, or a sequence having at least 90% identity with any one of SEQ ID NO 1 to 23,
b) Adenosylhomocysteinase of SEQ ID NO 24 to 28 or any fragments thereof, in particular fragments of SEQ ID NO 143 to 158 or fragments having at least 90% sequence identity with any one of SEQ ID NO 143 to 158, or a sequence having at least 90% identity with any one of SEQ ID NO 24 to 28,
c) 2-keto-3-deoxy-L-fuconate dehydrogenase of SEQ ID NO 29 to 31 or any fragments thereof, in particular fragments of SEQ ID NO 159 to 161 or fragments having at least 90% sequence identity with any one of SEQ ID NO 159 to 161, or a sequence having at least 90% identity with any one of SEQ ID NO 29 to 31,
d) Formate-tetrahydrofolate ligase of SEQ ID NO 32 or any fragments thereof, in particular fragments of SEQ ID NO 162 to 163 or fragments having at least 90% sequence identity with any one of SEQ ID NO 162 to 163, or a sequence having at least 90% identity with any one of SEQ ID NO 32,
e) Thioredoxin 1 of SEQ ID NO 33 to 39 or any fragments thereof, in particular fragments of SEQ ID NO 164 to 170 or fragments having at least 90% sequence identity with any one of SEQ ID NO 164 to 170, or a sequence having at least 90% identity with any one of SEQ ID NO 33 to 39,
f) Phosphate ABC transporter substrate-binding protein PstS of SEQ ID NO 49 or any fragments thereof, in particular fragments of SEQ ID NO 180 or fragments having at least 90% sequence identity with SEQ ID NO 180, or a sequence having at least 90% identity with SEQ ID NO 49,
g) 2-methylcitrate dehydratase of SEQ ID NO 40 or any fragments thereof, in particular fragments of SEQ ID NO 171 or fragments having at least 90% sequence identity with SEQ ID NO 171, or a sequence having at least 90% identity with SEQ ID NO 40,
h) Acyl-CoA dehydrogenase of SEQ ID NO 41 or any fragments thereof, in particular fragments of SEQ ID NO 172 or fragments having at least 90% sequence identity with SEQ ID NO 172, or a sequence having at least 90% identity with SEQ ID NO 41,
i) Elongation factor Tu of SEQ ID NO 42 to 48 or any fragments thereof, in particular fragments of SEQ ID NO 173 to 179 or fragments having at least 90% sequence identity with any one of SEQ ID NO 173 to 179, or a sequence having at least 90% identity with any one of SEQ ID NO 42 to 48,
j) ATP synthase subunit betaof SEQ ID NO 50 or any fragments thereof, in particular fragments of SEQ ID NO 181 to 186 or fragments having at least 90% sequence identity with any one of SEQ ID NO 181 to 186, or a sequence having at least 90% identity with SEQ ID NO 50, or any combinations thereof.

Still more preferably, the OMEVP is selected from the group consisting of fragments of the Chaperone protein DnaK of SEQ ID NO 51 to 142, or a sequence having at least 90% identity with any one of SEQ ID NO 51 to 142, fragments of the Adenosylhomocysteinase of SEQ ID NO 143 to 158, or a sequence having at least 90% identity with any one of SEQ ID NO 143 to 158, fragments of the 2-keto-3-deoxy-L-fuconate dehydrogenase of SEQ ID NO 159 to 161, or a sequence having at least 90% identity with any one of SEQ ID NO 159 to 161, fragments of the Formate-tetrahydrofolate ligase of SEQ ID NO 162 to 163, or a sequence having at least 90% identity with any one of SEQ ID NO 162 to 163, fragments of the Thioredoxin 1 of SEQ ID NO 164 to 170, or a sequence having at least 90% identity with any one of SEQ ID NO 164 to 170, fragments of the Phosphate-binding protein of SEQ ID NO 180, or a sequence having at least 90% identity with SEQ ID NO 180, fragments of the 2-methylcitrate dehydratase of SEQ ID NO 171, or a sequence having at least 90% identity with SEQ ID NO 171, fragments of the Acyl-CoA dehydrogenase of SEQ ID NO 172, or a sequence having at least 90% identity with SEQ ID NO 172, fragments of the Elongation factor Tu of SEQ ID NO 173 to 179, or a sequence having at least 90% identity with any one of SEQ ID NO 173 to 179 and fragments of the ATP synthase subunit beta of SEQ ID NO 181 to 186, or a sequence having at least 90% identity with any one of SEQ ID NO 181 to 186, or any combinations thereof.

Another aspect of the invention relates to the *in vitro* use of a combination of the OMEVPs Adenosylhomocysteinase of SEQ ID NO 24 to 28 or any fragments thereof as defined above for this protein, or a sequence having at least 90% identity with any one of SEQ ID NO 24 to 28, Formate-tetrahydrofolate ligase of SEQ ID NO 32 or any fragments thereof as defined above for this protein, or a sequence having at least 90% identity with SEQ ID NO 32, and Thioredoxin 1 of SEQ ID NO 33 to 39 or any fragments thereof as defined above for this protein, or a sequence having at least 90% identity with any one of SEQ ID NO 33 to 39, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK of SEQ ID NO 1 to 23 or any fragments thereof as defined above for this protein, or a sequence having at least 90% identity with any one of SEQ ID NO 1 to 23, 2-keto-3-deoxy-L-fuconate dehydrogenase of SEQ ID NO 29 to 31 or any fragments thereof as defined above for this protein, or a sequence having at least 90% identity with any one of SEQ ID NO 29 to 31 and Formate-tetrahydrofolate ligasein of SEQ ID NO 32 or any fragments thereof as defined above for this protein, or a sequence having at least 90% identity with SEQ ID NO 32, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK of SEQ ID NO 1 to 23 or any fragments thereof as defined above for this protein, or a sequence having at least 90% identity with any one of SEQ ID NO 1 to 23, Adenosylhomocysteinase of SEQ ID NO 24 to 28 or any fragments thereof as defined above for this protein, or a sequence having at least 90% identity with any one of SEQ ID NO 24 to 28, and Formate-tetrahydrofolate ligase of SEQ ID NO 32 or any fragments thereof as defined above for this protein, or a sequence having at least 90% identity with SEQ ID NO 32, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK of SEQ ID NO 1 to 23 or any fragments thereof as defined above for this protein, or a sequence having at least 90% identity with any one of SEQ ID NO 1 to 23, and 2-keto-3-deoxy-L-fuconate dehydrogenasein of SEQ ID NO 29 to 31 or any fragments thereof as defined above for this protein, or a sequence having at least 90% identity with any one of SEQ ID NO 29 to 31, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs 2-methylcitrate dehydratase of SEQ ID NO 40 or any fragments thereof as defined above for this protein, or a sequence having at least 90% identity with SEQ ID NO 40, and Acyl-CoA dehydrogenase. of SEQ ID NO 41 or any fragments thereof as defined above for this protein, or a sequence having at least 90% identity with SEQ ID NO 41, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs 2-methylcitrate dehydratase of SEQ ID NO 40 or any fragments thereof as defined above for this protein, or a sequence having at least 90% identity with SEQ ID NO 40, and Phosphate-binding protein of SEQ ID NO 49 or any fragments thereof as defined above for this protein, or a sequence having at least 90% identity with SEQ ID NO 49, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK of SEQ ID NO 1 to 23 or any fragments thereof as defined above for this protein, or a sequence having at least 90% identity with any one of SEQ ID NO 1 to 23 and Adenosylhomocysteinasein of SEQ ID NO 24 to 28 or any fragments thereof as defined above for this protein, or a sequence having at least 90% identity with any one of SEQ ID NO 24 to 28, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Adenosylhomocysteinase of SEQ ID NO 24 to 28 or any fragments thereof as defined above for this protein, or a sequence having at least 90% identity with any one of SEQ ID NO 24 to 28, and Elongation factor Tu of SEQ ID NO 42 to 48, or any fragments thereof as defined above for this protein, or a sequence having at least 90% identity with any one of SEQ ID NO 42 to 48, in the prognosis or diagnosis of, or in the determination of a risk of suffering from, mixed dementia, preferably in the diagnosis or prognosis of, or in determination of a risk of suffereing from, AD or VaD.

Another aspect of the invention relates to the *in vitro* use of a combination of the OMEVPs Chaperone protein DnaK of SEQ ID NO 1 to 23 or any fragments thereof as defined above for this protein, or a sequence having at least 90% identity with any one of SEQ ID NO 1 to 23, and ATP synthase subunit beta of SEQ ID NO 50 or any fragments thereof as defined above for this protein, or a sequence having at least 90% identity with SEQ ID NO 50, in the prognosis or diagnosis of, or in the determination of a risk of suffering from, mixed dementia, preferably in the diagnosis or prognosis of, or in determination of a risk of suffereing from, AD or VaD.

Therefore, the OMEVP can be detected in a variety of biological samples, being preferred a human-originated sample. So, in a preferred aspect of the present invention the biological sample is selected from saliva, mucus, gum plaque, blood, plasma, serum, urine, tear liquid, cerebrospinal fluid or brain tissues. In a particular embodiment of the invention, the brain tissues are currently obtained from dementia patients predominantly from post-mortem origin.

In a preferred embodiment, the sample wherein OMEVP are detected is brain tissue. In another preferred embodiment, the sample wherein OMEVP are detected is saliva. In another preferred embodiment, the sample wherein OMEVP are detected is blood. In another preferred embodiment, the sample wherein OMEVP are detected is plasma. In another preferred embodiment, the sample wherein OMEVP are detected is serum. In another preferred embodiment, the sample wherein OMEVP are detected is cerebroespinal fluid.

The present invention shows the presence of proteins from certain microorganisms of the oral microbiota, which arrive via extracellular vesicles to the brain of subjects with mixed dementias, finding variation in their levels throughout the disease progression. Therefore, specific proteins are up- or down-regulated in preclinical stage of the disease. Another subset of OMEVP are singularly modulated alongside the evolutionary course of the disease. Additionally, a third subset of OMEVP seem able to qualitatively sense impairment of the neurovascular unit in the disease.

The referred OMEVP show ability to interact with human proteins directly implicated in the neuropathology of mixed dementias. The table below shows the OMEVP, the microorganisms that can synthesize these proteins and the human proteins found in the examples with significant chance of interaction with the referred OMEVP.

**Table 1**

| **OMEVP** | **Microorganism strain** | | **Human Proteins (GS)** |
|---|---|---|---|
| | *A. actinomycetecomitans* | *L. plantarum* | TAU |
| | *A. aphrophilus* | *L. rimae* | APP |
| | *A. massiliensis* | *L.lactis* | PrP |
| | *Actinomyces sp.* | M. *neoaurum* | PSEN1 |
| | *Aggregatibacter sp. HMT 458* | P. *denticolens* | APOE |
| **Chaperone protein DnaK** | *C. coyleae* | *P. mirabilis* | Abeta |
| | *C. sputorum* s | *S. cristatus* | ATXN1 |
| | *C. ureolyticus* | *S. inopinata* | HTT |
| | *E. faecalis* | S. *intermedius* | MAP1LC3B |
| | *H. missouriensis* | S. *thermophilu* | PRKN |
| | *H. parainfluenzae* | *S. wiggsiae* | LRRK2 |
| | *H. pittmaniae* | | |
| | *M. aeruginosavorus* | | TAU |
| | *S. nitritireducens* | | APP |
| | *T. forsythia* | | PrP |
| | *T. sp. oral taxon 286* | | PSEN1 |
| **Adenosylhomocysteinas e** | *T. sp. oral taxon 808* | | ANKRD40 |
| | | | APPBP2 |
| | | | MAP1LC3B |
| | | | PRKN |
| | | | LRRK2 |
| | | | YWHAZ |
| **2-keto-3-deoxy-L-fuconate dehydrogenase** | *K. aerogenes* | | TAU |
| | *P.fluorescens* | | APP |
| | | | PrP |
| | | | PSEN1 |
| | | | Abeta42 |
| | | | RAB27B |
| **Formate-tetrahydrofolate ligase** | C. *granulosum* | | TAU |
| | | | APP |
| | | | PrP |
| | | | PSEN1 |
| | | | LRRK2 |
| **Thioredoxin 1** | *E. coli* | | TAU |
| | *C. sakazakii* | | APP |
| | *E. cancerogenus* | | PrP |
| | *E. hormaechei* | | PSEN1 |
| | *K. aerogenes* | | JPH3 |
| | *K. ascorbata* | | LRP8 |
| | *K. pneumoniae* | | DNM2 |
| | | | A2M |
| | | | BDNF |
| | | | Abeta42 |
| | | | MAP1LC3B |
| | | | YWHAZ |
| | | | PRKN |
| **2-methylcitrate dehydratase** | *P. dentalis* | | TAU |
| | | | APP |
| | | | PrP |
| | | | PSEN1 |
| | | | LRRK2 |
| | | | FBXL5 |
| | *R. picketti* | | TAU |
| | | | APP |
| | | | PrP |
| | | | PSEN1 |
| **Acyl-CoA dehydrogenase** | | | STX5 |
| | | | IRF7 |
| | | | KIF6 |
| | | | SEC22C |
| | | | RING1 |
| **Phosphate-binding protein** | *R. rhizogenes* | | TAU |
| | | | APP |
| | | | PrP |
| | | | PSEN1 |
| | | | PON1 |
| | *P. aeruginosa* | | TAU |
| | *A. propionica* | | APP |
| | *Arsenicicoccus sp. HMT 190* | | PrP |
| | *C. avidum* | | PSEN1 |
| **Elongation factor Tu** | *C. pneumoniae* | | CMTM5 |
| | *J. indicus* | | NLRX1 |
| | *K. sedentarius* | | FUNDC1 |
| | | | MAP1LC3B |
| | | | PRKN |
| | | | LRRK2 |
| **ATP synthase subunit beta** | *R. capsulatus* | | TAU |
| | | | APP |
| | | | PrP |
| | | | PSEN1 |
| | | | ATXN1 |
| | | | TTC5 |
| | | | LNX2 |
| | | | YWHAZ |
| | | | ATPAF2 |
| | | | BCL2L1 |
| | | | PPIF |
| | | | Abeta42 |
| | | | MAP1LC3B |

Table 1. Description of the OMEVP, the microorganism that produces said proteins and which human proteins OMEVP interact with.

More particularly, the table below shows each of the OMEVP of the present invention, the microorganism that produces said protein, the sequence of the protein and the peptides of each protein useful to carry out the present invention.

**Table 2**

| **Proteins** | **Microor ganisms** | **Sequence** | **Peptide** | s t a r t | E n d |
|---|---|---|---|---|---|
| Chapero ne protein DnaK | *Actinom yces massilie nsis* | (SEQ ID NO 1) MARAVGIDLGTTNSAIAVLEGGEPTIIPNAEGGRTTPSVVAFSKSGEVLVGEVAKRQAVTNVDRTISSVKRHMGTDWSVEIDGKKYTAQEISARILAKLKTDAESYLGESVTN AVITVPAYFNDAERQATKEAGTIAGLTVDRIVNEPTAAALAYGLDKGKEDELILVFDLGGGTFDVSLLEVGKDDDGFSTIQVRATNGDNRLGGDDWDARIVDWLVKQVKD KNGVDLSKDKIAMQRLKDAAEQAKKELSSATSTNINLQYLSMSEAGPIHLDEKLTRSHFEEMTADLLERTKVPFHNVIKDAGVKVSDIDHVILVGGSTRMPAVSEVVRELTG KEPNKGVNPDEVVAIGAALQAGVIQGDRKDVLLIDVTPLSLGIETKGGVMTKLIERNTAIPTKRSEVFSTAEDNQSSVLIQVYQGEREFARDNKPLGTFELTGIAPAPRGIPQI EVSFDIDANGIVHVSAKDRGTGKEQSMTISGGSALPKEDIDRMVKEAEAHAEEDKKRREDAETRNSAEQQAYSVEKLLKDNKDKLPESVHSEVSGAVDGLKKALEGEDIEP VKTAQEKLNEVAQKIGEALYQHEQAEQQTQQAQSSAGASSEASAEDDDIVDAEIIDDEDSK | (SEQ ID NO 51) RIVNEPTAAALA YGLDKG | 1 4 3 | 1 6 0 |
| | *Actinom yces sp. HMT 897* | (SEQ ID NO 2) MPKAVGIDLGTTNSVVSVLEGGDPVVIPNAEGSRTTPSVVAFSKDGEVLVGEVAKRQAITNPDRTIRSVKRHMGTNWTIEIDGKKYTPQEISARILQKLKRDAEEYLGDTVT EAVITVPAYFDDAQRTATKEAGQIAGLEVLRIINEPTAAALAYGLDKEGSEQTILVFDLGGGTFDVSILEIGEGVFEVKSTHGDTNLGGDDWDQRIIDWLVESFKNDHGVDLS KDPMALQRLKEAAEKAKIELSQTQSSQINLPFITATDAGPLHLDYTLTRAKFQEMTADLLERCKGPFEQAIKDANLTKADIDHVVLVGGSTRMPAVQDLVRELTGKEPNKGV NPDEVVAIGAAIQAGVLKGEVKDVLLLDVTPLSLGIETKGGVMTKLIERNTTIPTRKTEVFTTAEDNQPSVEIHVLQGEREMAAYNKTLGKFQLVDLPPAPRGVPQIEVTFDI DANGIVHVSAKDRATGKEQSMTITGQSSLDKNTIEQMIKDAEAHAEEDRRRREEVEIRNNADTLVYQTEKLLRDQGDQISDDEKSKIEEKIAAVKSALEGSDVEAIKDATEAL MAASQEFGQRLYEAAAAQERQASAGASQPADDEVVDAEIVDDES | (SEQ ID NO 52) RIINEPTAAALAY GLDK | 1 4 3 | 1 5 9 |
| | *Aggreg atibacte r actinom ycetemc omitans* | (SEQ ID NO 3) MGKIVGIDLGTTNSCVAVMEGEKPRVIENAEGDRTTPSIIAYTNDNETLVGQPAKRQAVTNPKNTLFAIKRLIGRRFEDEEVKRDIDIMPFAITKADNGDAWVEVKGEKLAP PQISAEVLKKMKKAAEDYLGEPVTEAVITVPAYFNDAQRQATKDAGRIAGLDVKRIINEPTAAALAYGLDKGTGNKTIAVYDLGGGTFDLSIIEIDEVGGEKTFEVLATNGDT HLGGEDFDNRIINYLVDEFKKEQGIDLRNDPLAMQRLKEAAEKAKIELSSAQQTDVNLPYITADATGPKHLNIKLTRAKLESLVEDLVAKSLEPVRIALKDAGKSPSEIDDVILV GGQTRMPLVQQEVEKFFGKAPRKDVNPDEAVAIGAAVQGGVLAGDVKDVLLLDVTPLSLGIETMGGVMTTLIEKNTTIPTKKSQVFSTAEDNQSAVTIHVLQGERKQASA NKSLGQFNLEGINPVPRGMPQIEVTFDIDADGIIHVSAKDKGTGKEQQITIKSSSGLSDEEIQQMVRDAEANAESDRKFEELVQARNQADHLIHSTRKQLDEAGDKVPAAD RGTIESALSDLEKAAKGEDKAVIEAKIKALAEVAQKLAQTAQPHGDPQQAQSNSKPNDDVVDAEFEEVKDNK | (SEQ ID NO 53) RMPLVQQEVEK F | 3 4 5 | 3 5 6 |
| | | | (SEQ ID NO 54) TNGDTHLGGED FDNRI | 22 1 | 2 3 6 |
| | | | (SEQ ID NO 55) ATNGDTHLGGE DFDNRI | 2 2 0 | 2 3 6 |
| | | | (SEQ ID NO 56) GDTHLGGEDFD NRI | 2 2 3 | 2 3 6 |
| | | | (SEQ ID NO 57) IINEPTAAALAYG LDKG | 1 6 8 | 1 8 4 |
| | | | (SEQ ID NO 58) LATNGDTHLGG EDFDNRI | 2 1 9 | 2 3 6 |
| | | | (SEQ ID NO 59) NGDTHLGGEDF DNRI | 2 2 2 | 2 3 6 |
| | | | (SEQ ID NO 60) RIINEPTAAALAY GLDKG | 1 6 7 | 1 8 4 |
| | *Aggreg atibacte r paraphr ophilus* | (SEQ ID NO 4) MRNSIHILQSVNHYTEFKISLRRKQMGKIIGIDLGTTNSCVAVMDGDKPRVIENAEGDRTTPSIIAYTNDNEILVGQPAKRQAVTNPKNTLFAIKRLIGRRFEDAEVKRDIDIM PFEITRADNGDAWVNVKGDKLAPPQISAEVLKKMKKTAEDFLGEPVTEAVITVPAYFNDAQRQATKDAGRIAGLEVKRIINEPTAAALAYGLDKGQGNKTIAVYDLGGGTF DLSIIEIDEVGGEKTFEVLATNGDTHLGGEDFDNRVINYLVDEFQKEQGIDLRKDPLAMQRLKEAGEKAKIELSSAQQTDVNLPYITADATGPKHLNIKLTRAKLESLVEDLVA KSLEPVKVALSDAGLSASQVDDVILVGGQTRMPLVQQKVEEFFGKAPRKDVNPDEAVAVGAAVQGGVLAGDVKDVLLLDVTPLSLGIETMGGVMTTLIEKNTTIPTKKSQ VFSTAEDNQSAVTIHVLQGERKQASANKSLGQFNLEGINPAPRGMPQIEVTFDIDADGIIHVSAKDKGTGKEQQITIKASSGLSDEEIQQMVRDAEANAESDRKFEELVQA RNQADHLVHSTRKQLSEAGDNVPAADRAAIESALSDLETAAKGEDKAVIEAKLQALAEVAQKLAQTAQPQGEPQQAQSNGKPNDDVVDAEFEEVKDNK | (SEQ ID NO 61) TNGDTHLGGED FDNRV | 2 4 6 | 2 6 1 |
| | | | (SEQ ID NO 62) ATNGDTHLGGE DFDNRV | 2 4 5 | 2 6 1 |
| | | | (SEQ ID NO 63) GDTHLGGEDFD NRV | 2 4 8 | 2 6 1 |
| | | | (SEQ ID NO 64) IINEPTAAALAYG LDKG | 1 9 3 | 2 0 9 |
| | | | (SEQ ID NO 65) LATNGDTHLGG EDFDNRV | 2 4 4 | 2 6 1 |
| | | | (SEQ ID NO 66) NGDTHLGGEDF DNRV | 2 4 7 | 2 6 1 |
| | | | (SEQ ID NO 67) RIINEPTAAALAY GLDKG | 1 9 2 | 2 0 9 |
| | *Aggreg atibacte r sp. HMT* 458 | (SEQ ID NO 5) MGKIIGIDLGTTNSCVAVMDGDKPRVIENAEGDRTTPSIIAYTNDNETLVGQPAKRQAVTNPKNTLFAIKRLIGRRFEDAEVKRDIDIMPFTITKADNGDAWVEVKGEKLAP PQISAEVLKKMKKTAEDFLGEPVTEAVITVPAYFNDAQRQATKDAGRIAGLEVKRIINEPTAAALAYGLDKGQGNKTIAVYDLGGGTFDLSIIEIDEVGGEKTFEVLATNGDT HLGGEDFDNRVINYLVDEFQKEQGIDLRKDPLAMQRLKEAGEKAKIELSSAQQTDVNLPYITADATGPKHLNIKLTRAKLESLVEDLVAKSLEPVKVALNDAGLTASQIDDVIL VGGQTRMPLVQQKVEEFFGKAPRKDVNPDEAVAIGAAVQGGVLAGDVKDVLLLDVTPLSLGIETMGGVMTTLIEKNTTIPTKKSQVFSTAEDNQSAVTIHVLQGERKQAS | (SEQ ID NO 68) ATNGDTHLGGE DFDNRV | 2 2 0 | 2 3 6 |
| | | | (SEQ ID NO 69) GDTHLGGEDFD NRV | 2 2 3 | 2 3 6 |
| | | ANKSLGQFNLEGINPAPRGMPQIEVTFDIDADGIIHVSAKDKGTGKEQQITIKASSGLSDEEIQQMVRDAEANAESDRKFEELVQARNQADHLVHSTRKQLSEAGDNVPA ADRAAIESALSDLESAAKGEDKAVIEAKLQALAEVAQKLAQTAQPQGEPQQAQSNGKPNDDVVDAEFEEVKDNK | (SEQ ID NO 70) IINEPTAAALAYG LDKG | 1 6 8 | 1 8 4 |
| | | | (SEQ ID NO 71) LATNGDTHLGG EDFDNRV | 2 1 9 | 2 3 6 |
| | | | (SEQ ID NO 72) NGDTHLGGEDF DNRV | 2 2 2 | 2 3 6 |
| | | | (SEQ ID NO 73) RIINEPTAAALAY GLDKG | 1 6 7 | 1 8 4 |
| | | | (SEQ ID NO 74) TNGDTHLGGED FDNRV | 2 2 1 | 2 3 6 |
| | *Campyl obacter sputoru* m | (SEQ ID NO 6) MGKVIGIDLGTTNSCVAIYEHGESKVIPNKEGKNTTPSVVAFTDKGEILVGDTAKRQAVTNPKKTIYSIKRIMGLMMNEPNAKEAKNRLPYAIVDRNGACAIDIDGKIYTPQE ISAKVLMKLKEDAESYLGEKVVDAVITVPAYFNDSQRKATKEAGTIAGLNVLRIVNEPTAAALAYGLDKKTAEKIVVYDLGGGTFDVTVLETGDNVVEVLSTGGNAFLGGDD FDNKLIDFLVSEFKSESGIDLKSDVMALQRLKEAAENAKKELSSAMETTINLPFITADATGPKHLTKTITRAKFESMIDSLVAETITTLNKVVKEAGLDKSDVKEVVMVGGSTRV PLVQDEVKKAFGKDLNKSVNPDEVVAIGAAIQGAVIKGDVKDVLLLDVTPLSLGIETLGGVMTKVIEKGTTIPTKKSQVFSTADDNQSAVTIHVLQGEREFSRDNKSLGQFNL EGIPPAPRGVPQIEVEFDIDANGILTVSAKDKATGKAQNITISGSSGLSEEEINKMVNDAEAHKEEDKKRKESVEARNNADALAHQTQKSLDELGEKIPAEDRAKIEAALNDL KETLKDENASKDQIDAKVKALSEVSHKLAESMYKNKDNSNNQSSDKTDKKDDDVIDAEVE | (SEQ ID NO 75) AVITVPAYFNDS QRK | 1 3 7 | 1 5 1 |
| | | | (SEQ ID NO 76) DAVITVPAYFND SQRK | 1 3 6 | 1 5 1 |
| | | | (SEQ ID NO 77) KEAGTIAGLNVL RI | 1 5 4 | 1 6 7 |
| | | | (SEQ ID NO 78) RIVNEPTAAALA YGLDKKT | 1 6 6 | 1 8 4 |
| | | | (SEQ ID NO 79) RIVNEPTAAALA YGLDKK | 1 6 6 | 1 8 3 |
| | | | (SEQ ID NO 80) TVPAYFNDSQR K | 1 4 0 | 1 5 1 |
| | | | (SEQ ID NO 81) VDAVITVPAYFN DSQRK | 1 3 5 | 1 5 1 |
| | | | (SEQ ID NO 82) VNEPTAAALAY GLDKKT | 1 6 8 | 1 8 4 |
| | *Campyl obacter ureolyti cus* | (SEQ ID NO 7) MSKVIGIDLGTTNSAVAIFERGESKIIPNKEGKNTTPSIVAFTDKGEVLVGDTAKRQAVTNPSKTIYSIKRIMGLMMNENNAKEAQKRLPYKIVDRNGAAAVEIDGKVYTPQE ISAKVLMKLKEDAESYLGGSVVDAVITVPAYFNDSQRKATKEAGTIAGLNVLRIVNEPTAAALAYGLDKKESEKIVVYDLGGGTFDVTVLETGDNVVEVLATGGDAFLGGDD FDNKLIDWLLTEFKSESGIDLKNDVMAMQRLKEAAENAKKELSSAMETTVNLPFITADQTGPKHLTKTLTRAKFESMIEDLVASTIKTLKNVMSDAGVSNSDIAEIVMVGGS TRVPLVQEEVKKAFGGKELNKSVNPDEVVAIGAAIQGAVIKGDVKDVLLLDVTPLSLGIETLGGVMTKVIEKGTTIPTKKSQVFSTAEDNQTAVTINVLQGEREFAKDNKTLG NFNLEGIMPAQRGIPQIEVQFDIDANGILTVSAKDKATGKATDIRITGSSGLSEAEIDKMVKDAELHKEEDSKRKELIETRNQADGIAHQTEKTLNEMGEKIPNDVRAKIESAL NDLKATLKDESATKEAIDSKVQALSKVAEDMYKAAASQNAGANNAGADNKSGKKNDDDVIDAEVE | (SEQ ID NO 83) AVITVPAYFNDS QRK | 1 3 7 | 1 5 1 |
| | | | (SEQ ID NO 84) DAVITVPAYFND SQRK | 1 3 6 | 1 5 1 |
| | | | (SEQ ID NO 85) KEAGTIAGLNVL RI | 1 5 4 | 1 6 7 |
| | | | (SEQ ID NO 86) RIVNEPTAAALA YGLDKKE | 1 6 6 | 1 8 4 |
| | | | (SEQ ID NO 87) RIVNEPTAAALA YGLDKK | 1 6 6 | 1 8 3 |
| | | | (SEQ ID NO 88) TVPAYFNDSQR K | 1 4 0 | 1 5 1 |
| | | | (SEQ ID NO 89) VDAVITVPAYFN DSQRK | 1 3 5 | 1 5 1 |
| | | | (SEQ ID NO 90) VNEPTAAALAY GLDKKT | 1 6 8 | 1 8 4 |
| | *Coryneb acteriu* m *coyleae* | (SEQ ID NO 8) MARAVGIDLGTTNSVVSVLEGGEPVVIANAEGSRTTPSVVAFAKNGEVLVGQSAKNQAVTNVDRTIRSVKRHMGEDWTVDIDGKKYTPQEISARTLQKLKRDAEAYLGDE VTEAVITVPAYFEDAQRQATKEAGQIAGLNVLRIVNEPTAAALAYGLDKAEKEETILVFDLGGGTFDVSLLEIGDGVVEVLATAGDNELGGDDWDNRVVEWLVDKFKSQN GVDLSKDKMAMQRLREAAEKAKIELSSAQQASINLPYITVDAEKNPLFLDETLTRTEFQKITSDLLDRTKSPFNQVIKDAGMSVGDIDHVVLVGGSTRMPAVSDLVKELTGK EPNKSVNPDEVVALGAALQAGVLRGDVKDVLLLDVTPLSLGIETKGGVMTKLIERNTTIPTKRSETFTTAEDNQPSVQIQVFQGEREIASANKLLGSFELAGIAPAPRGVPQIE VTFDIDANGIVSVSAKDKATGKENTIKIQDGSGLSDEEIDRMIKDAEAHADEDKKRREEQETRNNAETMAYQTRKFMDENGEKLSEDIKTRVTEAADAVDEALKGEDLDAI KSAVEKLTTESQAMGTALYEAQANEGATQADAGDDNVVDAEVVEDDDTDADNK | (SEQ ID NO 91) RIVNEPTAAALA YGLDKA | 1 4 3 | 1 6 0 |
| | *Cutibact erium sp. HMT 193* | (SEQ ID NO 9) MARSVGIDLGTTNSCVAVLEGGEPTVIPNAEGARTTPSVVAFTNSGETLVGEVAKRQAVTNVDRTVRSVKRHMGETWTMGVDDKTYKPQQISAFILQKLKRDAEAYLGE PVTNAVITVPAYFSDAQRQATKEAGEIAGLTVDRIVNEPTAAALAYGLDKADKEQTVLVFDLGGGTFDVSLLDISDGVFEVKATNGDNHLGGDDWDQRIVDWLVTQFKN ANGIDLAADKMAKQRLQEAAERAKIELSQASETHINLPYITAGAAGPLHLDEKLTRAEFQRMTSDLLERCRTPFNAVMKDAGLNVSQIDEVILVGGSTRMPAVAELVKELA | (SEQ ID NO 92) RIVNEPTAAALA YGLDKA | 1 4 3 | 1 6 0 |
| | | GKEPHKGVNPDEVVALGASLQAGVLKGEVKDVLLLDVTPLSLGIETKGGVMTKIIERNTTIPTKRSEVFTTAEDNQPSVMIQVFQGEREFVRDNKSLGNFELTGLMPAPRGI PQIEVSFDIDANGIVHVHAKDMATGKEQSMTVTGGSALGKDEIDRMVKEAEANAESDKKRREAVEMRNEADALAFRTEKLLDENSDKIPEDTKAPVTEAIAKLKETLKGTD NDDEVKAAMDDLNQKASAMGQAIYAAAQQAQAENPQGQDSESANSESGDDTVVDAEIVDDEDEKK | | | |
| | *Enteroc occus faecalis* | (SEQ ID NO 10) MSKIIGIDLGTTNSAVAVLEGGEAKIIANPEGNRTTPSVVSFKNGEIQVGEVAKRQAVTNPNTISSIKRHMGEAGYKVDVEGKSYTPQEVSAMILQYLKGFAEDYLGEKVEKA VITVPAYFNDAQRQATKDAGKIAGLEVERIVNEPTAAALAYGLDKTDKDEKILVFDLGGGTFDVSILELGDGVFDVLSTAGDNNLGGDDFDNKIIDYMVAEFKKENGIDLAN DKMALQRLKDAAEKAKKDLSGVTSTQISLPFITAGEAGPLHLEMNLTRAKFDELTSDLVERTKVPVRQALKDAGLNPSEIDEVILVGGSTRIPAVVEAVRKETNKEPNKSVNP DEVVAMGAAIQGGVITGDVKDVVLLDVTPLSLGIETMGGVFTKLIDRNTTIPTSKSQVFSTAADNQPAVDIHVLQGERPMAADNKTLGRFQLTDIPAAPRGVPQIEVSFDI DKNGIVNVRAKDLGTQKEQTITIKSSSGLSDDEIERMVKDAEANAEADKQRKEEVDLRNDADALLFTVDKTLKELEGKVDAEEVKKAEDARDELKAAIEANDIEQMKAKRD SLNEIVQNLTVKLYEQAAQQQAQENPEAAQGGADDVVDADFEEVNGDDK | (SEQ ID NO 93) RIVNEPTAAALA YGLDKT | 1 4 2 | 1 5 9 |
| | | | (SEQ ID NO 94) RIVNEPTAAALA YGLDKTDKDE | 1 4 2 | 1 6 3 |
| | *Haemo philus parainfl uenzae* | (SEQ ID NO 11) MGKIIGIDLGTTNSCVAVMDGDKPRVIENAEGDRTTPSIIAYTNDNEILVGQPAKRQAVTNPKNTLFAIKRLIGRRFDDAEVKRDIDIMPFEITKADNGDAWVNVKGDKLAP PQISAEVLKKMKKTAEDFLGEPVTEAVITVPAYFNDAQRQATKDAGRIAGLEVKRIINEPTAAALAYGLDKGQGNKTIAVYDLGGGTFDLSIIEIDEVGGEKTFEVLATNGDT HLGGEDFDNRVINYLVDEFKKEQGVDLRNDPLAMQRLKEAGEKAKIELSSAQQTDVNLPYITADATGPKHLNIKLTRAKLESLVEDLVAKSLEPVKVALNDAGLSASQIDDVI LVGGQTRMPLVQQKVEEFFGKAPRKDVNPDEAVAIGAAVQGGVLAGDVTDVLLLDVTPLSLGIETMGGVMTTLIEKNTTIPTKKSQVFSTAEDNQSAVTIHVLQGERKQA SANKSLGQFNLEGINPAPRGMPQIEVTFDIDADGIIHVSAKDKGTGKEQQITIKASSGLSDEEIQKMVRDAEANAEADRKFEELVQARNQADHLVHSTRKQLEEAGDKVPA EDRAAIESALSDLETAAKGEDKAAIEAKLQVLAEVAQKLVQAAQPQADTQQAQSSNPNDDVVDAEFEEVKDNK | (SEQ ID NO 95) TNGDTHLGGED FDNRV | 2 2 1 | 2 3 6 |
| | | | (SEQ ID NO 96) ATNGDTHLGGE DFDNRV | 2 2 0 | 2 3 6 |
| | | | (SEQ ID NO 97) GDTHLGGEDFD NRV | 2 2 3 | 2 3 6 |
| | | | (SEQ ID NO 98) IINEPTAAALAYG LDKG | 1 6 8 | 1 8 4 |
| | | | (SEQ ID NO 99) NGDTHLGGEDF DNRV | 2 2 2 | 2 3 6 |
| | | | (SEQ ID NO 100) RIINEPTAAALAY GLDKG | 1 6 7 | 1 8 4 |
| | *Haemo philus pittman iae* | (SEQ ID NO 12) MGKIIGIDLGTTNSCVAVMDGDKPRVIENAEGDRTTPSIIAYTNDNEILVGQPAKRQAVTNPKNTLFAIKRLIGRRFEDEEVKRDIDIMPFAITKADNGDAWVEVKGDKLAP PQISAEVLKKMKKAAEDFLGETVTEAVITVPAYFNDAQRQATKDAGRIAGLDVKRIINEPTAAALAYGLDKGQGNKTIAVYDLGGGTFDLSIIEIDEVGGEKTFEVLATNGDT HLGGEDFDNRIINYLVDEFKKEQGVDLRNDPLAMQRLKEAAEKAKIELSSAQQTDVNLPYITADATGPKHLNIKLTRAKLESLVEDLVAKSLEPVRIALKDAGKSASEIDDVILV GGQTRMPLVQQEVEKFFGKAPRKDVNPDEAVAIGAAVQGGVLAGDVTDVLLLDVTPLSLGIETMGGVMTTLIEKNTTIPTKKSQVFSTAEDNQSAVTIHVLQGERKQASA NKSLGQFNLEGINPAPRGLPQIEVTFDIDADGIIHVSAKDKGTGKEQQITIKASSGLSDDEIQQMVRDAEANAEADRKFEELVQARNQADHLVHSTRKQLEEAGDKVPAAD RAEIESALSNLETAAKGEDKAAIEAKLQALASVAQKLAQTAQSQGDAQQAQGANKANDDVVDAEFEEVKDNK | (SEQ ID NO 101) TNGDTHLGGED FDNRI | 2 2 1 | 2 3 6 |
| | | | (SEQ ID NO 102) ATNGDTHLGGE DFDNRI | 2 2 0 | 2 3 6 |
| | | | (SEQ ID NO 103) GDTHLGGEDFD NRI | 2 2 3 | 2 3 6 |
| | | | (SEQ ID NO 104) IINEPTAAALAYG LDKG | 1 6 8 | 1 8 4 |
| | | | (SEQ ID NO 105) NGDTHLGGEDF DNRI | 2 2 2 | 2 3 6 |
| | | | (SEQ ID NO 106) RIINEPTAAALAY GLDKG | 1 6 7 | 1 8 4 |
| | *Lactipla ntibacill* us *plantar* um | (SEQ ID NO 13) MASNKIIGIDLGTTNSAVAVLEGNEPKIITTPEGGRTVPSVVAFKDGETQVGEVAKRQAITNPNTVASIKRHMGEAGYKVSIEGKDYTPQQISAMILQYIKGFAEDYLGDTVE KAVVTVPAYFNDAQRQATKDAGKIAGLNIERIINEPTAAALAYGLDKTDKDEKILVYDLGGGTFDVSILELGDGVFEVLSTNGDTHLGGDDFDQKIIDWLVDGFKADNGVDL SKDKMALQRLKDAAEKAKKDLSGVSEAQISLPFISAGASGPLHLETTLTRAKFNELTADLVEKTRIPVENALKDADLSASDLDVVILNGGSTRIPAVQEAVEKWTGKESNHSIN PDEAVALGAAVQGGVITGDVKDVVLLDVTPLSLGIETMGGVFTKLIDRNTTIPTSKSQVFSTAADNQPAVDIHVLQGERPMAADNKTLGRFQLTDIPAAPRGVPQIEVKFDI DKNGIVNVSAKDMGTNKEQKITIKSSSGLSDDEIDQMVKEAKENEEADKKRKEEVDLKNEVDQLIFTTDKTLKDLEGKVSEDEVKKAKDARDALKKAQDDNNIDEMKAKK DDLNKIVQDLSVKLYQQAQEAQGAQGGADSNAAGNANSAKGSDDNTVDGDFEDLDKDKDKK | (SEQ ID NO 107) RIINEPTAAALAY GLDKT | 1 4 4 | 1 6 1 |
| | | | (SEQ ID NO 108) ADNGVDLSKDK MALQRL | 2 1 9 | 2 3 5 |
| | *Lactoco ccus lactis* | (SEQ ID NO 14) MSKIIGIDLGTTNSAVAVLEGTESKIIPNPEGNRTTPSVVAFKNGEIIVGDAAKRQAVTNPETIISIKSKMGTSEKVSANGKEYTPQEISAMILQNLKATAEAYLGEKVEKAVITV PAYFNDAQRQATKDAGKIAGLEVERIVNEPTAAALAYGLDKTDKDEKILVFDLGGGTFDVSILELGDGVFDVLATAGNNKLGGDDFDQKIIDWMVAEFKKENGIDLAQDK MALQRLKDAAEKAKKDLSGVTTTQISLPFITAGAAGPLHLEMALTRAKFDELTHDLVEATRQPVRQALSDAGLSTSDIDEVLLVGGSTRIPAVVELVRHETNKEPNKSVNPD EVVAMGAAIQGGVITGDVKDVVLLDVTPLSLGIETMGGVFTKLIDRNTTIPTSKSQVFSTAADNQPAVDIHVLQGERPMAADNKTLGRFQLTDIPAAPRGIPQIEVTFDIDK NGIVSVKAKDLGTQKEQTIVIKSNSGLSDEEIDKMMKDAEANADADAKRKEEVDTRNEADALVFQTEKTLKDLEGKVEEAEVKKAEEAKEELKKALEGEDIDDIKAKSEALSE IAQNLAVKLYEQANAAQGEAGQATDAQEAPKDDNTFDGDFEESK | (SEQ ID NO 109) RIVNEPTAAALA YGLDKT | 1 4 1 | 1 5 8 |
| | | | (SEQ ID NO 110) RIVNEPTAAALA YGLDKTDKDE | 1 4 1 | 1 6 2 |
| | | | (SEQ ID NO 111) TSDIDEVLLVGG STRI | 3 0 1 | 3 1 6 |
| | *Lancefie Idella rimae* | (SEQ ID NO 15) MGKILGIDLGTTNSAMAVLEGGEPTIIVNAEGDRTTPSVVGFRADGDRIVGKAAKNQAVTNPVNTVFSIKRFMGRKFDEVQSEMKTVPYKVKSGTDGRAVVEIEGTDYTP EQISAMILSKMKADAEKYLGEKVTDAVITVPAYFNDSQRQATKDAGKIAGLNVQRIVNEPTAAALAYGLDKKDKDQKVLVFDLGGGTFDVSLLDLADGVVEVLATNGDNH LGGDDWDQRVIDWLADKFNSDNGIDLRKDPMALQRLKEAAENAKKELSSAQQAQINLPFITADATGPKHLDYTLTRAEFERITRDLLDRCKTPVTKALQDAGLQISNVDEV ILVGGSTRMPAVQDLVKTMTGKQPNMSVNPDEVVADGAAVQGGVLTGDVSGILLLDVTPLSLGVETMGGVMTKMIDRNTTIPTSKTEIYSTAADNQTSVEINVLQGERE MAADNKSLGKFNLTGIPAARRGTPQIEVTFDIDANGIVKVTAKDKATGKSQEITISGSTALSDDEVDRMVKDAESHAEEDKKRKDEIEVRNQTDSLAYGTEQTLKDLGDKVP EDQKKATEEAVAEARKALEGTDIEAIKAAGEKLQEVGHKLAEVAYADAQAQTAGTDGASAGNSSDGDVEDADYEVVDDDKSQN | (SEQ ID NO 112) AVITVPAYFNDS QRQ | 1 3 6 | 1 5 0 |
| | | | (SEQ ID NO 113) DAVITVPAYFND SQRQ | 1 3 5 | 1 5 0 |
| | | | (SEQ ID NO 114) RIVNEPTAAALA YGLDKKD | 1 6 5 | 1 8 3 |
| | | | (SEQ ID NO 115) RIVNEPTAAALA YGLDKK | 1 6 5 | 1 8 2 |
| | | | (SEQ ID NO 116) TDAVITVPAYFN DSQRQ | 1 3 4 | 1 5 0 |
| | | | (SEQ ID NO 117) TVPAYFNDSQR Q | 1 3 9 | 1 5 0 |
| | | | (SEQ ID NO 118) VNEPTAAALAY GLDKKD | 1 6 7 | 1 8 3 |
| | *Mycolici bacteriu* m *neoauru* m | (SEQ ID NO 16) MARAVGIDLGTTNSVVAVLEGGDPVVVANSEGSRTTPSVVAFARNGEVLVGQPAKNQAVTNVDRTIRSVKRHMGKDWTVAIDGKDYTPQEISARTLQKLKRDAEAYLGE DITDAVITVPAYFNDAERQATKEAGQIAGLNVLRIVNEPTAAALAYGLDKGQKEQTILVFDLGGGTFDVSLLEIGDGVVEVRATSGDNHLGGDDWDDRIVEWLVDKFKSTS GIDLTKDKMAMQRLREAAEKAKIELSSGQSTSINLPYITVDADKNPLFLDEQLTRAEFQRITQDLLDRTRAPFQQVIKDAGISVGEIDHVVLVGGSTRMPAVTDLVKELTGGK EPNKGVNPDEVVAAGAALQAGVLKGEVKDVLLLDVTPLSLGIETKGGVMTKLIERNTTIPTKRSETFTTADDNQPSVQIQVFQGEREIASHNKLLGSFELTGIPPAPRGVPQI EVTFDIDANGIVHVTAKDKGTGKENTIKIQEGSGLSKEEIDRMIKDAEAHADEDRQRREEADVRNQAESLVYQTEKFVAEQRSAEGGSKVPEDTLAKVDSAIAEAKTALAGT DIAAIKSAMEKLGTESQALGQAIYEATQAEQGAGGDAGSQQADDNVVDAEVVEDDQERK | (SEQ ID NO 119) RIVNEPTAAALA YGLDKG | 1 4 3 | 1 6 0 |
| | | | (SEQ ID NO 120) SFELTGIPPAPRG | 4 3 0 | 4 4 2 |
| | *Parasca rdoviadenticol ens* | (SEQ ID NO 17) MGRAVGIDLGTTNSCIATLEGGQPTVIVNAEGARTTPSVVAFSKSGEILVGEVAKRQAVTNVDRTISSVKRHMGTDWTVEIDGKKWTPQEISAQVLMKLKRDAEAYLGEP VTDAVITCPAYFNDAQRQATKDAGTIAGLNVLRIINEPTAAALAYGLEKGKEDERILVFDLGGGTFDVSLLEIGKDDDGFSTIQVQATNGDNHLGGDDWDQKIIDWLVGEV | (SEQ ID NO 121) | 1 3 1 | 1 4 4 |
| | | KNKYGVDLSKDKIALQRLKEAAEQAKKELSSSQTTSISMQYLAMTPDGTPVHLDETLTRAHFESMTSDLLNRCRDPFNAVLRDSGISVSQIDHIVLVGGSTRMPAVQDLVKE LTGGKEANKTVNPDEVVAVGAAVQSGVIKGDRSDVLLIDVTPLSLGIETKGGIMTKLIDRNTAIPTKRSEVFSTAEDNQPSVLIQVYQGEREFARDNKPLGTFELTGIAPAPR GLPQIEVTFDIDANGIVHVSAKDKGTGKEQSMTITGGSGLPKDEIDRMVKEAESHEAEDKKRREDAETRNQAESFAYQTEKLVNDNKDKLSDDVAKEVTDKVNALKEALK GDDMEKIKSAQSELMTSAQKIGQQLYSQQAAAGADASAAGTGASSTSNGSASASDDDVVDAEVVDDDDKDKN | KDAGTIAGLNVL RI | | |
| | | | (SEQ ID NO 122) SGISVSQIDHIVL VGGSTRM | 3 0 5 | 3 2 4 |
| | | | (SEQ ID NO 123) SQIDHIVLVGGS TRM | 3 1 0 | 3 2 4 |
| | | | (SEQ ID NO 124) TAAALAYGLEKG | 1 4 9 | 1 6 0 |
| | | | (SEQ ID NO 125) VSQIDHIVLVGG STRM | 3 0 9 | 3 2 4 |
| | *Proteus mirabili s* | (SEQ ID NO 18) MGKIIGIDLGTTNSCVAVMDGKNARVIENGEGDRTTPSIVAYAQDGEILVGQPAKRQAVTNSQNTLFAIKRLIGRRFEDAEVQRDVSIMPYKIIKADNGDAWVEARNEKM APPQVSAEVLKKMKKTAEDYLGEPVTEAVITVPAYFNDAQRQATKDAGRIAGLDVKRIINEPTAAALAYGLDREVGNRTIAVYDLGGGTFDISIIEIDEVDGEKTYEVLSTNG DTHLGGEDFDSRLINYLVDEFKKEQGIDLRNDPLAMQRLKEAAEKAKIELSSAQQTDVNLPYVTADATGPKHLNIKVTRAKLESLVEDLVKRSMDPVKVALEDAGLKVSEVN DVILVGGQTRMPMVQKTVAEFFGKEPRKDVNPDEAVAMGAAVQGGVLAGDVKDVLLLDVTPLSLGIETMGGVMTSLIAKNTTIPTKHSQVFSTAEDNQSAVTIHVLQGE RKRASDNKSLGQFNLDGIQPAPRGMPQIEVTFDIDADGILHVSAKDKNSGREQNITIKASSGLNEEEIQKMVRDAEANAEADRKFEELVQTRNQADQLVHGTRKQIEEAG DKLAANDKEAIEKALSELEIASKGEDKAAIEAKLQALVEASKPLLEIAQQQAQAGAGNTADATDAGAKKDDDVVDAEFEEVDGKDKK | (SEQ ID NO 126) INEPTAAALAYG LDRE | 1 6 9 | 1 8 4 |
| | | | (SEQ ID NO 127) RIINEPTAAALAY GLDRE | 1 6 7 | 1 8 4 |
| | *Scardov ia inopinat a* | (SEQ ID NO 19) MGRAVGIDLGTTNSCIATLEGGQPTVIVNAEGVRTTPSVVAFSKSGEILVGEVAKRQAVTNVDRTISSVKRHMGTDWSVDIDGKKWTPQEVSAQILMKLKRDAEAYLGEP VTDAVITCPAYFNDAQRQATKDAGTIAGLNVLRIINEPTAAALAYGLEKGKEDERILVFDLGGGTFDVSLLEIGKDEDGFSTIQVQATNGDNHLGGDDWDQAIIDWLVSEV KNKYGVDLSKDKIALQRLKEAAEQAKKELSSSSSTSISMQYLAMTPDGTPVHLDETLTRAHFEEMTADLLNRCRDPFNAVLRDSGISVSQIDHIVLVGGSTRMPAVQDLVKE LTGGKEPNKTVNPDEVVAVGAAVQSGVIKGDRSDVLLIDVTPLSLGIETKGGIMTKLIDRNTAIPTKRSEVFSTAEDNQPSVLIQVYQGEREFARDNKPLGTFELTGIAPAPQ GVPKIEVTFDIDANGIVHVSAKDQGTGKEQSMTITGGSGLPKEEIDRMVKEAESHASEDKKRREEAETRNQAESFAYQTEKLVKDNKDKISDDLAKEVTDKVNALKEALKGE DIEAVKTAQTELMNSAQKIGQALYAQQQSAGADGAAAASGAGAGSSAGASSNDDDVVDAEVVDDDDKDKK | (SEQ ID NO 128) KDAGTIAGLNVL RI | 1 3 1 | 1 4 4 |
| | | | (SEQ ID NO 129) SGISVSQIDHIVL VGGSTRM | 3 0 5 | 3 2 4 |
| | | | (SEQ ID NO 130) SQIDHIVLVGGS TRM | 3 1 0 | 3 2 4 |
| | | | (SEQ ID NO 131) TAAALAYGLEKG | 1 4 9 | 1 6 0 |
| | | | (SEQ ID NO 132) VSQIDHIVLVGG STRM | 3 0 9 | 3 2 4 |
| | *Scardov ia wiggsia e* | (SEQ ID NO 20) MGRAVGIDLGTTNSCIATLEGGQPTVIVNAEGMRTTPSVVAFSKSGEILVGEVAKRQAVTNVDRTISSVKRHMGTDWSVDIDGKKWTPQEVSAQILMKLKRDAEAYLGE PVTDAVITCPAYFNDAQRQATKDAGTIAGLNVLRIINEPTAAALAYGLEKGKEDERILVFDLGGGTFDVSLLEIGKDEDGFSTIQVQATNGDNHLGGDDWDQAIINWLVRE VKNKYNVDLSKDKIALQRLKEAAEQAKKELSSSTSTNISMQYLAMTPDGTPVHLDEQLTRARFEEMTADLLDRCREPFNAVLRDSGVSVSQIDHIVLVGGSTRMPAVQELV KELTGGKEPNKTVNPDEVVAVGAAVQSGVIKGDRSDVLLIDVTPLSLGIETKGGIMTKLIDRNTAIPTKRSEVFSTAEDNQPSVLIQVYQGEREFARDNKPLGTFELTGIAPAP RGVPQIEVTFDIDANGIVHVSAKDKGTGKEQSMTITGGSGLPKDEIDRMVKEAESHEAEDKKRREDAETRNQAESFAYQTEKLVSDNKDKLSDDTAKEVTDKVNALKEALK GDDIEAVKSAQSELMASAQKIGQQLYSQQSADQAQAAGGTGASGTGSSAAGSDSGSNGSDDDVVDAEVVDDDKDNK | (SEQ ID NO 133) KDAGTIAGLNVL RI | 1 3 1 | 1 4 4 |
| | | | (SEQ ID NO 134) SQIDHIVLVGGS TRM | 3 1 0 | 3 2 4 |
| | | | (SEQ ID NO 135) TAAALAYGLEKG | 1 4 9 | 1 6 0 |
| | | | (SEQ ID NO 136) VSQIDHIVLVGG STRM | 3 0 9 | 3 2 4 |
| | *Streptoc occus cristatu s* | (SEQ ID NO 21) MSKIIGIDLGTTNSAVAVLEGTESKIIANPEGNRTTPSVVSFKNGEIIVGDAAKRQAVTNPDTVISIKSKMGTSEKVSANGKEYTPQEISAMILQYLKGYAEDYLGEKVTKAVIT VPAYFNDAQRQATKDAGKIAGLEVERIVNEPTAAALAYGLDKTDKEEKILVFDLGGGTFDVSILELGDGVFDVLATAGDNKLGGDDFDQKIIDHLVAEFKKENGIDLSTDKM AMQRLKDAAEKAKKDLSGVTSTQISLPFITAGEAGPLHLEMTLTRAKFDDLTRDLVERTKVPVRQALSDAGLSLSDIDEVILVGGSTRIPAVVEAVKAETGKEPNKSVNPDEV VAMGAAIQGGVITGDVKDVVLLDVTPLSLGIETMGGVFTKLIDRNTTIPTSKSQVFSTAADNQPAVDIHVLQGERPMAADNKTLGRFQLTDIPAAPRGIPQIEVTFDIDKNG IVSVKAKDLGTQKEQTIVIQSNSGLTDEEIDRMMKDAEANAEADKKRKEEVDLRNEVDQAIFATEKTIKETEGKGFDAERDAAQAALDELKKAQEDNNLDDMKAKLEALN EKAQALAVKLYEQAAAAQQAQAQAGAEGAQATGNAGDDVVDGEFTEK | (SEQ ID NO 137) LSDIDEVILVGGS TRI | 3 0 1 | 3 1 6 |
| | | | (SEQ ID NO 138) RIVNEPTAAALA YGLDKT | 1 4 1 | 1 5 8 |
| | *Streptoc occus interme dius* | (SEQ ID NO 22) MSKIIGIDLGTTNSAVAVLEGTESKIIANPEGNRTTPSVVSFKNGEIIVGDAAKRQAVTNPDTVISIKSKMGTSEKVSANGKEYTPQEISAMILQYLKGYAEEYLGEKVSKAVIT VPAYFNDAQRQATKDAGKIAGLEVERIVNEPTAAALAYGLDKTDKEEKILVFDLGGGTFDVSILELGDGVFDVLATAGDNKLGGDDFDQKIIDHMVAEFKKENGIDLSNDK MAVQRLKDAAEKAKKDLSGVTSTQISLPFITAGDAGPLHLEMTLTRAKFDDLTRDLVDRTKEPVRRALSDAGLSLSEIDEVILVGGSTRIPAVVEAVKAETGKEPNKSVNPDE VVAMGAAIQGGVITGDVKDVVLLDVTPLSLGIETMGGVFTKLIDRNTTIPTSKSQVFSTAADNQPAVDIHVLQGERPMAADNKTLGRFQLTDIPAAPRGIPQIEVTFDIDKN GIVSVKAKDLGTQKEQHIVIQSNSGLTDEEIDRMMKDAEANAEADKKRKEEVDLRNEVDQAIFATEKTIKETEGKGFDAERDAAQAALDELKKAQEDNNLDDMKAKLEAL NEKAQALAMKLYEQAAAAQQAQAGAEGANQANTGDNKGDDVVDGEFTEK | (SEQ ID NO 139) RIVNEPTAAALA YGLDKT | 1 4 1 | 1 5 8 |
| | | | (SEQ ID NO 140) VSKAVITVPAYF NDAQRQ | 1 0 9 | 1 2 6 |
| | *Streptoc occus thermo philus* | (SEQ ID NO 23) MSKIIGIDLGTTNSAVAVLEGTEPKIIANPEGNRTTPSVVSFKNGEIIVGDAAKRQAVTNPDTVISIKSKMGTSEKVSANGKEYTPQEISAMILQYLKSYAEEYLGEKVTKAVITV PAYFNDAQRQATKDAGKIAGLEVERIVNEPTAAALAYGLDKTDKEEKILVFDLGGGTFDVSILELGDGVFDVLATAGDNKLGGDDFDQKIIDYMVEEFKKENGIDLSTDKMA LQRLKDAAEKAKKDLSGVTSTQISLPFITAGEAGPLHLEMTLTRAKFDDLTRDLVERTKTPVRQALSDAGLSLSDIDEVILVGGSTRIPAVVEAVKAETGKEPNKSVNPDEVVA MGAAIQGGVISGDVKDVVLLDVTPLSLGIETMGGVFTKLIERNTTIPTSKSQVFSTAADNQPAVDIHVLQGERPMAADNKTLGRFQLTDIPAAPRGVPQIEVTFDIDKNGIV SVKAKDLGTQKEQTIVIQSNSGLTDEEIERMMKDAEANAEADAKRKAEVELRNEVDQAIFATEKTIKETEGKGFDTERDAAKSALDELKKAQESGDLDDMKAKLEALNEKA QALAVKLYEQAAAAQQAQAGAEGAQAADNSGDDVVDGEFTEK | (SEQ ID NO 141) LSDIDEVILVGGS TRI | 3 0 1 | 3 1 6 |
| | | | (SEQ ID NO 142) RIVNEPTAAALA YGLDKT | 1 4 1 | 1 5 8 |
| Adenosy Ihomocy steinase | *Micavib no aerugin osavoru s* | (SEQ ID NO 24) MSAVAAKAQTHDYAVKDITLADWGRKEIAIAETEMPGLMALRAEYGPKQILKGARIVGCLHMTIQTAVLIETLVALGAEVRWSSCNIFSTQDQAAAAIAAAGIPVFAWKG LSEEEFWDCIDRTITGPNGWTPNMILDDGGDLTIRMHEKFPDMMKDIKGLSEETTTGVHRLYEMAKKGTLKVPAINVNDSVTKSKFDNLYGCRESLVDGIKRATDVMLAG KIAVVAGFGDVGKGSAESLRSQGARVLVTEIDPICALQAAMEGYQVVTMEDAVSTADIFVTTTGNVDIITLDHMRQMKDRAIVCNIGHFDSEIQIEALRNYKWHNVKPQV DEVEFPDGKRLIVLAEGRLVNLGCATGHPSFVMSASFTNQVLAQIELWTNTSAYEAGKVYVLPKHLDEKVAALHLEKLGVKLTKLTKKQADYIGVAESGPYKPDHYRY | (SEQ ID NO 143) KGLSEETTTGVH RL | 1 6 0 | 1 7 3 |
| | | | (SEQ ID NO 144) KSKFDNLYGCRE | 1 9 5 | 2 0 5 |
| | | | (SEQ ID NO 145) KVPAINVNDSVT KS | 1 8 4 | 1 9 6 |
| | | | (SEQ ID NO 146) VPAINVNDSVTK S | 1 8 5 | 1 9 6 |
| | *Stenotr ophomo nas nitritire ducens* | (SEQ ID NO 25) MNAVAKTFSTEGDYKIRDITLADWGRKELDIAEHEMPGLMSIRRKYAAAKPLKGVRVTGSLHMTIQTAVLIETLKDIGGDVRWACNIFSTQDQAAAAIAATGTPVFAWKG ETLEEYWDCTLEALTFKSADGTLLGPELVVDDGGDVTLLIHKGYELENGSDWVNTASGSHEEQVIKNLLKRVHAERPGYWTRVVKDWKGVSEETTTGVHRLYQLAEAGTL LVPAINVNDSVTKSKFDNLYGCRESLADGLKRADVMLAGKVAVVCGYGDVGKGSAHSLRAYGARVIVTEIDPICALQAAMEGFEVNTIESTLGRGDIYVTTTGNKDIIRIEHL SAMKDQAIVCNIGHFDNEIQVDALVKFPGVQHINIKPQVDKYVFPNGNAIFLLAEGRLVNLGCATGHPSFVMSNSFANQTLAQIDLWANKDSYKADVYRLPKKLDEEVAR LHLEKIGVKLTTLTQEQADYLGVPVDGPFKPEHYRY | (SEQ ID NO 147) KSKFDNLYGCRE | 2 3 4 | 2 4 5 |
| | | | (SEQ ID NO 148) LVPAINVNDSVT KS | 2 2 2 | 2 3 5 |
| | | | (SEQ ID NO 149) VPAINVNDSVTK S | 2 2 3 | 2 3 5 |
| | *Tannere lla* | (SEQ ID NO 26) MAKETTNTLPYKVADITLADFGRKEIEIAEHEMPGLMALRKKYADKKPLQGARITGSLHMTIQTAVLIETLVDLGADVRWASCNIFSTQDHAAAAIAKAGVPVFAWKGETL EEYWWCTDQALRFPDGKGPNLIVDDGGDASLLFHQGYAAENDPRILKRKAANHEEQVILNLLESIHKENPQRWHNMLKEMKGVSEETTTGVHRLYQMMERGELLVPAI | (SEQ ID NO 150) KSKFDNLYGCRE | 2 2 7 | 2 3 8 |
| | *forsythi a* | NVNDSVTKSKFDNLYGCRESLADGIKRATDVMIAGKVVVVAGYGDVGKGCAHSMRSYGARVIVTEIDPICALQASMEGFEVTTMEEGIKEGDIFVTTTGNCDVITIDHMK AMKDQAIVCNIGHFDNEIQVEKLMTFPGIKHQNIKPQVDKYTFPDGHSIFLLAEGRLVNLGCATGHPSFVMSNSFTNQTLAQIDLWQKKYEVGVYCLPKHLDEEVARLHLE RVGVKLTKLTQKQADYIGVPVNGPFKSDIYRY | (SEQ ID NO 151) LVPAINVNDSVT KS | 2 1 5 | 2 2 8 |
| | | | (SEQ ID NO 152) VPAINVNDSVTK S | 2 1 6 | 2 2 8 |
| | *Tannere lla sp. HMT 286* | (SEQ ID NO 27) MVKESTEKLPYKVADINLADFGRKEIEIAEHEMPGLMALREKYAGKKPLAGARIMGSLHMTIQTAVLIETLVDLGADVRWASCNIFSTQDHAAAAIAAAGVPVFAWKGETL EEYWWCTNEALSFPGGKGPNLIVDDGGDASLLVHRGYAAENDPTLLDRKGSNKEEQVIIDLLKAVHAATPTRWHDMLKEMKGVSEETTTGVHRLYQMMERGELLVPAI NVNDSVTKSKFDNLYGCRESLADGIKRATDVMIAGKVVVVAGYGDVGKGCSHSMRSYGARVLVTEIDPICALQAAMEGFEVTTMEEAVKEGNIFVTTTGNCDVITIDHMS QMKDQAIVCNIGHFDNEIQVDQLENFPGIVRLNIKPQVDKYTFPDGHSIFLLAEGRLVNLGCATGHASFVMSNSFTNQTLAQLDLWQKDYKPGVYCLPKYLDEEVARLHLA RIGVKLSKLTQKQADYIGVPVDGPFKSEIYRY | (SEQ ID NO 153) KSKFDNLYGCRE | 2 2 7 | 2 3 8 |
| | | | (SEQ ID NO 154) LVPAINVNDSVT KS | 2 1 5 | 2 2 8 |
| | | | (SEQ ID NO 155) VPAINVNDSVTK S | 2 1 6 | 2 2 8 |
| | *Tannere lla sp. HMT 808* | (SEQ ID NO 28) MPLCGASLSPFPLSFLFYFRAQFIQTKQRNRIIMVKESTEKLPYKVADINLADFGRKEIEIAEHEMPGLMALREKYAGQKPLAGARITGSLHMTIQTAVLIETLLDLGADVRWA SCNIFSTQDHAAAAIAADGVPVFAWKGETLEEYWWCTNEALSFPGGKGPHLIVDDGGDASLLVHRGYAAENDPTLLDRKGSNREEQVIIDLLKRVHEATPTRWHNMLKE MKGVSEETTTGVHRLYQMMERGELLVPAINVNDSVTKSKFDNLYGCRESLADGIKRATDVMIAGKVVVVAGYGDVGKGCSHSMRSYGARVLITEIDPICALQAAMEGFE VTTMEEAVKEGNIFVTTTGNCDVITIDHMRQMKDQAIVCNIGHFDNEIQVDQLQNFPGIVRLNIKPQVDKYTFPDGHAIFLLAEGRLVNLGCATGHASFVMSNSFTNQTL AQIDLWQKDYKPGVYCLPKHLDEEVARLHLARIGVKLSKLTQKQADYIGVPVDGPFKSEIYRY | (SEQ ID NO 156) KSKFDNLYGCRE | 2 2 7 | 2 3 8 |
| | | | (SEQ ID NO 157) LVPAINVNDSVT KS | 2 1 5 | 2 2 8 |
| | | | (SEQ ID NO 158) VPAINVNDSVTK S | 2 1 6 | 2 2 8 |
| 2-keto-3-deoxy-L-fuconat e dehydro genase | *Pseudo monas luteola* | (SEQ ID NO 29) MSVAGKRVLVTAAAQGIGRASVLAFAEAGADVIAVDINATALAELPNVRTHVLDVTQPDAIQALASELGAIDVLFNCAGYVHSGTILDCDEDAWKFSFDLNVTAMYRMIR AFLPAMIENGGGSIINMSSVASSVKGVPNRFAYCASKAAVLGLTKSVAADFVAKGIRCNAICPGTVDSPSLRQRIAAQAQQEGRAEDEVYAAFVSRQPMGRIGKPEEIAAL ALYLGSDASGFTTGTINVIDGGWVA | (SEQ ID NO 159) NGGGSIINMSSV ASSVKG | 1 1 9 | 1 3 6 |
| | *Pseudo monas fluoresc ens* | (SEQ ID NO 30) MNLQNKRVLVTAAGQGIGLASAIAFARAGAEVFATDIDIHALAGIEEVTAMPLDVTSTTAISAACERIGGLDVLFNCAGYVHSGNILQCDEAAWARSMDLNVTATYRMIR AFLPGMLARGGGSIINMSSVASSVKGVPNRFAYATSKAAVVGLTKAVAIDFVSQGIRCNAICPGTVDSPSLRQRIADQAVQQGVDEAQVYRQFLDRQPMGRIGSTEEIAQ LAVYLGGDASAYTTGAIHVIDGGMSI | (SEQ ID NO 160) RGGGSIINMSSV ASSVKG | 1 1 9 | 1 3 6 |
| | *Klebsiell a aerogen es* | (SEQ ID NO 31) MELTGKRVLITAAGQGIGNSCARLFAAAGAEVIASDINIAGLSDIPGVQPLQLDVTRADAIAAAAETLGPLDVLFNCAGVVHSGSILECSEREWQFALDLNVTAMFHTIRAFL PAMIARGGGSIINMSSVASSVKGVPNRFAYSASKAAVIGLTRSVAADFVTQGIRCNAICPGTVDSPSLRGRIAEQAREQGLSEEAVYEAFVARQPIGRIGKPEEIAQLALYLAS DASAYTTGTVQIIDGGWSN | (SEQ ID NO 161) RGGGSIINMSSV ASSVKG | 1 1 9 | 1 3 6 |
| Formate tetrahyd rofolate ligase | *Cutibact erium granulo sum* | (SEQ ID NO 32) MKSDLQIAREARLEPIEHVANRASIPESYLEHYGRDVAKVSLDIEQAHEGRPRAKYVVVTAVTPTPLGEGKTTTAVGLAQGLEKIGRKTMLALRQPSMGPTFGIKGGA AGAGYSQVLPMEKLNLHLTGDFHAITAAHNLLAAMIDNHLHQGNALDIEPHSISWRRVVDVNDRALRNTIVGLGSRIDGVPRQTGFDITAASEVGVILSLATSLADLR ARLGRIVVAYTRSGEPVSAEDLHAAGAMTVIMKDAIKPNLLQTTENTPVLVHAGPFGNIATGNSSVIADQVGSRCGDFLLTEAGFGADMGAERFFNIKCRTSGLHPDA AVLVVTVRALKAHSGRYKVVAGKPLPPDMLADSPEDVLAGADNLRKHIEIVRTFGVSPIVALNVFPDDHDDEIDAVRRVAEQEGAHFAASTHVVDGGNGAMELARAVV DACDQPTDFRYTYDLADSLETKIEKIATEVYGADGIDISPQAAKDLAHFEELGYGRLPVVMAKTHLSLSHDPQLKGRPTGWRLPVREVRAAVGAGYVVYAICGNMRTMP GLGAHPAAERIDIDDNGQVVGLF | (SEQ ID NO 162) KYVVVTAVTPTP LGEGKT | 5 5 | 7 2 |
| | | | (SEQ ID NO 163) YVVVTAVTPTPL GEGKT | 5 6 | 7 2 |
| Thiored oxin 1 | *Cronob acter sakazak ii* | (SEQ ID NO 33) MSDKIIHLTDDSFDTDVLKADGLTLVDFWAEWCGPCKMIAPILDEIADEYQGKLTVAKLNIDQNPGTAPKYGIRGIPTLLLFKNGEVAATKVGALSKGQLKEFLDANLA | (SEQ ID NO 164) KLNIDQNPGTAP KY | 5 6 | 6 1 |
| | *Enterob acter cancero genus* | (SEQ ID NO 34) MSDKIIHLTDDSFDTDVLKADGLILVDFWAEWCGPCKMIAPILDEIADEYQGKLTVAKLNIDQNPGTAPKYGIRGIPTLLLFKNGEVAATKVGALSKGQLKEFLDANLA | (SEQ ID NO 165) KLNIDQNPGTAP KY | 5 8 | 7 1 |
| | *Enterob acter hormae chei* | (SEQ ID NO 35) MSDKIIHLTDDSFDTDVLKADGLILVDFWAEWCGPCKMIAPILDEIADEYQGKLTVAKLNIDQNPGTAPKYGIRGIPTLLLFKNGDVAATKVGALSKGQLKEFPDANPA | (SEQ ID NO 166) KLNIDQNPGTAP KY | 5 8 | 7 1 |
| | *Escheric hia coli* | (SEQ ID NO 36) MSDKIIHLTDDSFDTDVLKADGAILVDFWAEWCGPCKMIAPILDEIADEYQGKLTVAKLNIDQNPGTAPKYGIRGIPTLLLFKNGEVAATKVGALSKGQLKEFLDANLA | (SEQ ID NO 167) KLNIDQNPGTAP KY | 5 8 | 7 1 |
| | *Klebsiell a aerogen es* | (SEQ ID NO 37) MSDKIIHLTDDSFDTDVLKADGLTLVDFWAEWCGPCKMIAPILDEIAEEYQGKLTVAKLNIDQNPGTAPKYGIRGIPTLLLFKNGEVAATKVGALSKGQL | (SEQ ID NO 168) KLNIDQNPGTAP KY | 5 8 | 7 1 |
| | *Klebsiell a pneumo niae* | (SEQ ID NO 38) MSDKI IH LTDDSFDTDVLKADG LTLVDFWAEWCG PCKM I API LDEI AEEYQG KLTVAKLN I DQN PGTAPKYG I RG I PTLLLFKNG EVAATKVG ALSKGQLKEFLDAN LA | (SEQ ID NO 169) KLNIDQNPGTAP KY | 5 8 | 7 1 |
| | *Kluyver aascorba to* | (SEQ ID NO 39) MSDKI IH LTDDSFDTDVLKADG LTLVDFWAEWCG PCKM I API LDEI AEEYQG KLTVAKLN I DQN PGTAPKYG I RG I PTLLLFKNG EVAATKVG ALSKGQLKEFLDAN LA | (SEQ ID NO 170) | 5 8 | 7 1 |
| | | | KLNIDQNPGTAP KY | | |
| 2-methylci trate dehydra tase (2-methyl-trans-aconitat e forming) | *Prevotel la dentalis* | (SEQ ID NO 40) MILDRDMLRHFYASYASRVEAARQALRRPLTYAEKVLYAHLYDPRELRPFSRGEAYVNFRPDRVAMQDATAQMALLQFMNAGKDRVAVPASVHCDHLILADEGAEHDL PAARRQNREVYDFLRSVSAKYRIGFWAPGAGIIHQVVLENYAFPGGMMVGTDSHTPNAGGLGMVAVGVGGADAVDVLTAQPWELRMPRLIGVRLTGRLSGWAAPKD VILKILGMLTVKGGTNAILEYFGEGVQTLSATGRATICNMGAELGATCSLFPFDDNTGEYLRRTDRQEIATMACGVADELRADEAVTANPERFYDRVIHIDLSEVEPHLNGPF TPDAATPLSQMKAKGPAAGYPMTVEVGLVGSCTNSSYQDLARAASVARQAAEKHIPVRGQLIINPGSEQIRYTAERDGLLDDFRRIGALIMTNACGPCIGQWKRHTDDAE RKNAIVTSFNRNFRRRADGNPNTYAFIGSPEITVAQAIAGRLDFNPATDALTDEAGHSVMLDEPRGFDFPPRGFRAGADETLTGVEVLEREGLFIRPSGDHRDIAVEIAPDS NRLQRLEPFAPWDGKELVGMPLLIKTQGKCTTDHISMAGPWLRFRGHLQNISDNLLMGAVNAFNGESNRVKDQLDGQYKEVSAAAKDYKAHAMPSIVVAEDNYGEGS SREHAAMEPRYLNVRVILAKSFARIHETNLKKQGMLALTFVDKADYDRVQEDDRIDVTGLADMAPGSLLDVTLHHRDGSQERLKAAHTYNETQINWFRAGSALNFSSME R | (SEQ ID NO 171) MPSIVVAEDNY GEGSSRE | 6 4 2 | 6 5 9 |
| Acyl-CoA dehydro genase | *Ralstoni a pickettii* | (SEQ ID NO 41) MRTALREDHEQFRDQVRKFVQREIAPHHHEWEKVGCVPRETWLKAGREGLLLCAIPEQYGGGGGDFGHSIVVIEELMRVLATGPGFPLHSDIVAPYILAYGTEAQKESWL PPMARGEKIGAIAMTEPGIGSDLKSLRTTARRDGDSYVINGQKTFITNGANSDLVIVACKTDPAAGTRGISLIVVEAGTPGFTKGPLLEKIGLKAQDTAELFFDEVRVPAANLL GEEGKGFGYLMHQLAQERLMVSVRSPVVMETMLDETIAYTKSRKAFGQPVFDLQNTRFKLAEIKAKTVACRVFIDHCIALHFEKKLTSEMAAMAKLYSTEAQGQAVDELL QLHGGYGFMREYGIARAFVDSRVQRIYGGTSEIMKEIIGRSL | (SEQ ID NO 172) KAQDTAELFFDE VRV | 2 0 8 | 2 2 2 |
| Elongati on factor Tu | *Arachni a propioni ca* | (SEQ ID NO 42) MAKAKFERTKPHCNIGTIGHVDHGKTTLTAAITKVLHDKYPDLNELSPFDSIDKAPEERQRGITISIAHVEYQTEKRHYAHVDCPGHADYVKNMITGAAQMDGAILVVAAT DGPMAQTHEHILLARQVGVPAIVVALNKCDMIAEEDADLIDLVEMELREILSAQEFDGDNLPIVRVAAFPALNGDEKWAGTILELMDAVDEYIPQPERDTEKPFLMPVED VFTITGRGTVITGRIERGIVKTGDTVDLVGIRPEKQTTTVTGVEMFRKILDEGRAGENVGLLLRGTKKEDVERGMVVIKPGSTTPHTDFEATVYVLNKEEGGRHKPFFSNYSP QFYFRTTDVTGVVQLPEGKEMVMPGDNTDMTVHLNKPIAMEENLKFAIREGGRTVGAGKVTKIIK | (SEQ ID NO 173) RAGENVGLLLRG | 2 7 5 | 2 8 6 |
| | *Arsenici coccus bolidens is* | (SEQ ID NO 43) MAKAKFERTKPHVNIGTIGHIDHGKTTLTAAISKVLHDEYPDLNPQFAFEDIDKAPEERQRGITISIAHIEYQTESRHYAHVDCPGHADYVKNMITGAAQMDGAILVVAATD GPMPQTKEHVLLARQVGVPYIVVALNKTDMVDDEEILELVEMEVRELLTDYEFPGDDLPVVRISALKALEGDPEWTKTVVELMKTVDEYIPEPERDTDKPFMMPIEDVFTI TGRGTVVTGRIERGVLKVNEEVEIVGIREKTQKTTVTGIEMFRKLLDEGRAGENVGLLLRGLKREEVERGQVVCKPGSITPHTEFEASAYILSKDEGGRHTPFYDNYRPQFYFR TTDVTGVVHLPEGTEMIMPGDNTSMKVDLIQPIAMEEGLKFNIREGGRTVGAGQVTKIIK | (SEQ ID NO 174) RAGENVGLLLRG | 2 7 3 | 2 8 4 |
| | *Chlamy dia pneumo niae* | (SEQ ID NO 44) MSKETFQRNKPHINIGTIGHVDHGKTTLTAAITRALSGDGLASFRDYSSIDNTPEEKARGITINASHVEYETPNRHYAHVDCPGHADYVKNMITGAAQMDGAILVVSATDG AMPQTKEHILLARQVGVPYIVVFLNKVDMISQEDAELIDLVEMELSELLEEKGYKGCPIIRGSALKALEGDANYIEKVRELMQAVDDNIPTPEREIDKPFLMPIEDVFSISGRGT VVTGRIERGIVKVSDKVQLVGLGETKETIVTGVEMFRKELPEGRAGENVGLLLRGIGKNDVERGMVVCQPNSVKPHTKFKSAVYVLQKEEGGRHKPFFSGYRPQFFFRTTD VTGVVTLPEGTEMVMPGDNVELDVELIGTVALEEGMRFAIREGGRTIGAGTISKINA | (SEQ ID NO 175) RAGENVGLLLRG | 2 7 0 | 2 8 1 |
| | *Cutibact erium avidum* | (SEQ ID NO 45) MAKAKFERTKPHCNIGTIGHIDHGKTTLTAAISKVLHDKYPELNEESPFDQIDKAPEERQRGITISIAHIEYQTEKRHYAHVDCPGHADYVKNMITGAAQMDGAILVVAATD GPMPQTREHVLLARQVGVPAIVVALNKCDMVDDEELIELVEMEVRELLTSQDFDGDNCPIVRISAFQALQGDEKWTQSILDLMDAVDEYVPQPERDLDKPFLMPIEDVFT ITGRGTVVTGRVERGVVKTSEEVEIVGIHDKIQKTTVTGVEMFRKILDEGRAGENVGILLRGTKKEDVVRGMVLCKPGSTTPHTDFEGQVYVLKKDEGGRHKPFFSHYSPQF YFRTTDVTGTVELPEGTEMVMPGDNTDMTVHLIHPIAMEEQLKFAIREGGRTVGAGRVTKIIK | (SEQ ID NO 176) RAGENVGILLRG | 2 7 3 | 2 8 4 |
| | *Janibact er indicus* | (SEQ ID NO 46) MAKAKFERSKPHVNIGTIGHIDHGKTTLTAAISRVLHDKLPDLNEASAFDTIDKAPEEKQRGITISIAHIEYQTESRHYAHVDCPGHADYVKNMITGAAQMDGAILVVAATD GPMPQTKEHVLLARQVGVPYIVVALNKADMVDDEEIMELVEMEVRELLSSYEFPGDDVPVVKVSALKALEGDAQWGESIMELMTAVDEYIPEPERDLDKPFMMPVEDV FTITGRGTVVTGRIERGILKVNEEVEIVGIREEKTTTTVTGIEMFRKLLDEGRAGENVGLLLRGTKREDVERGQVICKPGSITPHTEFEAQVYILSKEEGGRHTPFYDSYRPQFYF RTTDVTGVVSLPEGTEMVMPGDNTDMKVELIQPIAMEEGLKFNIREGGRTVGAGRVTKIIK | (SEQ ID NO 177) RAGENVGLLLRG | 2 7 3 | 2 8 4 |
| | *Kytococ cus sedenta rius* | (SEQ ID NO 47) MAKAKFERSKPHVNIGTIGHIDHGKTTLTAAISKVLHDRYPELNEASPFDTIDKAPEEKQRGITISISHIEYQTEERHYAHVDCPGHADYVKNMITGAAQMDGAILVVAATDG PMPQTKEHVLLARQVGVPYICVALNKSDMVDDEEIMELVEMEVRELLSEYDFPGDDVPVVQVSALKALEGDEKWGDQIIELMTEVDKAIPEPERDIDKPFLMPVEDVFTIT GRGTVVTGRIERGILNVNDEIEIVGIQEEKQSTTVTGIEMFRKLLDEGRAGENVGLLLRGTKREDVERGQVICKPGSITPHKKFEGSVYILSKDEGGRHTPFYDNYRPQFYFRT TDVTGVVDLPEGTEMVMPGDNTEMSVELIQPVAMEDGLKFAIREGGRTVGAGQVTKILD | (SEQ ID NO 178) RAGENVGLLLRG | 2 7 3 | 2 8 4 |
| | *Pseudo monas aerugin 050* | (SEQ ID NO 48) MAKEKFERNKPHVNVGTIGHVDHGKTTLTAALTKVCSDTWGGSARAFDQIDNAPEEKARGITINTSHVEYDSAVRHYAHVDCPGHADYVKNMITGAAQMDGAILVCSA ADGPMPQTREHILLSRQVGVPYIVVFLNKADMVDDAELLELVEMEVRDLLNTYDFPGDDTPIIIGSALMALEGKDDNGIGVSAVQKLVETLDSYIPEPVRAIDQPFLMPIED VFSISGRGTVVTGRVERGIIKVQEEVEIVGIKATTKTTCTGVEMFRKLLDEGRAGENVGILLRGTKREDVERGQVLAKPGTIKPHTKFECEVYVLSKEEGGRHTPFFKGYRPQF YFRTTDVTGNCELPEGVEMVMPGDNIKMVVTLIAPIAMEDGLRFAIREGGRTVGAGVVAKIIE | (SEQ ID NO 179) RAGENVGILLRG | 2 7 3 | 2 8 4 |
| Phospha te ABC transpor ter substrat e-binding protein PstS | *Rhizobi* um *rhizoge nes* | (SEQ ID NO 49) MTAKKEMLKQADVPFAVALFKVRQGGFTMNALKSSIAALVASVTFAGAAMARDQIQISGSSTMFPYAKIVAETFGETFTNYKTPVVESGGSGAGLKEFCKGVGPDSIDIAN SSRPIKGSEVDACKAAGVADIQQVKIGYDGIVFAYDKSNPDIRFVPTDLYKALAAEVVVNGKLVANPYKKWSEINPALPDFEIAAYIPGEKHGTREVFEEKVLAAGCKDSGAL DVIKAAVPDAGKQAEKCIAVRKDGVAVDIDGDYPETLARINANNHGVGVFGLYFYQNNADKLKVATVGGIVPSGQTIADGTYPVSRPLFFYVKKAHLGVIPGLKEYVDFFTS DQMIGPDSPLAEYGLVPAPDAERDQIRKDVANGKIM | (SEQ ID NO 180) KTPVVESGGSG AGLKE | | |
| ATP synthas e subunit beta | *Rhodob acter capsula tus* | (SEQ ID NO 50) MASKGKVTQVIGAVVDVQFEDGLPAILNALETTNNGKRLVLEVAQHLGENTVRTIAMDATEGLVRGAAVSDTGGPITVPVGNATLGRILNVIGEPVDERGDVSKAEARAI HQPAPDFAAQSTESQILVTGIKVIDLLAPYSKGGKIGLFGGAGVGKTVLIMELINNIAKVHSGFSVFAGVGERTREGNDLYHEMIESGVINLEKLEESKVALVYGQMNEPPGA RARVALTGLTLAEQFRDQSGTDVLFFVDNIFRFTQAGSEVSALLGRIPSAVGYQPTLATDMGALQERITSTKAGSITSVQAIYVPADDLTDPAPATSFAHLDATTVLSRAISEL GIYPAVDPLDSTSRILDPQVVGEEHYQVARDVQGMLQRYKSLQDIIAILGMDELSEEDKLTVARARKIQRFLSQPFDVAKVFTGSDGVQVPLEDTIKSFKAVVAGEYDHLPE AAFYMVGGIDDVIAKAQRLAAAA | (SEQ ID NO 181) GIYPAVDPLDST SRI | 3 3 8 | 3 5 2 |
| | | | (SEQ ID NO 182) KIGLFGGAGVGK T | 1 4 5 | 1 5 7 |
| | | | (SEQ ID NO 183) KVALVYGQMNE PPGARA | 2 0 9 | 2 2 5 |
| | | | (SEQ ID NO 184) RFTQAGSEVSAL LGRI | 2 5 5 | 2 7 0 |
| | | | (SEQ ID NO 185) RIPSAVGYQPTL ATDMGALQERI | 2 6 9 | 2 9 1 |
| | | | (SEQ ID NO 186) YPAVDPLDSTSRI | 3 4 0 | 3 5 2 |

The following table 3 provides the NCBI tax ID of each of the microorganisms herein mentioned.

**Table 3**

| **Microorganisms** | **NCBI taxID** |
|---|---|
| *Actinomyces massiliensis* | 461393 |
| *Actinomyces sp. HMT 897* | 2789424 |
| *Aggregatibacter actinomycetemcomitans* | 714 |
| *Aggregatibacter paraphrophilus* | 732 |
| *Aggregatibacter sp. HMT 458* | 712148 |
| *Rhizobium rhizogenes* | 358 |
| *Arachnia propionica* | 1750 |
| *Arsenicicoccus bolidensis* | 229480 |
| *Campylobacter sputorum* | 206 |
| *Campylobacter ureolyticus* | 827 |
| *Chlamydia pneumoniae* | 83558 |
| *Corynebacterium coyleae* | 53374 |
| *Cronobacter sakazakii* | 28141 |
| *Cutibacterium avidum* | 33010 |
| *Cutibacterium granulosum* | 33011 |
| *Cutibacterium sp. HMT 193* | 671223 |
| *Enterobacter cancerogenus* | 69218 |
| *Enterobacter hormaechei* | 158836 |
| *Enterococcus faecalis* | 1351 |
| *Escherichia coli* | 562 |
| *Haemophilus parainfluenzae* | 729 |
| *Haemophilus pittmaniae* | 249188 |
| *Janibacter indicus* | 857417 |
| *Klebsiella aerogenes* | 548 |
| *Klebsiella pneumoniae* | 573 |
| *Kluyvera ascorbata* | 51288 |
| *Kytococcus sedentarius* | 1276 |
| *Lactiplantibacillus plantarum* | 1590 |
| Lactococcus *lactis* | 1358 |
| *Lancefieldella rimae* | 1383 |
| *Micavibrio aeruginosavorus* | 349221 |
| *Mycolicibacterium neoaurum* | 1795 |
| *Parascardovia denticolens* | 78258 |
| *Prevotella dentalis* | 52227 |
| *Proteus mirabilis* | 584 |
| *Pseudomonas aeruginosa* | 287 |
| *Pseudomonas fluorescens* | 294 |
| *Pseudomonas luteola* | 47886 |
| *Ralstonia pickettii* | 329 |
| *Rhodobacter capsulatus* | 1061 |
| *Scardovia inopinata* | 78259 |
| *Scardovia wiggsiae* | 230143 |
| *Stenotrophomonas nitritireducens* | 83617 |
| *Streptococcus cristatus* | 45634 |
| *Streptococcus intermedius* | 1338 |
| *Streptococcus thermophilus* | 1308 |
| *Tannerella forsythia* | 28112 |
| *Tannerella sp. HMT 286* | 712710 |
| *Tannerella sp. HMT 808* | 712711 |

In a prefered aspect of the invention, the OMEVP is present in the following normalized amounts obtained from brain tissue samples:
1) Chaperone protein DnaK ratio range: [0.29-1.61]
2) Adenosylhomocysteinase ratio range: [0.26-1.05]
3) 2-keto-3-deoxy-L-fuconate dehydrogenase ratio range: [0-2.57]
4) Formate-tetrahydrofolate ligase ratio range: [0-0.35]
5) Thioredoxin 1 ratio range: [0-0.36]
6) 2-methylcitrate dehydratase ratio range: [1-1.67]*
7) Acyl-CoA dehydrogenase ratio range: [1-5.44]*
8) Phosphate-binding protein ratio range: [1-2.56]*
9) Elongation factor Tu ratio range: [0-2.51]
10) ATP synthase subunit beta ratio range: [0-2.5] ;
wherein the value of 1 is excluded and " * " means that an Haldane-Anscombe correction is applied.

In a theoreticaly valid extrapolation, these ratios are applicable also in other biological samples of the subject.

*In these cases the control value was zero so the Haldane-anscombe correction has been applied and 0.5 was added to each value before doing the normalization. This means that in those cases where the control had a value other than 0 could be normalised. However, in those cases in which the control has a value of 0 and therefore the Haldane-anscome correction had to be applied (giving a value of 0.5 to the control and adding that same value to all the others to be able to calculate the normalisation and the ratios).

The "value 1 is excluded" is because in the ratios that are considered, value 1 indicates that the OMEVP value is the same in the control as in the other conditions and therefore there is no difference. In other words, it is a value that is not indicative of disease. The ratios are made in such a way that 1 indicates the level of the controls for a specific OMEVP, and values below it indicate that the OMEVP is less expressed in the cases than in the controls, while levels above indicate that the OMEVP is more expressed in the cases than in the controls.

Table 4 below shows the OMEVP that play a role in the development of mixed dementias, the microorganisms that can produce them and the ratios calculated between mixed dementia patients and cognitively healthy age-matched controls, for each microorganism.

**Table 4. Correlation between the OMEVP, the microorganisms that produce said OMEVP and the ratios calculated between mixed dementia patients and cognitively healthy age-matched controls, for each microorganism.**

| OMEVP | **Microorganism strain** | **Ratios compared to age-matched controls** |
|---|---|---|
| Chaperone protein DnaK | *Actinomyces massiliensis* | [1,22-2,56] |
| | *Actinomyces sp._HMT_897* | [1,44-3] |
| | *Aggregatibacter actinomycetemcomitans* | [0-35,67] |
| | *Aggregatibacter paraphrophilus* | [0,22-1,78] |
| | *Aggregatibacter sp. HMT 458* | [0,3-1,78] |
| | *Campylobacter sputorum* | [0,2-1,36] |
| | *Campylobacter ureolyticus* | [0,21-1,42] |
| | *Corynebacterium coyleae* | [1,44-3] |
| | Cutibacterium sp._HMT_193 | [1,44-2,56] |
| | *Enterococcus faecalis* | [0-3,10] |
| | *Haemophilus parainfluenzae* | [0,22-1,72] |
| | *Haemophilus pittmaniae* | [0,22-1,85] |
| | *Lactiplantibacillus plantarum* | [1-1,89] |
| | *Lactococcus lactis* | [0-3,10] |
| | *Lancefieldella rimae* | [0,16-1,36] |
| | *Mycolicibacterium neoaurum* | [0,08-2,33] |
| | *Parascardovia denticolens* | [0-0,8] |
| | *Proteus mirabilis* | [0,10-0,96] |
| | *Scardovia inopinata* | [0-0,8] |
| | *Scardovia wiggsiae* | [0-0,60] |
| | *Streptococcus cristatus* | [2,67-12] |
| | *Streptococcus intermedius* | [1,67-4,11] |
| | *Streptococcus thermophilus* | [1,44-2,11] |
| Adenosylhomocysteinase | *Micavibrio aeruginosavorus* | [0,21-0,94] |
| | *Stenotrophomonas nitritireducens* | [0,25-0,95] |
| | *Tannerella forsythia* | [0,25-0,95] |
| | *Tannerella sp. HMT 286* | [0,25-0,95] |
| | *Tannerella sp. HMT 808* | [0,25-0,95] |
| 2-keto-3-deoxy-L-fuconate dehydrogenase | *Pseudomonas luteola* | [0-2,14] |
| | *Pseudomonas fluorescens* | [0-2,14] |
| | *Klebsiella aerogenes* | [0-2,14] |
| Formate--tetrahydrofolate ligase | *Cutibacterium granulosum* | [0-0.35] |
| Thioredoxin 1 | *Cronobacter sakazakii* | [0-0,36] |
| | *Enterobacter cancerogenus* | [0-0,36] |
| | *Enterobacter hormaechei* | [0-0,36] |
| | *Escherichia coli* | [0-0,36] |
| | *Klebsiella aerogenes* | [0-0,36] |
| | *Klebsiella pneumoniae* | [0-0,36] |
| | *Kluyvera ascorbata* | [0-0,36] |
| Phosphate-binding protein | *Rhizobium rhizogenes* | [1-2.56] |
| 2-methylcitrate dehydratase (2-methyl-trans-aconitate forming) | *Prevotella dentalis* | [1-1.67] |
| Acyl-CoA dehydrogenase | *Ralstonia pickettii* | [1-5.44] |
| Elongation factor Tu | *Arachnia propionica* | [0-2,50] |
| | *Arsenicicoccus bolidensis* | [0-2,50] |
| | *Chlamydia pneumoniae* | [0-2,50] |
| | *Cutibacterium avidum* | [0-2,50] |
| | *Janibacter indicus* | [0-2,50] |
| | *Kytococcus sedentarius* | [0-2,50] |
| | *Pseudomonas aeruginosa* | [0-2,94] |
| ATP synthase subunit beta | *Rhodobacter capsulatus* | [0-2.5] |

Another aspect of the present invention is the use of a bacterial profile of the OM of a subject in the diagnosis or prognosis of mixed dementias, the bacterial profile comprising a cluster of bacteria producing at least one of the OMEVP of the invention, or combinations thereof.

The tables below shows the different clusters of OMEVP as described, with their related microorganisms.

**Table 5. Cluster of proteins FHS, trxA and AHCY, and the microorganisms that produce them, that show similar behaviour in all study groups analized in example 1.**

| OMEVP | **Microorganism strain** |
|---|---|
| Formate--tetrahydrofolate ligase | *Cutibacterium granulosum* |
| Thioredoxin 1 | *Cronobacter sakazakii* |
| | *Enterobacter cancerogenus* |
| | *Enterobacter hormaechei* |
| | *Escherichia coli* |
| | *Klebsiella aerogenes* |
| | *Klebsiella pneumoniae* |
| | *Kluyvera ascorbata* |
| Adenosylhomocysteinase | *Micavibrio aeruginosavorus* |
| | *Stenotrophomonas nitritireducens* |
| | *Tannerella forsythia* |
| | *Tannerella sp. HMT 286* |
| | *Tannerella sp. HMT 808* |

**Table 6. Cluster of proteins SDR, DNAK and FHS, and the microorganisms that produce them, that show similar behaviour in all AD conditions.**

| OMEVP | **Microorganism strain** |
|---|---|
| 2-keto-3-deoxy-L-fuconate dehydrogenase | *Pseudomonas luteola* |
| | *Pseudomonas fluorescens* |
| | *Klebsiella aerogenes* |
| | *Aggregatibacter actinomycetemcomitans* |
| | *Aggregatibacter paraphrophilus* |
| | *Aggregatibacter sp. HMT 458* |
| | *Campylobacter sputorum* |
| | *Campylobacter ureolyticus* |
| | *Enterococcus faecalis* |
| Chaperone protein DnaK | *Haemophilus parainfluenzae* |
| | *Haemophilus pittmaniae* |
| | *Lactococcus lactis* |
| | *Lancefieldella rimae* |
| | *Mycolicibacterium neoaurum* |
| | *Parascardovia denticolens* |
| | *Proteus mirabilis* |
| | *Scardovia inopinata* |
| | *Scardovia wiggsiae* |
| | *Streptococcus intermedius* |
| Formate--tetrahydrofolate ligase | *Cutibacterium granulosum* |

**Table 7. Cluster of proteins DNAK, AHCY and FHS, and the microorganisms that produce them, that show similar behaviour in AD3 stage of AD subjects.**

| OMEVP | **Microorganism strain** |
|---|---|
| Chaperone protein DnaK | *Aggregatibacter actinomycetemcomitans* |
| | *Aggregatibacter paraphrophilus* |
| | *Aggregatibacter sp. HMT 458* |
| | *Haemophilus parainfluenzae* |
| | *Haemophilus pittmaniae* |
| | *Proteus mirabilis* |
| | *Scardovia wiggsiae* |
| Adenosylhomocysteinase | *Micavibrio aeruginosavorus* |
| | *Stenotrophomonas nitritireducens* |
| | *Tannerella forsythia* |
| | *Tannerella sp. HMT 286* |
| | *Tannerella sp. HMT 808* |
| Formate--tetrahydrofolate ligase | *Cutibacterium granulosum* |

**Table 8. Cluster of proteins SDR and DNAK, and the microorganisms that produce them, that show similar behaviour in AD4 stage of AD subjetcs.**

| OMEVP | **Microorganism strain** |
|---|---|
| 2-keto-3-deoxy-L-fuconate dehydrogenase | *Pseudomonas luteola* |
| | *Pseudomonas fluorescens* |
| | *Klebsiella aerogenes* |
| | *Aggregatibacter actinomycetemcomitans* |
| | *Aggregatibacter paraphrophilus* |
| | *Aggregatibacter sp. HMT 458* |
| | *Campylobacter sputorum* |
| | *Campylobacter ureolyticus* |
| | *Enterococcus faecalis* |
| | *Haemophilus parainfluenzae* |
| Chaperone protein DnaK | *Haemophilus pittmaniae* |
| | *Lactococcus lactis* |
| | *Lancefieldella rimae* |
| | *Mycolicibacterium neoaurum* |
| | *Parascardovia denticolens* |
| | *Proteus mirabilis* |
| | *Scardovia inopinata* |
| | *Scardovia wiggsiae* |
| | *Streptococcus intermedius* |

**Table 9. Cluster of proteins PsTs and acnA, and the microorganisms that produce them, that show similar behaviour in AD6 stage of AD subjetcs.**

| OMEVP | **Microorganism strain** |
|---|---|
| Phosphate-binding protein | *Rhizobium rhizogenes* |
| 2-methylcitrate dehydratase | *Prevotella dentalis* |

**Table 10. Cluster of proteins DNAK and AHCY, and the microorganisms that produce them, that show similar behaviour in all study groups analized in example 1.**

| OMEVP | **Microorganism strain** |
|---|---|
| Chaperone protein DnaK | *Aggregatibacter actinomycetemcomitans* |
| | *Aggregatibacter paraphrophilus* |
| | *Aggregatibacter sp. HMT 458* |
| | *Haemophilus parainfluenzae* |
| | *Haemophilus pittmaniae* |
| | *Proteus mirabilis* |
| | *Scardovia wiggsiae* |
| Adenosylhomocysteinase | *Micavibrio aeruginosavorus* |
| | *Stenotrophomonas nitritireducens* |
| | *Tannerella forsythia* |
| | *Tannerella sp. HMT 286* |
| | *Tannerella sp. HMT 808* |

**Table 11. Cluster of proteins AHCY and tuF, and the microorganisms that produce them, that show similar behaviour in VaD subjetcs.**

| OMEVP | **Microorganism strain** |
|---|---|
| Adenosylhomocysteinase | *Micavibrio aeruginosavorus* |
| | *Stenotrophomonas nitritireducens* |
| | *Tannerella forsythia* |
| | *Tannerella sp.HMT 286* |
| | *Tannerella sp.HMT 808* |
| Elongation factor Tu | *Arachnia propionica* |
| | *Arsenicicoccus bolidensis* |
| | *Chlamydia pneumoniae* |
| | *Cutibacterium avidum* |
| | *Janibacter indicus* |
| | *Kytococcus sedentarius* |
| | *Pseudomonas aeruginosa* |

**Table 12. Cluster of proteins DNAK and ATPD, and the microorganisms that produce them, that show similar behaviour in VaD subjetcs.**

| OMEVP | **Microorganism strain** |
|---|---|
| Chaperone protein DnaK | *Aggregatibacter actinomycetemcomitans* |
| | *Aggregatibacter paraphrophilus* |
| | *Aggregatibacter sp. HMT 458* |
| | *Haemophilus parainfluenzae* |
| | *Haemophilus pittmaniae* |
| | *Lactiplantibacillus plantarum* |
| | *Proteus mirabilis* |
| | *Scardovia wiggsiae* |
| ATP synthase subunit beta | *Rhodobacter capsulatus* |

Therefore, being different clusters related to different conditions/different stages of AD, the main advantage of the OMEVP is that the are used to identify each stage of the disease and therefore its prognosis.

In a another aspect, the present invention referes to a method of diagnosis or prognosis of mixed dementia, comprising:
- assessing the level of an OMEVP in a biological sample, preferably brain tissue, blood, saliva or plasma, obtained from a subject, and
- determining whether said level is within the range as defined in the first aspect of the present invention;
wherein a value within the range of normalized values above or below the normalized value with respect to a healthy subject is indicative of the prognosis of mixed dementia.

In a preferred embodiment the method of diagnosis of mixed dementia is assessed in a biological sample which is selected from the group consisting of saliva, mucus, gum plaque, urine, blood or tear liquid.

In a preferred embodiment the method of prognosis of mixed dementia the subject is a mammal and more preferred is a human.

In a preferred embodiment the method of diagnosis of mixed dementia is assessed in a biological sample which is selected from the group consisting of cerebrospinal fluid or brain tissues and subject is a dead mammal and more preferred is a dead human.

In a preferred embodiment, the method of diagnosis of mixed dementia comprises determining whether said level is within the range as defined in the normalized amounts of OMEVP obtained from brain tissue samples, disclosed previously in the present document.

In a another aspect, the present invention referes to a method of prognosis of mixed dementia, comprising:
- assessing the level of an OMEVP in a biological sample, preferred brain tissue, obtained from a subject, and

determining whether said level is within the range as defined in the normalized amounts of OMEVP obtained from brain tissue samples, disclosed previously in the present document,
wherein a value within the range of normalized values with respect to a healthy subject is indicative of the prognosis of mixed dementia.

In a preferred embodiment the method of prognosis of mixed dementia is assessed in a biological sample which is selected from the group consisting of saliva, mucus, gum plaque, urine, blood or tear liquid.

In another preferred embodiment the method of prognosis of mixed dementia the subject is a mammal and more preferred is a human.

Finally, another embodiment of the present invention is a system for the prediction of the evolution of a subject to mixed dementia comprising data processing means, said data processing means been configured:
- to assess in a biological sample obtained from said subject the level of an OMEVP as defined in the present invention;
- to determine whether said level is within the range as defined as defined in the normalized amounts of OMEVP obtained from brain tissue samples, disclosed previously in the present documen; and
- to predict the functional outcome of mixed dementia in the subject evaluating the result of the previous determination,
wherein a value above or below the normalized value with respect to a healthy subject is indicative of the diagnosis or the prognosis of the disease.

In the context of this invention, the term "healthy subject" refers to an individual who falls within the age range associated with a high prevalence of dementia risk but does not exhibit any clinical symptoms of dementia.

In the present invention, the term "diagnosis" refers to the clinical identification of molecular signs linked to the presence of aging-associated cortical dementia.

In the present invention, the term "prognosis" is refers to the clinical identification of moleular signs linked to the progression and clinical stratification of subjects with aging-associated cortical dementia.

In a preferred embodiment, the sample us a being said sample a biological sample. In preferred embodiment, the sample is from a mammal, in a more preferred embodiment the sample is form a human.

### BRIEF DESCRIPTION OF THE FIGURES

To complement the description being made and in order to aid in a better understanding of the features of the invention, there is attached as an integral part of said description, a set of drawings wherein, with illustrative and non-limiting character, the following has been represented.

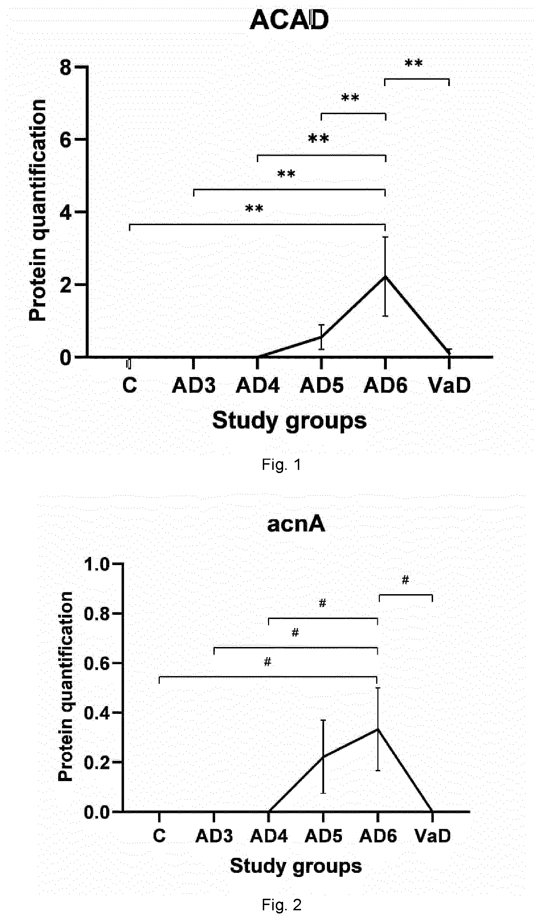

Fig 1. Characterization of brain-derived OMEVP ACAD levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 2. Characterization of brain-derived OMEVP acnA levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.

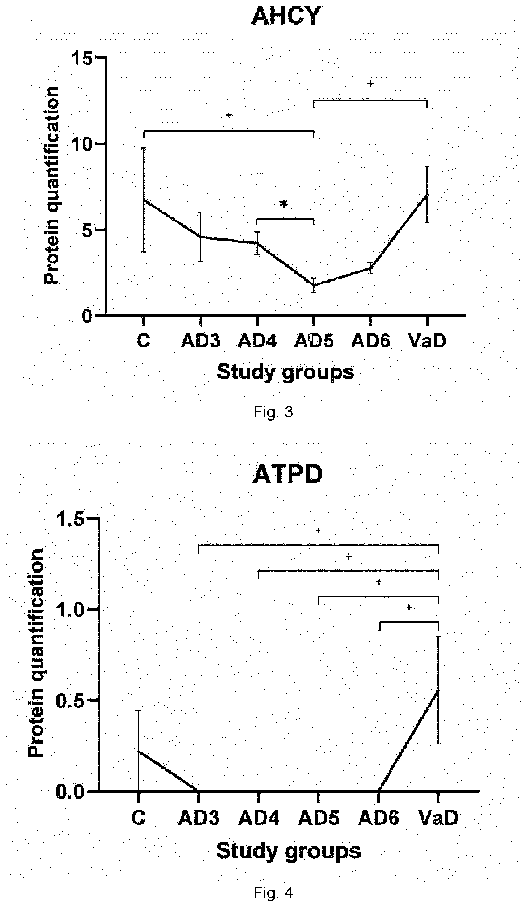

Fig 3. Characterization of brain-derived OMEVP AHCY levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 4. Characterization of brain-derived OMEVP ATPD levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.

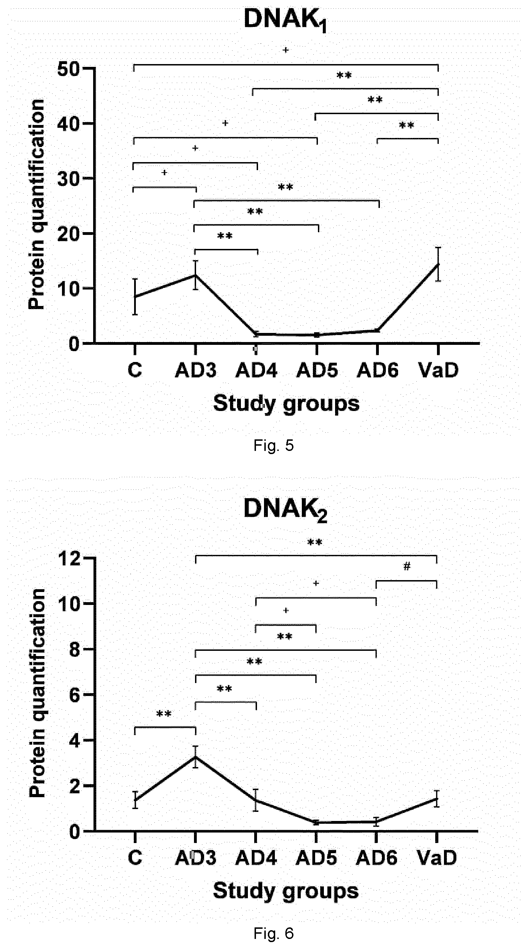

Fig 5. Characterization of brain-derived OMEVP DNAK, levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 6. Characterization of brain-derived OMEVP DNAK₂ levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.

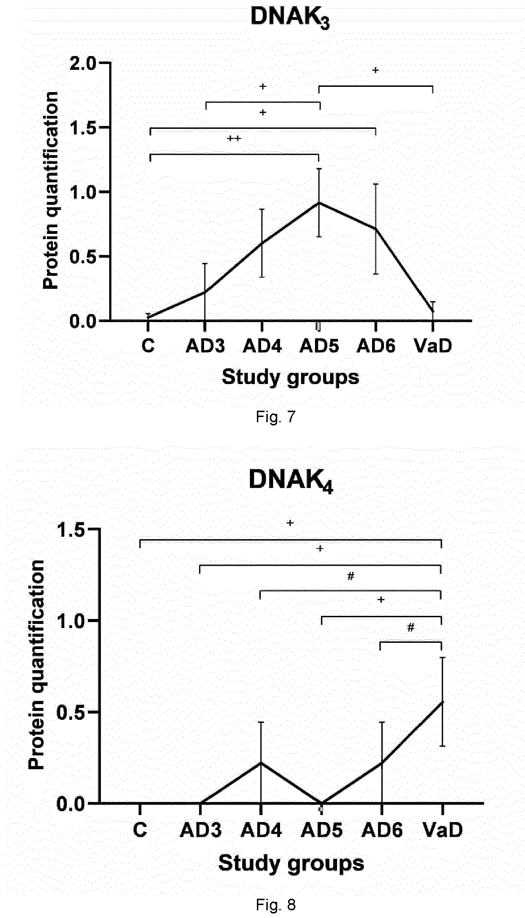

Fig 7. Characterization of brain-derived OMEVP DNAK₃ levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 8. Characterization of brain-derived OMEVP DNAK₄ levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.

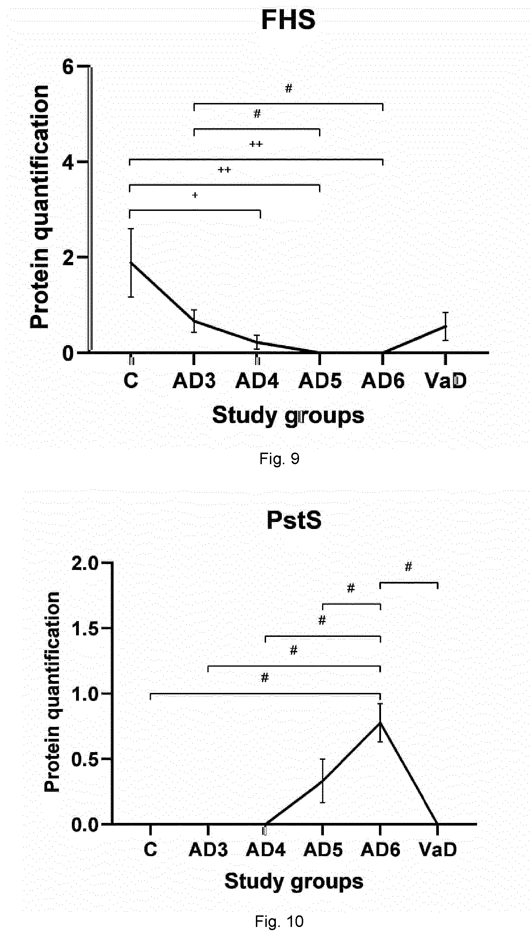

Fig 9. Characterization of brain-derived OMEVP FHS levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 10. Characterization of brain-derived OMEVP PstS levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.

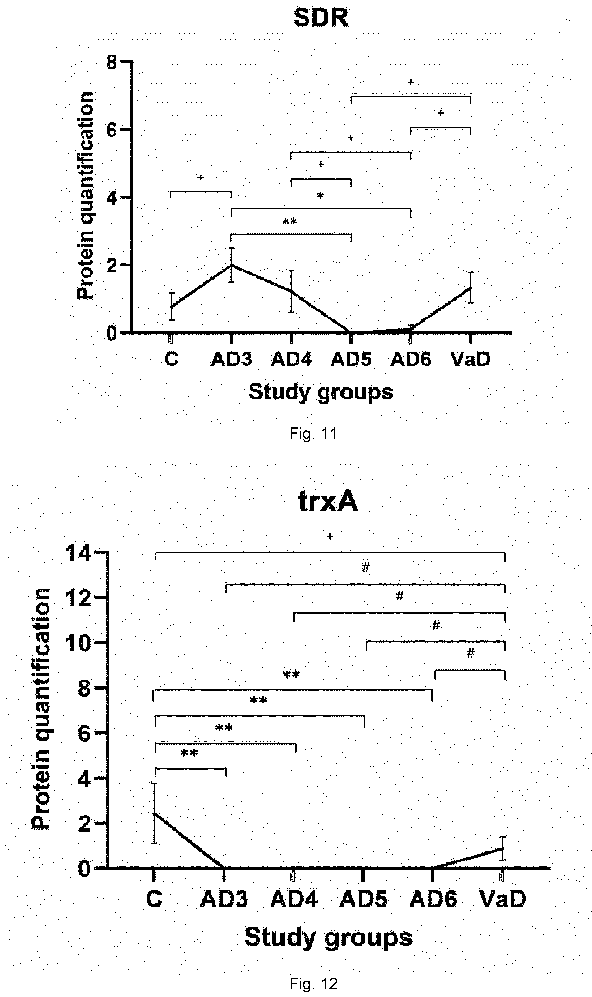

Fig 11. Characterization of brain-derived OMEVP SDR levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 12. Characterization of brain-derived OMEVP trxA levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.

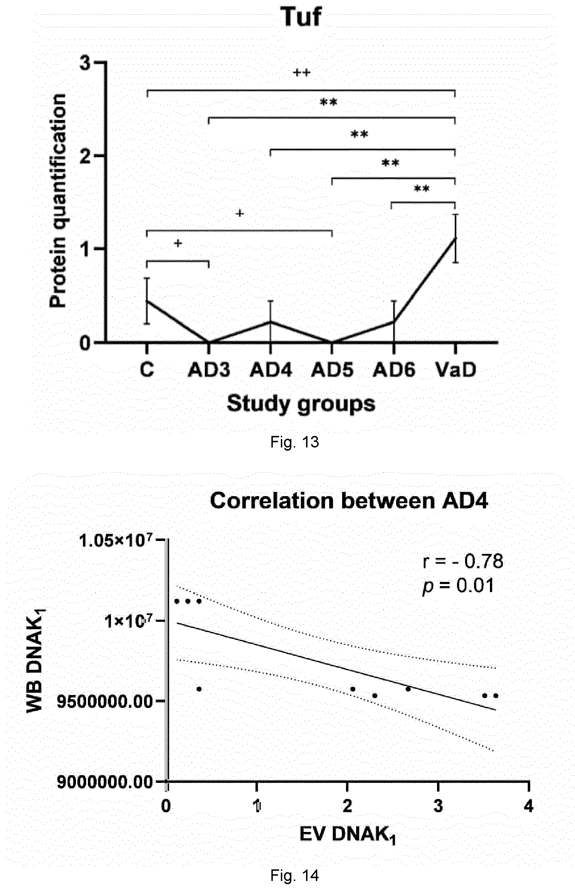

Fig 13. Characterization of brain-derived OMEVP Tuf levels with significant differences between mixed dementia (AD or VaD) groups and age-matched controls. OMdP in bEVs levels from subjects with AD or VaD and age-matched controls. Average levels and standard error of the mean are represented. Significant differences were analyzed by two-way ANOVA with Bonferroni correction for multiple comparisons. *Significant differences P < 0.05. **Significant differences P < 0.01. ⁺Significant differences determined by ANOVA with Fisher's LSD P < 0.05. ⁺⁺Significant differences determined by ANOVA with Fisher's LSD P < 0.01. ^{#}Significant differences determined by nonparametric one-way ANOVA on ranks P < 0.05.
Fig 14. Correlation between brain-derived OMEVP and WB of DNAK, proteins in AD4 study group. Dotted lines show the 95% confidence score.

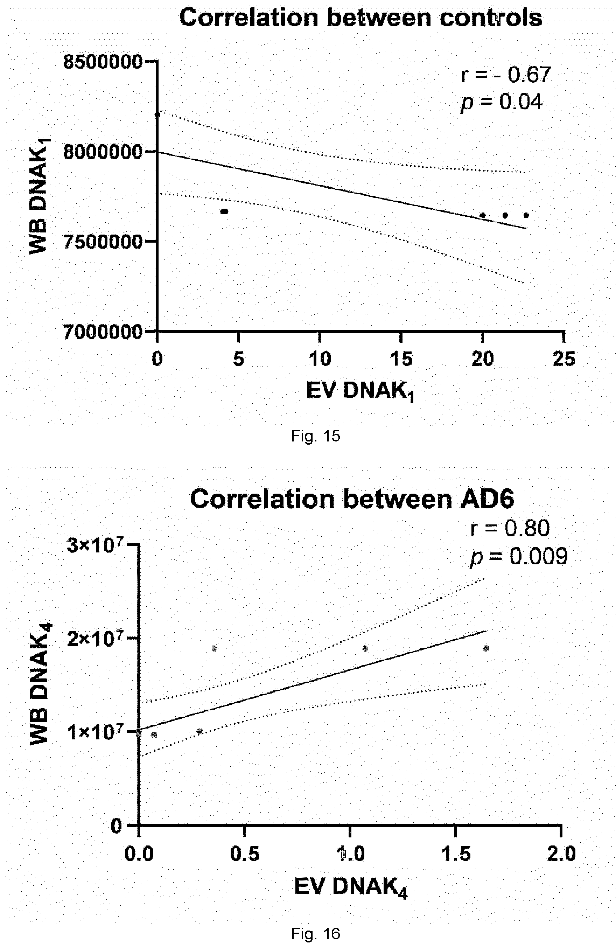

Fig 15. Correlation between brain-derived OMEVP and WB of DNAK, proteins in age-matched-control study group. Dotted lines show the 95% confidence score.
Fig 16. Correlation between brain-derived OMEVP and WB of DNAK₄ proteins in AD6 study group. Dotted lines show the 95% confidence score.

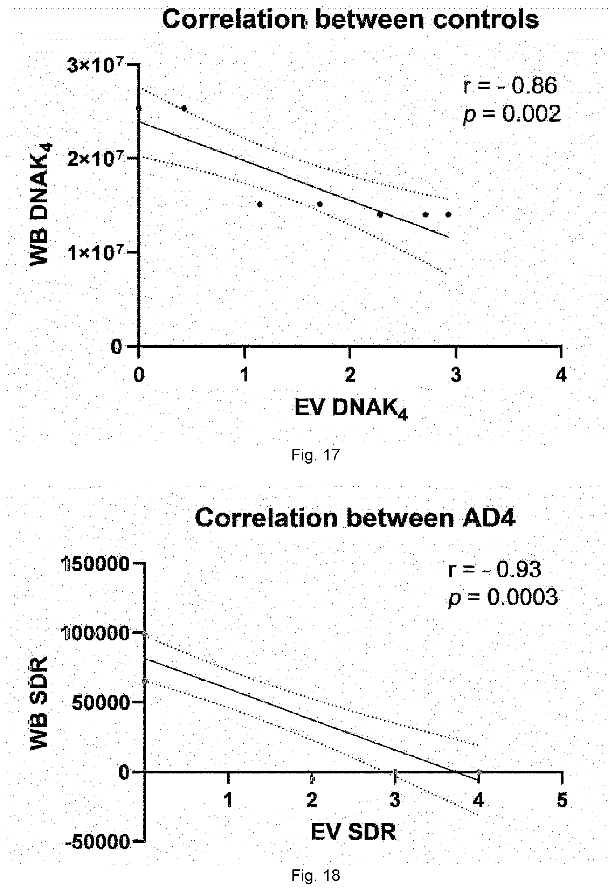

Fig 17. Correlation between brain-derived OMEVP and WB of DNAK₄ proteins in age-matched-control study group. Dotted lines show the 95% confidence score.
Fig 18. Correlation between brain-derived OMEVP and WB of SDR proteins in AD4 study group. Dotted lines show the 95% confidence score.

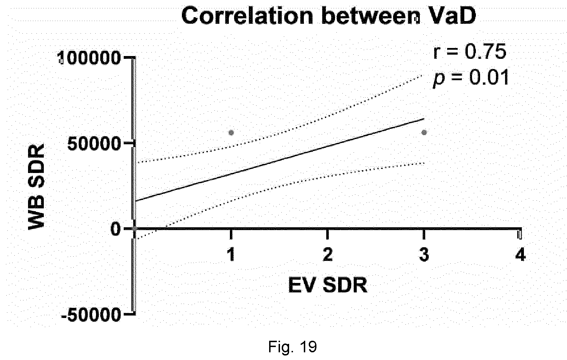

Fig 19. Correlation between brain-derived OMEVP and WB of SDR proteins in VaD study group. Dotted lines show the 95% confidence score.

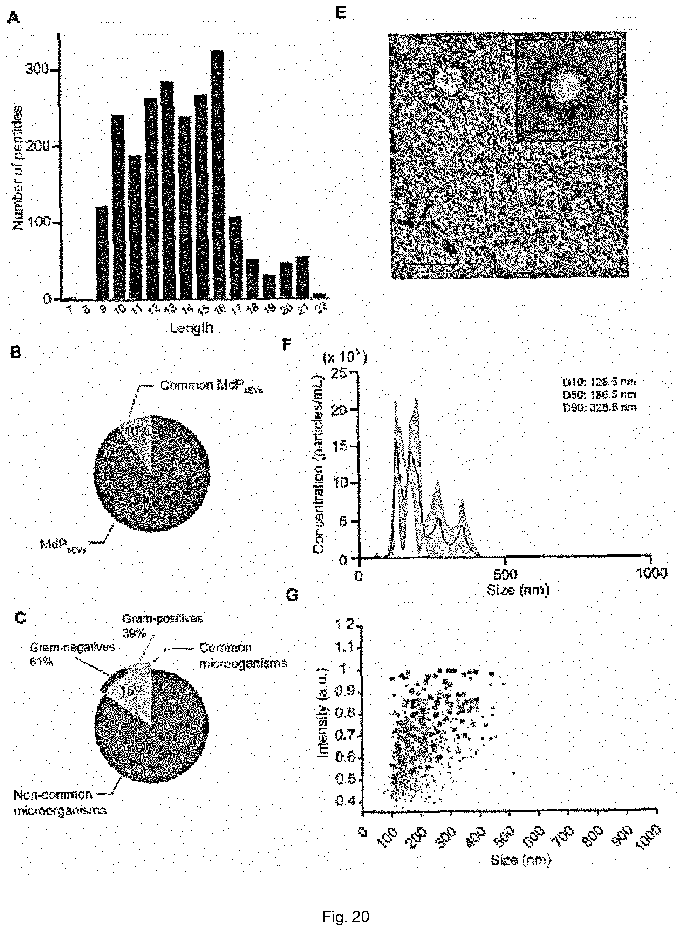

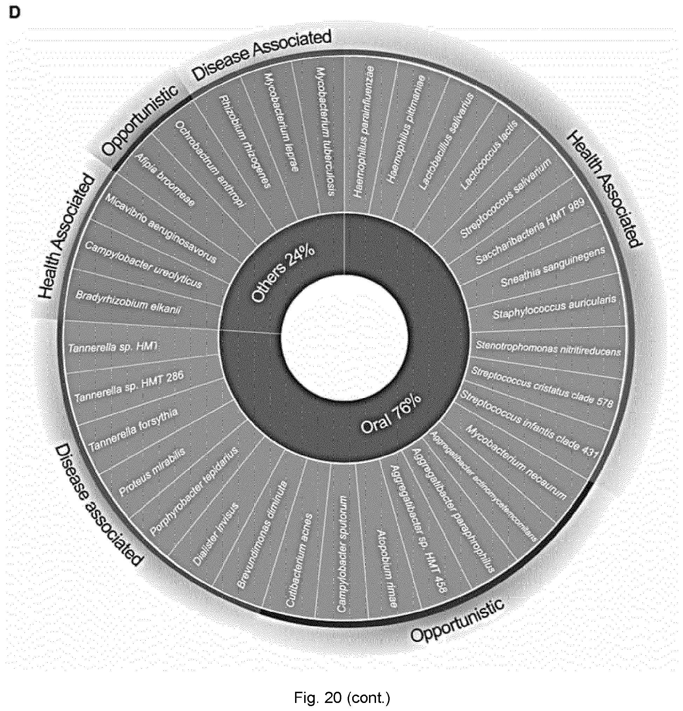

Fig 20. Characterization of microbiome-derived proteomes in brain extracellular vesicles (MdP in bEVs) isolated from human post-mortem brain tissues considering all experimental conditions. A. Peptide sequence length distribution of MdP in bEVs ranging from 7 to 22 amino acids detected by LC-MS/MS after tryptic digestion. B. Percentage of MdP in bEVs regularly identified in all the subjects (Common MdP in bEVs) and percentage of MdP in bEVs not identified in all the subjects (MdP in bEVs). C. Sequence of the common MdP in bEVs are found in the 15% of all the microorganisms' proteomes potentially producing MdP in bEVs. Common microorganisms refer to the microorganisms that potentially produce the common MdP in bEVs. The rest of the microorganisms constitute the non-common microorganisms. The classification of microorganisms into Gram-negative and Gram-positive is indicated by dark green and light green segments, respectively, within the subset of common MdP in bEVs-producing microorganisms identified across all experimental conditions. D. List of microorganisms potentially producing the identified MdP in bEVs categorized according to their origin, (oral: belonging to oral microbiome, or other: belonging to gut, respiratory or skin microbiome [inner pie chart]), and pathogenicity (disease associated: microorganisms usually related to disease, opportunistic: they cause disease under specific conditions, and health associated: microorganisms usually related to health) [outer circle]. E. Representative transmission electron microscopy (TEM) micrographs of brain extracellular vesicles (bEVs) isolated by PROSPR from human post-mortem brain tissues. Scale bars represent 100 nm. Morphometric and quantitative characterization of bEVs assessed by nanoparticle tracking analysis (NTA). F. Histograms showing the particle-size distribution. Standard deviation is represented as an orange shadow. G. Histogram showing the scattering distributions of bEVs. Independent runs are shown in different green tones.

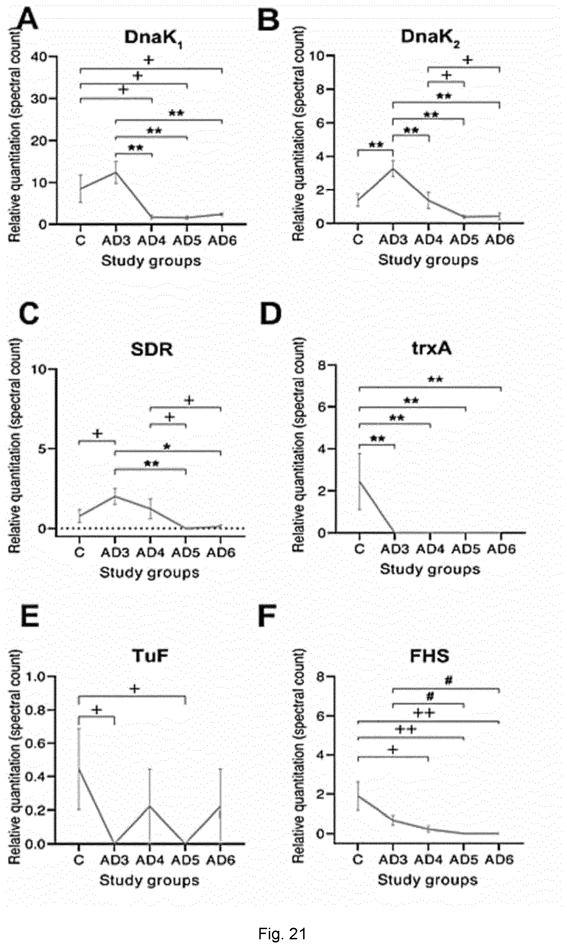

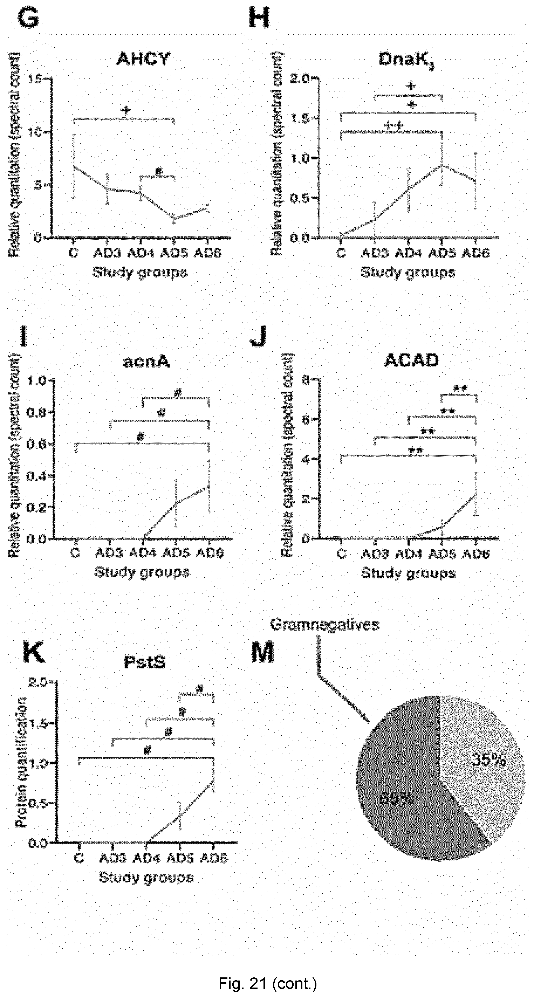

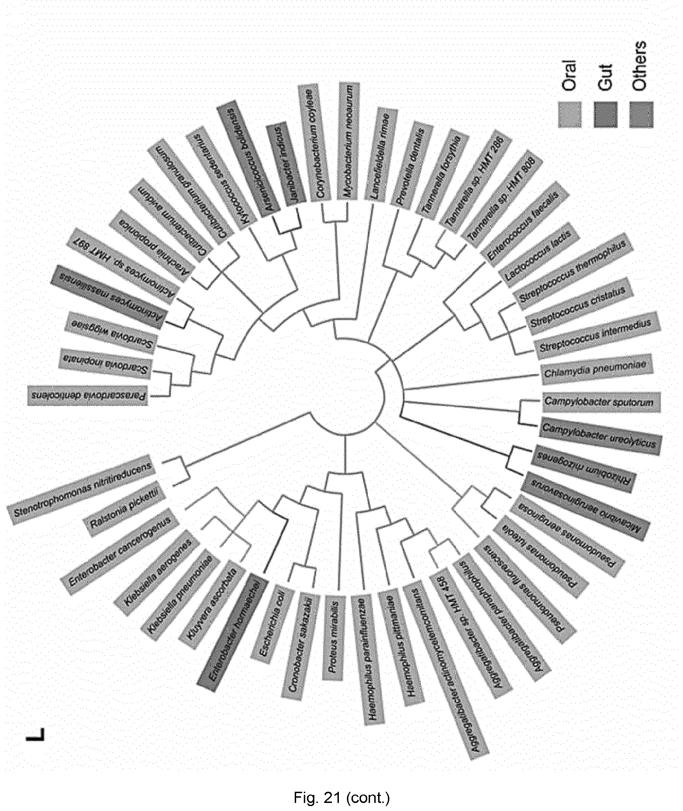

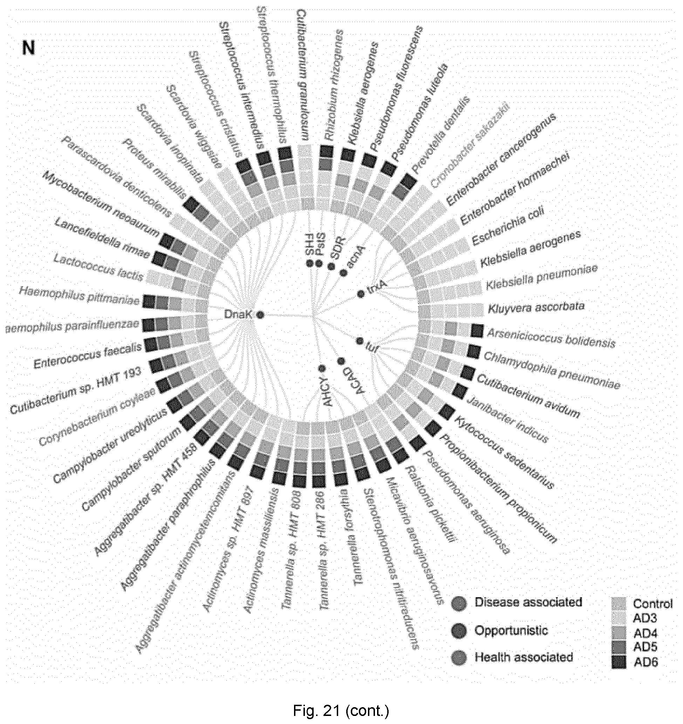

Fig 21. Characterization of oral microbiome-derived proteins in brain extracellular vesicles (OMdP in bEVs) alongside the progression of Alzheimer's disease (AD). A-K. LC-MS/MS quantitation of the significantly modulated OMdP in bEVs in post-mortem brain tissues of subjects at early AD (Braak stage ADS), intermediate AD (Braak stage AD4), advanced AD (Braak stage AD5), final stage AD (Braak stage AD6) and age-matched controls (C). Proteins are identified by their gene symbol and protein quantitation is expressed as averaged sum of spectral count ± standard error of the mean (SEM). L. Phylogenetic analysis of the microorganisms potentially producing the OMdP in bEVs significantly modulated in AD and its belonging habitat (oral: belonging to oral microbiome, gut: belonging to gut microbiome, or others: belonging to respiratory or skin microbiome). M. Percentage of gram-positive and gram-negative in the OMdP in bEVs-producing microorganisms. N. Identification of OMdP in bEVs-producing microorganisms in experimental group based on the presence of unique peptide sequences (peptide sequences existing exclusively in a single protein). Gene symbol of OMdP in bEVs are indicated in the inner part of the diagram. All the microorganisms potentially producing every OMdP in bEVs are included. The presence of unique peptides in every experimental condition is identified with a color code. Garnet tones indicate the presence of unique peptides from this specific microorganism in the different stages of AD and age-matched control condition is represented in green. Grey represents absence of unique peptides for this microorganism in the specific condition. Microorganism names are colored according to their pathogenicity, as disease associated (red), opportunistic (blue) and health associated (green). Significant differences were assessed by two-way ANOVA with Bonferroni correction for multiple comparisons or nonparametric one-way ANOVA as indicated: *indicates significant differences at p ≤ 0.05, "indicates significant differences at p ≤ 0.01, + indicates significant differences determined by ANOVA with Fisher's LSD p ≤ 0.05, ++ indicates significant differences determined by ANOVA with Fisher's LSD p ≤ .01 and # indicates significant differences determined by nonparametric one-way ANOVA on ranks p ≤ 0.05.

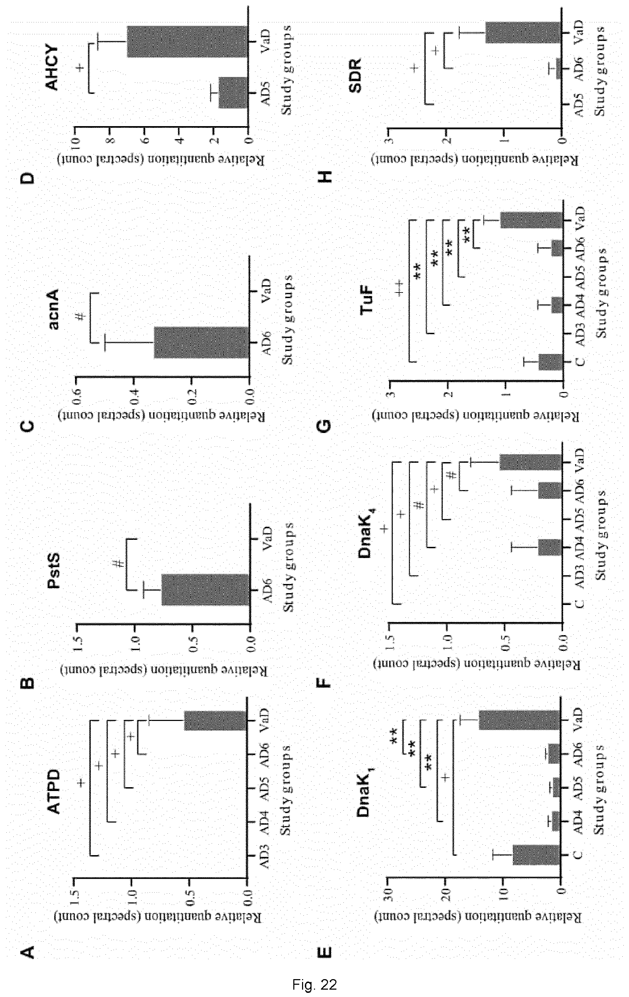

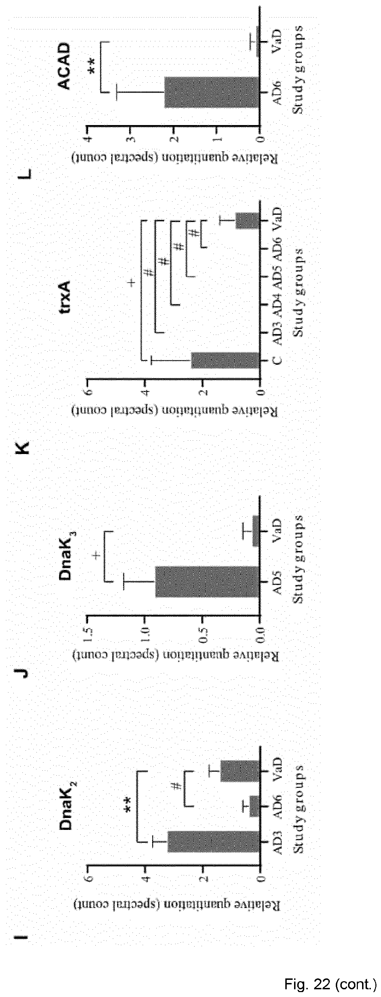

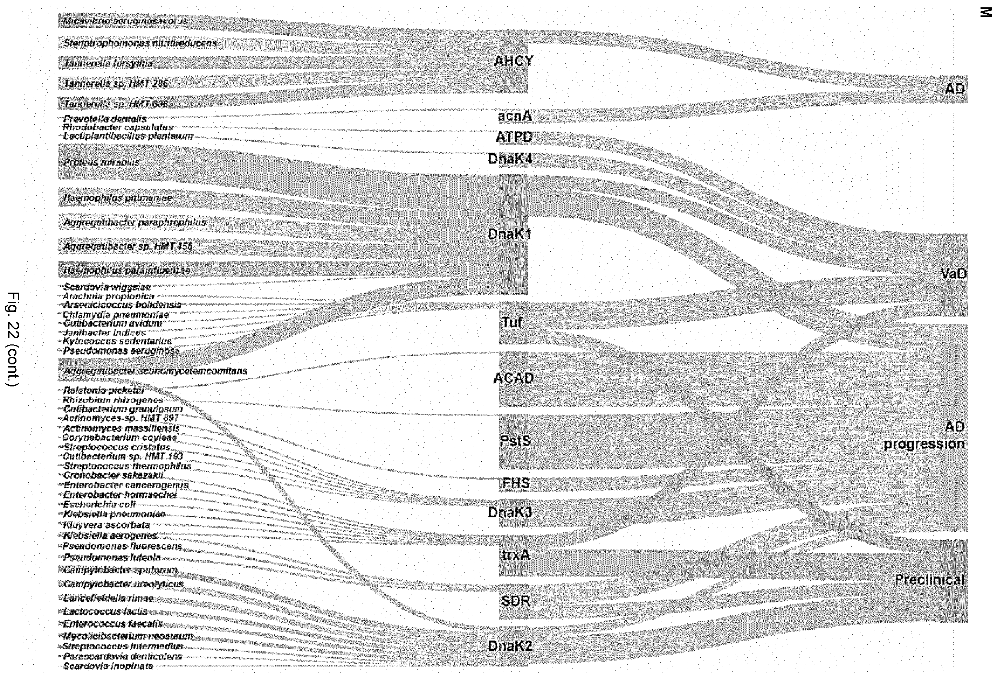

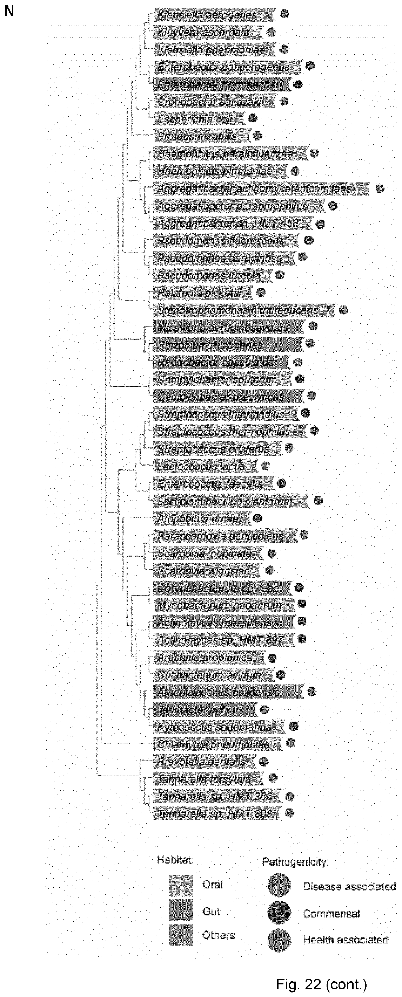

Fig 22. Characterization of oral microbiome-derived proteins in brain extracellular vesicles (OMdP in bEVs) in vascular dementia (VaD). A-L. LC-MS/MS quantitation of the significantly modulated OMdP in bEVs in post-mortem brain tissues of subjects suffering from VaD compared to early AD (AD3), intermediate AD (AD4), advanced AD (AD5), final stage AD (AD6) and age-matched controls (C). Only conditions that shown significant differences comparing to VaD are displayed. Proteins are identified by their gene symbol. Protein quantitation is expressed as averaged sum of spectral count ± standard error of the mean (SEM). M. Sankey diagram representing the connection between OMdP in bEVs-producing microorganisms, OMdP in bEVs and respective dementia-related categories including the following categories: Alzheimer's disease (AD) for OMdP in bEVs significatively modulated or only present in AD groups without an specific tendency toward the early stage of final stage of the AD progression, vascular dementia (VaD) for OMdP in bEVs significatively modulated or only present in VaD, AD progression for OMdP in bEVs that are significantly modulated alongside the AD progression and early AD for those OMdP in bEVs significantly modulated or only present in early AD. N. Phylogenetic analysis of the microorganisms potentially producing the OMdP in bEVs significantly modulated in VaD, its pathogenicity (disease associated, opportunistic or health associated) and its belonging habitat (oral: belonging to oral microbiome, gut: belonging to gut microbiome, or others: belonging to respiratory or skin microbiome). Significant differences were assessed by two-way ANOVA with Bonferroni correction for multiple comparisons or nonparametric one-way ANOVA as indicated: *indicates significant differences at p ≤ 0.05, "indicates significant differences at p ≤ 0.01, + indicates significant differences determined by ANOVA with Fisher's LSD p ≤ 0.05, ++ indicates significant differences determined by ANOVA with Fisher's LSD p ≤ 0.01 and # indicates significant differences determined by nonparametric one-way ANOVA on ranks p ≤ 0.05.

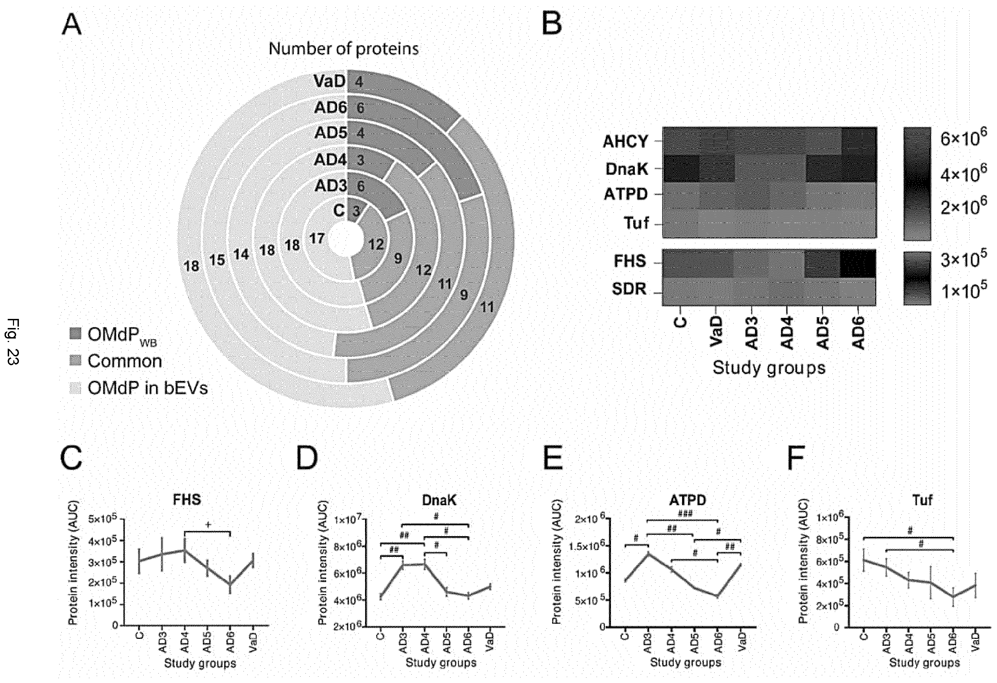

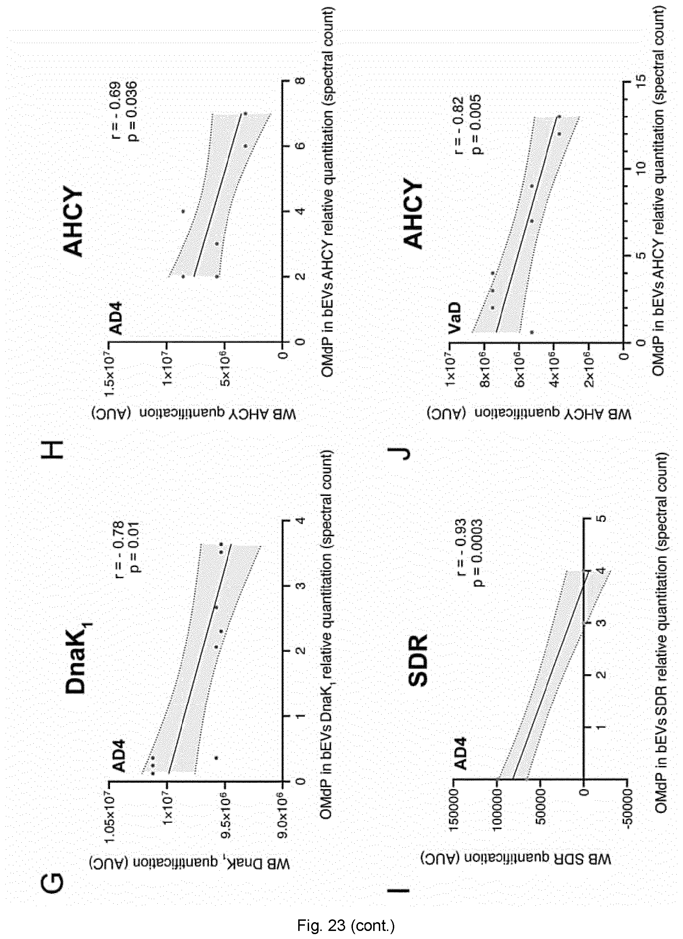

Fig. 23. Potential exchange ability of oral microbiome-derived proteins (OMdP) between brain extracellular vesicles (bEVs) and brain tissues in Alzheimer's diseases (AD) and vascular dementia (VaD). A. Comparative analysis of the presence of OMdP exclusively identified in brain extracellular vesicles (OMdP in bEVs), OMdP exclusively identified in the remaining whole brain (WB) proteome after isolation of bEVs (OMdP in WB) and identification of OMdP commonly present in both fractions bEVs and WB (Common) in early AD (AD3), intermediate AD (AD4), advanced AD (AD5), final stage AD (AD6), VaD and age-matched controls (C). Numbers indicate protein count. B. LC-MS/MS quantitation of OMdP in WB that displayed significant differences between groups in bEVs. Proteins are identified by their gene symbol. Red and green colors in the heatmap represent higher and lower intensity, respectively. C-F. LC-MS/MS quantification of OMdP in WB, expressed as area under the curve (AUC), that displayed significant differences between some of the analyzed groups. Average distribution and standard error of the mean (SEM) are represented. +Significant differences determined by ANOVA with Fisher's LSD P ≤ 0.05. #Significant differences determined by nonparametric one-way ANOVA on ranks P ≤ 0.05. ##Significant differences determined by nonparametric one-way ANOVA on ranks P ≤ 0.01. ###Significant differences determined by nonparametric one-way ANOVA on ranks P ≤ 0.001. G-J. Correlation between the levels of common OMdP detected in bEVs and WB. Only significant correlations with p ≤ 0.05 are displayed. Red shadow shows the 95% confidence score.

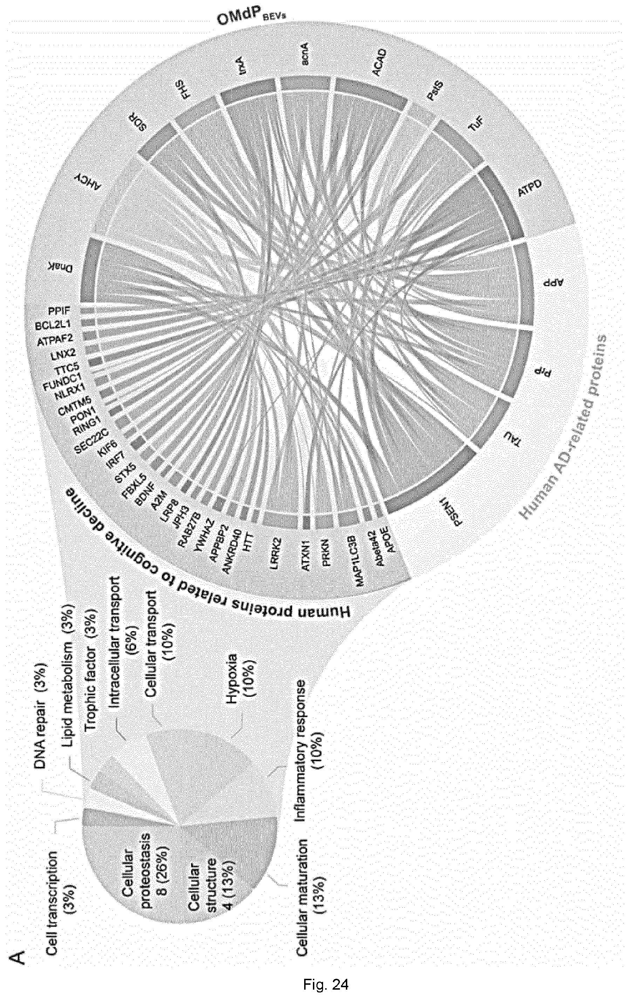

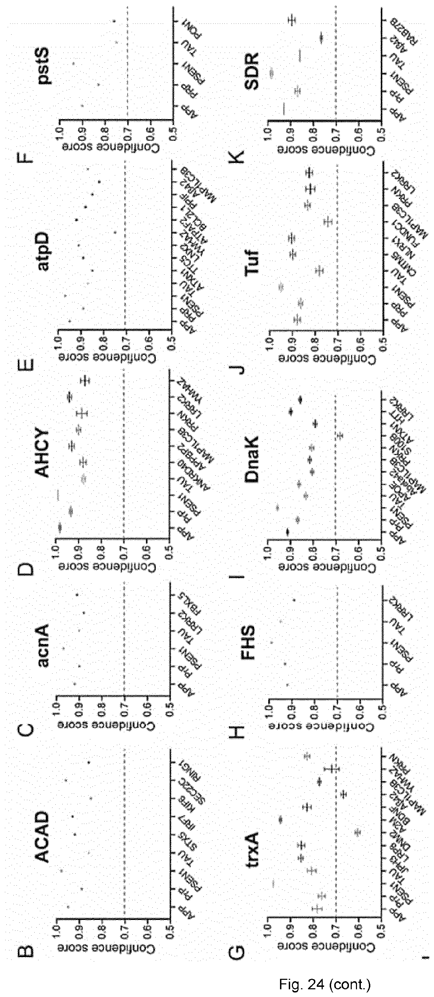

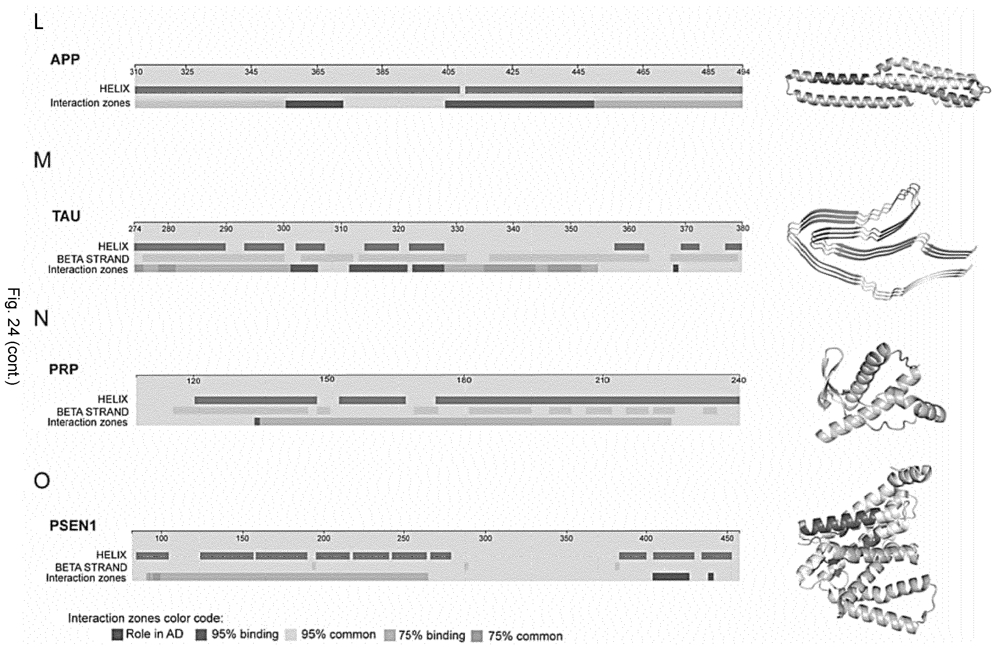

Fig. 24. Prediction of the interaction between oral microbiome-derived proteins in brain extracellular vesicles (OMdP in bEVs) and human proteins associated with cognitive decline and the neuropathology of human dementias. A. Chord diagram presenting the OMdP in bEVs significantly modulated in AD (orange) related to human proteins associated to Alzheimer's disease (AD) (yellow) and cognitive decline (green). The association of proteins is based on in-silico docking simulations. The proteins associated to cognitive decline were manually screened based on scientific literature describing their association with the human homologue of the OMdP in bEVs proteins. Only proteins with confidence score > 0.7 are displayed. The functional analysis of the proteins associated to mental disorders with capacity to interact with OMdP in bEVs is displayed in the pie chart located on the left. B-K. Graphical representation of the docking confidence scores obtained in the docking simulations between OMdP in bEVs and human proteins related to AD and cognitive decline. Docking simulations were performed using all the available PDBs of the OMdP in bEVs of every identified OMdP in bEVs-producing microorganism. Average scores ± standard error of the mean (SEM) is represented, except when only one docking simulation was performed. Dashed red line represents the 0.7 threshold. L-O. AD-related proteins structure and regions involved in the interaction with OMdP in bEVs. For the proteins, amyloid precursor protein (APP, PDB: 5BUO), microtubule-associated protein TAU (TAU, PDB: 6VH7), presenilin 1 (PSEN1, PDB: 7D8X) and prion protein (PRP, PDB: 4KML), the linear representation of the sequence and the 3-dimensional structure are displayed. The protein regions involved in the interaction with OMdP in bEVs are indicated with color according to the chance to participate in the interaction with OMdP in bEVs. Protein region participating in ≥ 95% or 75% of the interactions with the significantly modulated OMdP in bEVs are represented in dark green and light green, respectively. Protein regions documented to play a role in the pathophysiology of AD participating in ≥ 95% or 75% of the interactions are displayed in yellow or orange, respectively. Purple areas represent the documented protein areas that are involved in AD neuropathology based on scientific literature.

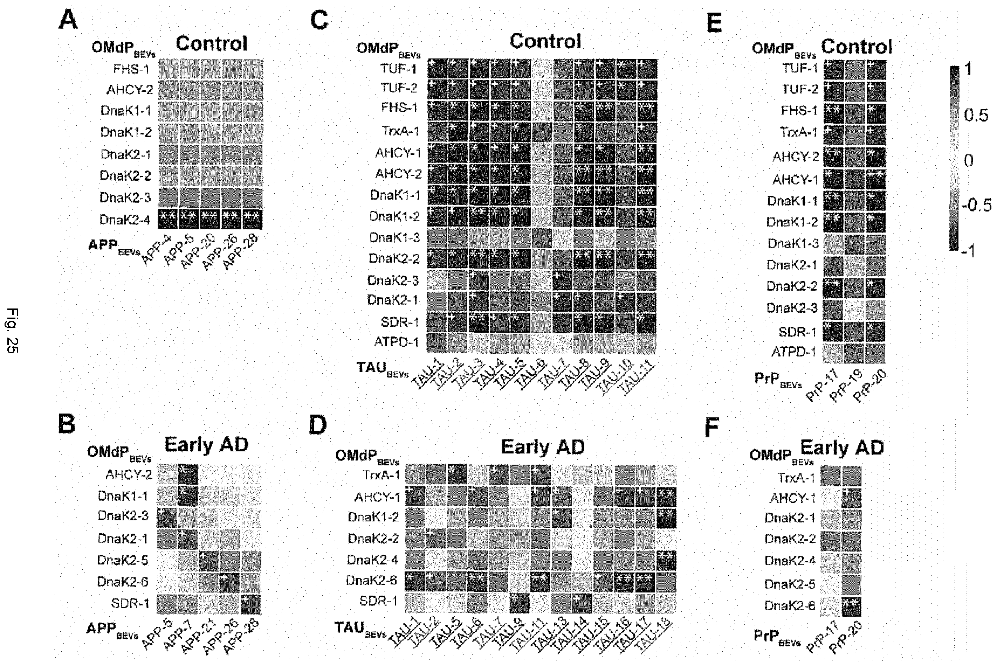

Fig. 25. Heatmap correlation plots including peptides from oral microbiome-derived proteins in brain extracellular vesicles (OMdP in bEVs) significantly modulated in Alzheimer's disease (AD) or vascular dementia (VaD) and peptides from the AD-related proteins detected in brain extracellular vesicles (bEVs). The relative abundance of all detected peptides from the AD-related proteins amyloid precursor protein (APP), microtubule-associated protein TAU (TAU), presenilin 1 (PSEN1) and prion protein (PRP) in bEVs were considered in all these correlation analyses. Correlations were performed using the Pearson correlation method based on the relative quantitation of every detected peptide. R values are represented as different degree of color intensity with blue shades for positive correlations and red shades for negative correlations. A-B. Heatmap correlations between peptides from OMdP in bEVs and APP in age-matched controls (Control) and early AD (AD3), respectively. C-D. Heatmap correlations between peptides from OMdP in bEVs and TAU in Control and AD3, respectively. E-F. Heatmap correlations between peptides from OMdP in bEVs and PrP in Control and AD3, respectively. Peptides are codified according to the gene symbol of the protein followed by a number. Peptide names in red indicate peptides that play a relevant role in AD based on existent literature. Underlined TAU peptides indicate peptides that can be phosphorylated and therefore may play a role in the hyperphosphorylation of TAU that occurs during AD. All peptides reported have at least a correlation > 0.5. + indicates significant correlations with a p ≤ 0.05; * indicates significant correlations with a p ≤ 0.01 and ** indicates significant correlations with a p ≤ 0.001).

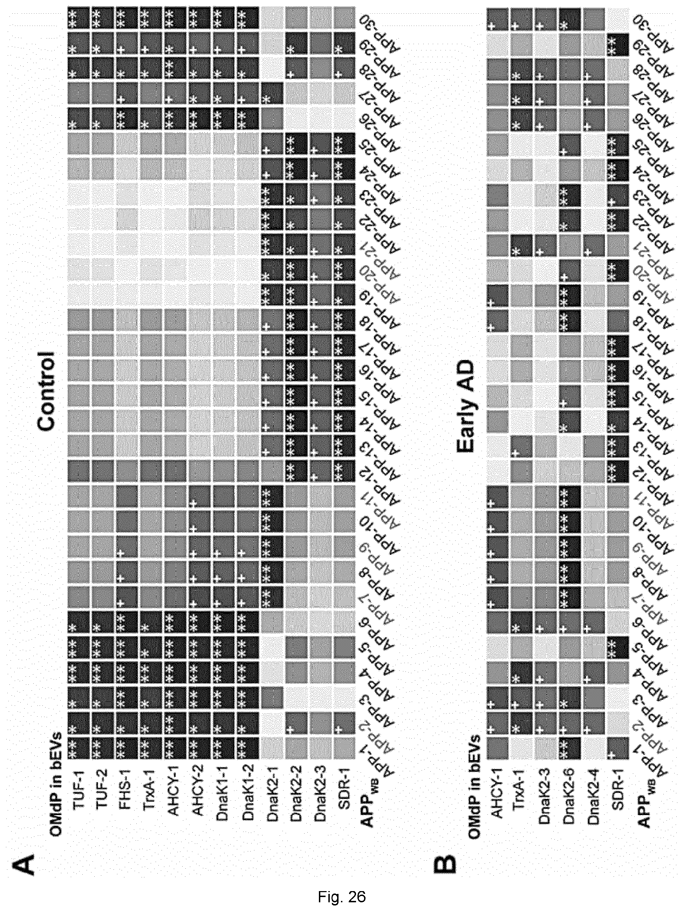

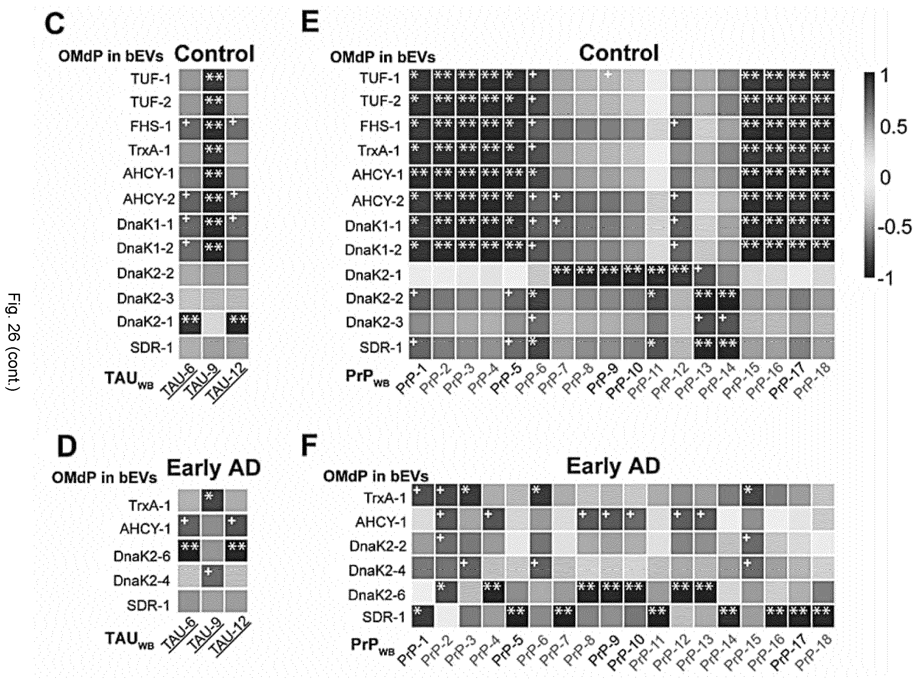

Fig. 26. Heatmap correlation plots including peptides from oral microbiome-derived proteins in brain extracellular vesicles (OMdP in bEVs) significantly modulated in Alzheimer's disease (AD) or vascular dementia (VaD) and peptides from the AD-related proteins detected in the remaining whole brain proteome after isolation of bEVs (WB). The relative abundance of all detected peptides from the AD-related proteins amyloid precursor protein (APP), microtubule-associated protein TAU (TAU), presenilin 1 (PSEN1) and prion protein (PRP) in WB were considered in these correlation study. Correlations were performed using the Pearson correlation method based on the relative quantitation of every detected peptide. R values are represented as different degree of color intensity with blue shades for positive correlations and red shades for negative correlations. A-B. Heatmap correlations between peptides from OMdP in bEVs and APP detected in WB (APPWB) in age-matched controls (Control) and preclinical AD (AD3), respectively. C-D. Heatmap correlations between peptides from OMdP in bEVs and TAU detected in WB (TAUWB) in Control and AD3, respectively. E-F. Heatmap correlations between peptides from OMdP in bEVs and PrP detected in WB (PrPWB) in Control and AD3, respectively. Peptides are codified according to the gene symbol of the protein followed by a number. Peptide names in red indicate peptides that play a relevant role in AD based on existent literature. Underlined TAU peptides indicate peptides that can be phosphorylated and therefore may play a role in the hyperphosphorylation of TAU that occurs during AD. All peptides reported have at least a correlation > 0.5. + indicates significant correlations with a p ≤ 0.05; * indicates significant correlations with a p ≤ 0.01 and ** indicates significant correlations with a p ≤ 0.001).

### EXAMPLES

The purpose of the examples given below is illustrative and is not intended to limit in any way the scope of the invention.

### Example 1: Characterization of extracellular vesicles by Nanoparticle Track Analysis

### Post-mortem brain tissues.

Post-mortem brain tissues (Brodmann area BA21) were obtained from donors with histological signs at time of autopsy of mixed dementia subjects including i) subjects with post-mortem signs of AD neuropathology and accomplishing incipient or advanced neurovascular unit disfunction based on determination of protein markers (named as AD subjects), ii) subjects with post-mortem signs of VaD and accomplishing physiopathology of AD based on protein markers (named as VaD subjects), or age-matched controls (control). Histologically, AD cases displayed neurofibrillary tangles burden consistent with Braak stage III to VI (AD3 to AD6), whereas cases of VaD displayed significant cerebrovascular lesions including small vessel disease predominantly affecting the temporo-parietal region. Brain tissues were frozen in liquid nitrogen at the time of autopsy and stored at - 150 °C until use. BA21 tissues were dissected in small pieces and large blood vessels were removed. Tissues were then washed three times with 1X PBS for 30 min. Three biological replicates were included per condition and were analyzed independently unless otherwise stated.

### Processing of brain tissues prior to obtention of EV fractions

Brain tissues from each subject (~ 80 mg) were homogenized with detergent-free homogenization buffer (100 mM ammonium acetate (AA) supplemented with protease inhibitor cocktail tablets) preserving EV fractions and using the tissue homogenizer bullet blender (Next Advance, NY, USA). Next Advance metallic beads (0.9-2.0 mm of diameter) were washed per triplicate with 1X PBS during 30 min prior to mixing them with brain tissues at 1:1 ratio (w/w). All the detailed procedures for the obtention of EV brain fractions were performed at 4 °C. Brain homogenization was performed in four cycles with 300 µL of homogenization buffer and 5 min of homogenization per cycle. The first two cycles were conducted at medium intensity whereas the last two cycles were conducted at maximum intensity. At the end of each cycle, the homogenate was centrifuged at 15000×g for 10 min and the obtained supernatants were combined.

### Enrichment of brain EV by PROSPR

Brain EV were enriched from the detergent-free homogenates using PROSPR Brain. Methodology. Briefly, brain homogenates processed as previously indicated to minimize tissue contaminants in brain EV preparations were mixed with four volumes of chilled acetone (- 20 °C), vortexed, and centrifuged at 5000×g for < 1 min. Supernatants containing hydrophobic EV fractions were then concentrated to near dryness in the vacuum concentrator (Concentrator Plus, Eppendorf AG, Hamburg, Germany).

### Label-free in solution digestion of brain EV fractions

PROSPR-isolated EV were dissolved in 16 M urea, 100 mM ammonium bicarbonate (ABB) buffer, then diluted twofold with HPLC water. EV constituent proteins were then digested. Briefly, lysed EV were reduced with 20 mM dithiothreitol (DTT) at 30 °C for 3 h, followed by alkylation with 55 mM iodoacetamide (IAA) for 1 h at room temperature in dark environment. Samples were then diluted to < 1 M urea with 50 mM ABB. Overnight trypsin digestion was performed at 37 °C using a 1:20 enzyme-to-protein ratio (w/w) with sequencing grade modified trypsin. Proteolytic digestion was quenched by addition of 0.5% formic acid (FA). Tryptic digested peptides from EV fractions were then desalted using C18 Sep-Pak cartridge (Waters, Milford, MA). Elution of brain EV peptides was performed using 1 ml of 75% CAN, 0.1% FA. Eluates were then dried in the vacuum concentrator and reconstituted in 200 µL of 0.02% ammonium hydroxide (mobile phase A) for the subsequent fractionation by HPLC.

### Proteome extraction of complementary brain tissues

Complementary brain tissue proteome fractions that remained after processing brain tissues as described in the section "Processing of brain tissues prior to obtention of EV fractions" were processed to obtain the remaining brain proteomes in this study. Thus, brain protein fractions without EV fractions were further homogenized using homogenization buffer with addition of 1% sodium deoxycholate (SDC), as described above. Homogenization was performed by 5 homogenization cycles at maximum intensity using the bullet blender homogenizer until no obvious pellet was observable. Extracted proteins were then subjected to acetone precipitation mixing brain tissue homogenates with chilled acetone for 3 h before centrifugation at 20000×g for 10 min. Supernatants were subsequently discarded, and precipitated proteins were airdried to remove excess of acetone.

### High-pressure liquid chromatography fractionation

Brain EV digested proteomes were subjected to HPLC fractionation. Briefly, dried peptides were reconstituted in 200 µL of mobile phase A (10 mmol/L ammonium hydroxide in water) and fractionated using a Xbridge^{™} BEH130 C18 column (3.5µm 4.6 × 250 mm, Waters, Elstree, UK) on a Shimadzu Prominence UFLC system (Dionex, Sunnyvale, CA) with UV monitoring of peptide intensities at 280 nm performed throughout. Peptide separation was performed at 1 mL/min using a 60-min gradient as follows: 0-5% B (0 mmol/L ammonium hydroxide in ACN) for 3 min, 5-35% B for 40 min, 35-70% B for 12 min, and 70-100% for 5 min. Fractions were collected at 1-min intervals and combined by concatenation. Concatenated fractions were then completely dried in the vacuum concentrator.

### Liquid chromatography tandem-mass spectrometry of brain EV proteomes

All experiments detailed were performed per triplicate generating three technical replicates for each biological sample analyzed. Dried peptide fractions of brain EV proteomes were reconstituted in mobile phase A (3% ACN, 0.1% FA) before label-free shotgun proteomics analysis by LC-MS/MS using a Dionex UltiMate 3000 UHPLC system coupled with an Orbitrap Elite mass spectrometer (Thermo Fisher, Inc., Bremen, Germany). Spray was generated using an EASY-Spray ion source (Thermo Fisher Scientific, Inc.) working at 1.5 kV. Peptide separation was performed using a reverse-phase Acclaim PepMap RSL column (75 µm ID × 15 cm, 2-µm particle size, Thermo Scientific, Inc.) maintained at 35 °C and working at 300 nL/min. The 60-min gradient used for peptide separation was as follows: 3% mobile phase B (90% ACN, 0.1% FA) for 1 min, 3 to 35% for 47 min, 35 to 50% for 4 min, 80% for 6 s, 80% (isocratic) for 78 s, 80 to 3% for 6 s, and then maintained at 3% for 6.5 min. Orbitrap Elite data acquisition was performed in positive mode using Xcalibur 2.2 software (Thermo Fisher Scientific, Inc., Bremen, Germany) alternating between full Fourier transform mass spectrometry (FT-MS; 350-2000 m/z, resolution 60,000, with 1 µscan per spectrum) and FT-MS/MS (150-2000 m/z, resolution 30,000, with 1 µscan per spectrum). Fragmentation of the 10 most intense precursors with charge > + 2 and isolated within a 2 Da window was performed using high-energy collisional dissociation mode using 32% normalized collision energy. A threshold of 500 counts was enabled. For full FT-MS and FT-MS/MS, automatic gain control was set to 1 × 106.

### Morphometric characterization of bEVs by Nanoparticle Tracking Analysis

Vesicle content from brain EV enriched by PROSPR from AD and VaD subjects was subjected to Nanoparticle Track Analysis (NTA). Brain vesicle preparations were observed and analyzed through a Nanosight NS300 sCMOS instrument (Malvern, UK). Analysis parameters in Nanosight instrument were set as follows: capture time 60 s, camera level 4, slider shutter 50, slider gain 100, FPS 32.5, syringe pump speed 100, total volume per sample 1 mL, viscosity 0.906-0.910 cP, and temperature ~ 24 °C. No restricted areas were established during analyses; thus, all vesicle content in the sample was subjected to characterization.

### Bioinformatics and data analysis

Analysis of OMEVP was carried out using Peaks studio X pro version with a precursor ion tolerance of 10 ppm and fragment ion tolerance of 0.05 Da. The proteomes available at Human Oral Microbiome database (HOMD) were compiled and used as database in database searching. FDR < 1% was established for protein identification, and trypsin was set as proteolytic enzyme. Cluster analysis based on label-free protein quantitation (total number of spectra detected for all peptides in each protein) was performed using the fuzzy c-mean algorithm in R software. Only proteins with a fuzzy partition coefficient ≥ 0.5 were considered. Lists of clustered proteins were further independently analyzed by parametric one-way ANOVA with Bonferroni correction for multiple comparisons, and statistical significance was set at corrected p < 0.05, unless otherwise specified. Homoestadicity of data from brain EV proteomes was also analyzed by Brown-Forsythe test, and in cases where non-parametric analysis was required, one-way ANOVA on ranks was used and statistical significance was set at p < 0.05, unless otherwise stated. Only proteins confidently identified, based on the detailed statistical criteria, in all subjects in at least one experimental group have been reported. Statistical analysis were performed in Graphpad prism.

Peptides identified by PEAKS studio X pro using the HOMD were further analyzed to eliminate potential human proteome homologous peptides. For that, Isoleucine/Leucine variants of each peptide were generated and compared with the human proteome. Any peptide that coincided with the human proteome was eliminated, confirming the microbial origin of indentified peptides. Accession duplicates were then removed and the count values of these peptides were summed.

In order to study the interaction between OM-derived EVs associated proteins and human brain proteins involved in the neuropathology of AD (as APP, PSEN1 or TAU), we downloaded the respective PDB models and performed an *in silico* docking simulation. For this purpose, we used the software HDOCK to calculate the probability of pairs of proteins interacting. To perform the docking you are first given the pdb of the receptor molecule (the larger one) and then the pdb of the ligand molecule (the smaller one), then the simulation is launched and the software returns the 10 most likely models in pdb format, the list of interacting residues and the probability of this interaction called *confidence score* (values between 0 and 1 appear, only a probability between 0.7 and 1 indicates that it is very likely that the two molecules actually interact).

### Results

OMEVPs positively associated with AD or VaD were further individually analyzed. Some of this OMEVPs, like ACAD, acnA, PstS (Fig. 1-2 and Fig. 10) showed a positive association with the evolution of AD, mainly in AD6, with significant differences with the other study groups. Also, DNAK₃ (Fig. 7) showed a similar behaviour but with higher levels at AD5 stage. There are another group of OMEVPs that show higher leves mainly in age-matched-controls and in VaD, like AHCY (Fig. 3) or ATDP and trxA (Fig. 4 and 12) that indeed don't even appear in AD groups. Something similar occurs with the protein FHS (Fig. 9) that has its highest positive association with controls and is gradually decreasing during AD stages until it desappears at AD5 and AD6 stages, increasing again in VaD group. Furthermore, a subset of OMEVPs, including DNAK₁, DNAK₂ and SDR (Fig. 5-6 and Fig. 11), that displayed a positive association with presymptomatic AD and a slightly lower positive association with VaD. Finally, regarding DNAK₄ and Tuf (Fig. 8 and Fig. 13), these proteins showed a positive association with first symptomatic AD stage and also a higher association during the last stage of AD and VaD.

To confirm a potential link between the increase in OMEVPs and the levels of these proteins observed in WB, we performed a correlation study. A negative and significant interaction between DNAK, protein of both tissues was identified in age-matched-controls (r= -0.67 and p value= 0.04) (Fig. 15) and in AD4 (r= -0.78 and p value = 0.01)(Fig. 14). A positive and significant interaction between DNAK₄ protein of both tissues was identified in AD6 (r = 0.80 and p value = 0.009) (Fig. 16) and a negative and significant in age-matched-controls (r = -0.86 and p value = 0.002)(Fig. 17). Finally, a negative and significant interaction between SDR protein of both tissues was identified in AD4 (r = -0.93 and p value= 0.0003) (Fig. 18) and a positive and significant in VaD (r= 0.75 and p value = 0.01)(Fig. 19). This results reinforce the idea that the proteins are undergoing an exchange between the EVs and WB.

### Example 2:

### Materials and methods

Human samples from a total of 48 subjects were analyzed. Post-mortem brain tissue samples (Brodmann area BA21) were obtained within a post-mortem delay of 18.0 ± 6 h from individuals who showed early to advanced histological signs of AD or VaD at the time of autopsy with an average age of 72.4 ± 8 years. All the cases exhibited amyloid plaques and neurofibrillary tangles. Some of them were in the pathological Braak stage III (early AD3) and as preclinical AD without symptoms of cognitive deterioration. Other cases were at pathological Braak stages IV to VI (intermediate AD4, advanced AD5, to final AD6) with clinical symtoms of dementia. The VaD cases showed AD pathology mixed with significant cerebrovascular lesions, including small vessel disease primarily affecting the temporo-parietal region as well as lacunar or gross infarcts. The post-mortem tissue samples were provided by the Harvard Brain Tissue Resource Center (HBTRC) in Boston, Massachusetts, USA, and the Newcastle Biobank in Newcastle, UK. Subjects identified as early AD were not exhibiting symptoms of the condition at the cognitive level. Brain tissues were obtained at the time of autopsy and stored in liquid nitrogen at -150°C until further processing. The BA21 tissues were cut into small pieces, and large blood vessels were removed. The tissues were then washed three times with 1X PBS for 30 minutes. Three biological replicates were included per condition, and each was analyzed independently in triplicate, unless specified otherwise.

Plasma samples were obtained from the median cubital vein from patients with AD, MCI and age- and gender-matched controls. Neurological conditions were clinically verified. Briefly, AD diagnoses were based on the criteria from the National Institute of Neurological and Communicative Disorders and Stroke and the Alzheimer's Disease and Related Disorders Association (NINCDS-ADRDA) and the International Classification of Diseases, 10th Edition (ICD-10). MCI diagnosis was based on the Petersen criteria. Ten biological replicates were independently analyzed per duplicate per condition. All experimental procedures were strictly performed in accordance with the relevant institutional guidelines.

### Brain tissue processing prior to the obtention of EVs

Brain tissue samples from each subject (~80 mg) were homogenized using detergent-free homogenization buffer (consisting of 100 mM ammonium acetate (AA) supplemented with protease inhibitor cocktail tablets). The tissue homogenizer bullet blender from Next Advance (NY, USA) was utilized for this process. Specifically, metallic beads with a diameter of 0.9 to 2.0 millimeters from Next Advance were washed three times with 1X PBS for 30 minutes before being combined with the brain tissue samples at a ratio of 1:1 (weight to weight). All steps in the process of obtaining EV brain fractions were carefully conducted a temperature of 4°C. Brain homogenization was performed four times, with each cycle comprising 300 µL of homogenization buffer and 5 minutes of homogenization. The initial two cycles were carried out at medium intensity, while the final two cycles were conducted at maximum intensity. After each cycle, the homogenate was centrifuged at 15,000 × g for 10 minutes, and the resulting supernatants were combined.

### Obtention of EVs

bEVs were enriched from detergent-free homogenates by Protein Organic Solvent Precipitation (PROSPR-Brain). Briefly, brain homogenates were combined with four volumes of chilled acetone (at -20°C), and then vortexed and centrifuged at 5000 × g for less than 1 minute. The resulting supernatants containing hydrophobic EVs were subsequently concentrated to near dryness using a vacuum concentrator (Concentrator Plus, Eppendorf AG, Hamburg, Germany). Plasma EVs were enriched from 1 mL of plasma, by performing two cycles of centrifugation at 100,000 × g, 1h at 4°C. EVs were then washed with PBS and resuspended in 20 µL PBS.

### In solution digestion processing of EVs

We carried out a Label-free in solution digestion of PROSPR-isolated brain EV. Briefly, the solubilization of bEVs was performed using 16 M urea and 100 mM ammonium bicarbonate (ABB) buffer, which was then diluted twofold with high-pressure liquid chromatography (HPLC) water. The lysed EVs were subjected to reduction with 20 mM dithiothreitol (DTT) at 30 °C for 3 hours, followed by alkylation with 55 mM iodoacetamide (IAA) for 1 hour at room temperature in the dark. The samples were diluted to a urea concentration of less than 1M with 50 mM ABB. Subsequently, overnight trypsin digestion was carried out at 37 °C using a 1:20 enzyme-to-protein ratio (w/w) with sequencing grade modified trypsin. The proteolytic digestion was halted by the addition of 0.5% formic acid (FA). The tryptic digested peptides from the EV fractions were desalted using a C18 Sep-Pak cartridge (Waters, Milford, MA). Elution of brain EV peptides was achieved using 1 mL of 75% acetonitrile (ACN) with 0.1% FA. The eluates were then dried in a vacuum concentrator and reconstituted in 200 µL of 0.02% ammonium hydroxide for subsequent fractionation by HPLC. For plasma EVs commercial S-trap Micro Spin Columns (Protifi, NY, USA) were used following manufacturer's instructions [45].

### Proteome extraction of remaining Whole Brain tissues

The subsequent processing of the brain tissue homogenates that were depleted of EVs involved the use of a homogenization buffer supplemented with 1% sodium deoxycholate. This process was carried out to obtain the whole brain (WB) proteome. The homogenization process was carried out through 5 cycles at maximum intensity using the bullet blender homogenizer until no discernible pellet remained visible. The extracted proteins were then subjected to acetone precipitation by mixing brain tissue homogenates with chilled acetone for 3 hours, followed by centrifugation at 20000 × g for 10 minutes. The resulting supernatants were then discarded, and the precipitated WB proteomes were subjected to air-drying to eliminate excess acetone.

### In-solution tryptic digestion and isobaric peptide labeling of WB proteomes

WB proteomes were solubilized in lysis buffer at pH 8.5, which included 1% SDC and 100 mM triethylammonium bicarbonate (TEAB), along with a complete protease inhibitor cocktail. Individual WB samples from subjects were combined to yield 200 µg protein/condition. Bicinchoninic acid assay was used for protein quantification. WB proteomes were subjected to reduction using 10 mM tris 2-carboxyethyl phosphine hydrochloride for 2 hours at 55 °C, followed by alkylation with 55 mM IAA for 1 hour at room temperature protected from the light. The SDC concentration was then reduced to 0.5% using 100 mM TEAB. Overnight trypsin digestion was conducted with sequencing-grade modified Promega trypsin at a 1:25 protein-to-enzyme ratio (w/w). The digestion process was stopped by adding a final concentration of 0.5% FA. SDC elimination from the digested samples was achieved through acidification, and peptides were retrieved from SDC precipitates. Tryptic peptides were dried in a vacuum concentrator before being reconstituted in 100 mM TEAB. The digested WB proteomes were then labeled using 6-plex isobaric tags (tandem mass tag, TMT, from ThermoScientific (San Jose, CA)), following the manufacturer's instructions. The TMT isobaric group labels were assigned as follows: 126 for age-matched controls (control), 127 for VaD, 128 for preclinical AD (AD3), 129 for early symptomatic AD (AD4), 130 for advanced stage AD (AD5), and 131 for final stage AD (AD6). Following labeling, peptides were desalted using a C-18 Sep-pack 200 mg cartridge (Waters, Milford, MA), and the eluates were dried thoroughly in the vacuum concentrator.

### Simplification of brain EVs proteomes by High-Pressure Liquid Chromatography

Digested bEVs proteomes were simplified by being subjected to HPLC fractionation. To outline, the dried peptides were reconstituted in 200 µL of 10 mmol/L ammonium hydroxide in water (mobile phase A) and separated using a Xbridge^{™} BEH130 C18 column (3.5 µm 4.6 × 250 mm, Waters, Elstree, UK) on a Shimadzu Prominence UFLC system (Dionex, Sunnyvale, CA). Peptide intensities were monitored via UV at 280 nm throughout the process [53]. Peptide separation occurred at a flow rate of 1 mL/min over a 60-minute gradient: 0-5% B (consisting of 0 mmol/L ammonium hydroxide in ACN) for 3 minutes, 5-35% B for 40 minutes, 35-70% B for 12 minutes, and 70-100% for 5 minutes. Fractions were collected every minute and subsequently combined through concatenation. Following concatenation, fractions were completely dried in the vacuum concentrator.

### Liquid chromatography tandem-mass spectrometry of EVs proteomes

Dried peptide fractions of bEVs proteomes were reconstituted in mobile phase A (comprised of 3% acetonitrile and 0.1% formic acid) prior to undergoing label-free shotgun proteomics analysis by means of liquid chromatography-tandem mass spectrometry (LC-MS/MS). This process was carried out utilizing a Dionex UltiMate 3000 UHPLC system coupled with an Orbitrap Elite mass spectrometer (Thermo Fisher, Inc., Bremen, Germany).

The ion source utilized for spray generation was the EASY-Spray (Thermo Fisher Scientific, Inc.), which operated at a voltage of 1.5 kV. Peptide separation was executed using a reverse-phase Acclaim PepMap RSL column (75 µm ID × 15 cm, 2-µm particle size, Thermo Scientific, Inc.), maintained at a temperature of 35°C and operating at a flow rate of 300 nL/min. A 60-minute gradient was implemented for peptide separation, which commenced with 3% mobile phase B (comprised of 90% acetonitrile and 0.1% formic acid) for 1 minute, followed by a linear increase to 35% over 47 minutes, a subsequent increase to 50% over 4 minutes, a brief period of 80% for 6 seconds, a 78-second period of constant 80%, a brief period of 80% (isocratic) for 6 seconds, a return to 3% for 6 seconds, and finally, a constant 3% for 6.5 minutes. The Orbitrap Elite data acquisition was carried out in positive mode using Xcalibur 2.2 software (Thermo Fisher Scientific, Inc., Bremen, Germany) alternating between full Fourier transform mass spectrometry (FT-MS; 350-2000 m/z, resolution 60,000, with 1 µscan per spectrum) and FT-MS/MS (150-2000 m/z, resolution 30,000, with 1 µscan per spectrum). Fragmentation of the 10 most intense precursors with charge > + 2 and isolated within a 2 Da window was performed using high-energy collisional dissociation mode using 32% normalized collision energy. A threshold of 500 counts was enabled. For full FT-MS and FT-MS/MS, automatic gain control was set to 1 × 10⁶. Liquid chromatography-tandem mass spectrometry of plasma EVs proteomes was performed using an Orbitrap Fusion Tribrid mass spectrometer (Waltham, MA, USA) doing a separation of peptides on an analytical EASY-Spray Column (50 mm × 75 µm, PepMap RSCL, C18, 2 mm, 100 Å, Thermo Scientific) in a 91 min-gradient.

### Liquid chromatography tandem-mass spectrometry of isobaric-labeled WB proteomes

The reconstituted dried fractions of the WB isobaric-labeled proteome samples were analyzed using a Dionex Ultimate 3000 RSLCnano system coupled with a Q Exactive mass spectrometer (Thermo Fisher, Inc., Bremen, Germany). The ionization was achieved using an EASY-Spray ion source (Thermo Fisher Scientific, Inc.) operating at 1.5 kV. The peptide separation was carried out using a PepMap C18 column (3 µm, 100 Å, Thermo Fisher Scientific, Inc.) maintained at 35 °C and operating at a flow rate of 300 nL/min. The separation of peptides occurred over a 60-minute gradient with mobile phase A (0.1% FA in water) and mobile phase B (0.1% FA in 90% ACN) as follows: 3-30% B for 45 minutes, 30-50% B for 9 minutes, 50-60% B for 1 minute, 60% B for 2 minutes, and finally maintained isocratically at 3% B for 3 minutes. The Q Exactive data acquisition was performed in positive ion mode using Xcalibur 3.0.63 software (Thermo Fisher Scientific, Inc., Bremen, Germany), alternating between full Fourier transform MS (FT-MS; 350-1600 m/z range, resolution of 70,000 at m/z 200, 1 µscan per spectrum) and FT-MS/MS (resolution 35,000) for the 10 most intense ions with charge > + 2 and isolated within a 2 Da window. The ion fragmentation was achieved through high-energy collisional dissociation fragmentation mode using 28% normalized collision energy. A threshold of 500 counts was applied. For both full FT-MS and FT-MS/MS, automatic gain control was set to 5 × 106 and 2 × 105, respectively.

### Morphometric characterization of bEVs by Nanoparticle Tracking Analysis

Representative fractions of bEVs obtained from post-mortem brains of subjects diagnosed with preclinical AD were thoroughly characterized by Nanoparticle Tracking Analysis (NTA). NTA analyses were carried out utilizing a Nanosight NS300 sCMOS instrument (Malvern, UK) configured as follows: capture time of 60 seconds, camera level set to 4, slider shutter at 50, slider gain at 100, frames per second (FPS) at 32.5, syringe pump speed at 100, total volume per sample at 1 milliliter, viscosity in the range of 0.906-0.910 centipoise, and temperature maintained at approximately 24 degrees Celsius. In these analyses, no specific areas were designated as off-limits, enabling the random screening of unrestricted flowing samples.

### Ultrastructural characterization of bEVs

Representative bEVs samples obtained from post-mortem brains of AD subjects were prepared for transmission electron microscopy (TEM) analysis. The EVs samples were applied onto Cu-Formvar-carbon grids and allowed to settle for 20 minutes at room temperature (RT). Subsequently, the grids were washed with HPLC water, and the bEVs preparations were fixed using 1% glutaraldehyde in PBS for 5 minutes. After fixation, the EVs were stained with uranyl oxalate for 5 minutes, followed by embedding in methylcellulose-uranyl-oxalate and air-drying for permanent preservation. Electron micrographs of the preparations were captured using a Jeol Jem 1010 electron microscope operated at 80 kV. The acquired ultrastructural images were then scale-calibrated, bi-leveled, and further analyzed using the open-source software imaged (National Institutes of Health, Bethesda, MD, USA).

### Next generation proteomics of circulating blood plasma EVs

In this example, we aimed to verify the circulating capabilities of OMdP in bEVs. To accomplish this, we sought out publicly accessible datasets from specialized repositories, such as ProteomeXchange, that had conducted analyses on blood plasma EVs in individuals with AD using next-generation discovery-driven proteomics. Upon conducting a search for these criteria, we discovered the datasets that had been submitted by JE Nielsen, B Honoré, K Vestergård, RG Maltesen, G Christiansen, AU Bøge, SR Kristensen and S Pedersen with identifier PXD024216. The datasets in question comprise LC-MS/MS proteomics data of plasma EV obtained from a total of 30 subjects, including 10 subjects diagnosed with AD, 10 subjects diagnosed with mild cognitive impairment (MCI), and 10 healthy age-matched controls. In this example, plasmatic EVs were obtained by double centrifugation at 100,000 × g for 1 hour at 4°C. Plasma EVs proteomes were processed and digested using the commercial strategy S-Trap Micro Spin Column according to the manufacturer's instructions (Protifi, NY, USA). The label-free LC-MS/MS analysis of plasma EVs was carried out using a high-throughput instrument, the Orbitrap Fusion Tribrid mass spectrometer.

### Bioinformatics and statistical analyses

### Mass spectrometry data analysis

Analysis of proteomics raw data was performed using PEAKS Studio X Pro software (version 10.6 Bioinformatics Solutions). FDR < 1% was established for protein identification in all samples and trypsin was set as proteolytic enzyme. Carbamidomethylation of Cys was set as fixed modification. A precursor tolerance of 10 ppm and fragment ion tolerance of 0.05 Da was allowed in the searches. Data were searched against an in-house created database compiling all the available protein sequences in the Human Oral Microbiome Database (HOMD) (4.028.421 sequences, 25th Feb 2020). Database search of OMdP in whole brain proteome after isolation of bEVs was conducted with a restricted database containing the sequences of the OMdP identified in bEVs. In that search the TMT 6 plex tag masses were included as fixed modifications in the database search of the isobaric TMT labeling raw data. Database search results were thoroughly exported into comma separated values files. Cluster analysis based on label-free protein quantitation (total number of spectra detected for all peptides in each protein) was performed in the data from the bEVs proteomes using the fuzzy c-mean algorithm in R software (version 4.2.1). Only proteins with a fuzzy partition coefficient ≥ 0.5 were considered. Lists of clustered proteins were further independently analyzed by parametric one-way ANOVA with Bonferroni correction for multiple comparisons, and statistical significance was set at corrected p ≤ 0.05, unless otherwise specified.

### Identification of oral microbiome-derived peptides and statistical analyses

In order to accurately identify the tryptic digested peptides derived from OMdP in bEVs present in the analyzed samples, we utilized a rigorous screening process to remove any human-homologous peptide. For that purpose, all possible isoleucine/leucine variants of each identified OMdP in bEVs peptide were generated and compared against the human Uniprot proteome database (79.703 sequences, 27th June 2022) using an in-house script created using Spyder software (Python version 3.11). At the end, any peptide present in the human proteome was excluded from further analysis to ensure proper accuracy of the obtained results. The Identified proteins and peptides were then further analyzed by parametric one-way ANOVA with Bonferroni correction for multiple comparisons, and statistical significance was set at corrected p ≤ 0.05, unless otherwise specified. Statistical analyses were performed in Graphpad Prism version 9.0.2. Multiple correlation analyses were performed in R by calculating the Pearson's correlation coefficients between study groups at protein level. A 95% of confidence interval was used, and strong interaction between correlated variables was considered when r ≥ 0.70, whereas very strong interaction between correlated variables was considered when r ≥ 0.85. Significance of the interaction was established when p ≤ 0.05.

### Oral microbiome

Information regarding habitat of the microorganisms potentially producing the identified OMdP in bEVs was obtained from the HOMD. This information together with information regarding pathogenicity was curated manually based on existent literature.

### In silico Molecular dynamics

In silico docking simulations were carried out using the HDOCK software. Protein structures were obtained in Protein Data Bank (PDB) format from the Research Collaboratory for Structural Bioinformatics (RCSB) ([www.rcsb.org](http://www.rcsb.org)) or the Alphafold database from the European Bioinformatics Institute ([https://alphafold.ebi.ac.uk](https://alphafold.ebi.ac.uk)). The 10 most probable interaction models generated in each in silico simulation were carefully examined, and that with higher confidence score was selected. From every docking simulation, the interaction in PDB format was generated, along with a list of interacting residues and a confidence score (confidence score > 0.7 indicates that the two molecules are likely to bind[58]). Chignolin was used as a negative control for the docking simulation. The selection of human brain proteins linked to neuropathology was predicated on their common interactions with the human homologues of the OMdP in bEVs. Human homologous proteins of OMdP in bEVs were identified from Uniprot and blast analysis was conducted to verify sequence homology. Functions of every human homologous protein of OMdP in bEVs was obtained from Uniprot and GeneCards. Protein images were created and processed using PyMOL (The PyMOL Molecular Graphics System, Version 3.0.2 Schrödinger, LLC).

### RESULTS

### Identification of common microbiome-derived proteomes in bEVs preparations

The primary objective in this example was to assess the relationship between bEVs, which may function as intercellular and inter-species communication mediators, and specific microbiome-derived proteomes (MdP) in the brain. Using next-generation proteomics in conjunction with advanced systems biology, we observed the presence of MdP in all bEVs preparations we analyzed. By adopting a bottom-up approach, we identified 536 MdPbEVs peptides, ranging in length from 7 to 22 residues, in bEVs from all analyzed subjects (Figure 20A). These shared MdP peptides specifically correspond to 71 different proteins that were potentially attributed to 245 distinct microorganisms. Additionally, 401 unique peptides that could be attributed to 219 specific microorganisms were also identified. Further analysis revealed that 10% of the total MdP in bEVs and 15% of the identified microorganisms that synthesize these proteins were conditions and of the microorganisms producing MdP in bEVs, 61% were Gram-negative, while 39% were Gram-positive (Figure 20B and Figure 20C, respectively). Our findings also revealed that 76% of these common microorganisms preferentially belong to the OM and were linked to the seven specifically common OM-derived proteins (OMdP in bEVs) 2,3-bisphosphoglycerate-dependent phosphoglycerate mutase, adenosylhomocysteinase, ATP synthase subunit alpha, ATP synthase subunit beta, chaperone protein DnaK, nucleoside diphosphate kinase and triosephosphate isomerase (Figure 20D). The remaining 24% of these specifically attributed microorganisms belonged to other realms, as illustrated in Figure 20D. Similarly, 32% of OMdP in bEVs identified in this example displayed disease-associated properties (Figure 20D). Notable species in this category included Tannerella forsythia, Proteus mirabilis, Dialister invisus, and Brevundimonas diminuta, as depicted in Figure 20D. Additionally, 24% of the OM microorganisms identified, which were linked to particular OMdP in bEVs, displayed commensal abilities. These include species and microorganism strains such as Aggregatibacter sp. HMT 458 or Aggregatibacter paraphrophilus, among others, as extensively detailed in Figure 20D.

### Ultrastructural characteristics and nanoparticle distribution of bEVs including OMdP

Subsequently, to explore the physicochemical traits of bEVs containing OMdP, we subjected various randomly chosen preparations of bEVs to thorough ultrastructural characterization. Electron microscopy (EM) micrographs revealed that these bEVs preparations exhibited intact, regular bi-layered spherical shapes within the commonly observed bEVs diameter range (Figure 20E), with a diameter predominance in the 90 - 200 nm range. Further characterization of these bEVs preparations was performed using NTA. Screening of the obtained bEVs using NTA without restrictions confirmed our above-mentioned EM observations and demonstrated that these bEVs reached their peak concentration at diameters of approximately 150 nm (Figure 20F-G), while the entire distribution ranged from approximately 40 nm to approximately 400 nm (Figure 20F-G).

### OMdP levels in bEVs undergo specific modulation throughout AD progression

Subsequently, our objective was to assess whether MdP exhibit alterations in presence and abundance levels in bEVs alongside the progression of AD. To accomplish this, we performed a clustering analysis and sequential statistical analyses and found that a specific group of MdP in bEVs showed statistically significant differences during both early and clinical stages of AD, as demonstrated in Figure 21A-K. In general, every MdP fitted within a specific cluster, except DnaK which linked to different potential microorganisms of origin displayed distinct modulation patterns alongside the AD progression based on abundance. Thus, four different DnaK isoforms were considered in subsequent analysis according to their disease modulation indicated by subscript numbers (DnaK1 to DnaK4). Specifically, the following MdP in bEVs heat shock proteins (DnaK1 and DnaK2) and short-chain dehydrogenase (SDR) demonstrated a significant increase in early AD (Figure 21A-C). In contrast, MdP in bEVs nerve growth factor-like (trxA) and elongation factor (TuF) showed a significant decrease in this preclinical phase of AD (Figure 21D-E). Several of these microorganism proteins were also linked to the clinical progression of AD by showing specific downtrend and uptrend patterns in bEVs, including formate--tetrahydrofolate ligase (FHS), adenosylhomocysteinase (AHCY) (Figure 21F-G), and bacterial heat shock protein (DnaK3), aconitate hydratase A (ACNA), acyl-CoA dehydrogenase (ACAD) and phosphate-binding protein (PstS) (Figure 21H-K).

Further screening revealed that these 11 formerly referred MdPbEVs, found significantly dysregulated linked to the progression of AD, can be specifically produced by 46 specific microorganisms (Figure 21L). The origin of these microorganisms was classified as from the OM, with over 84% belonging to this category, while less than 3% belonged to the gut microbiota and the remaining 13% remaining unclassified (Figure 21L). Moreover, we found that 65% of these microorganisms were gram-negative bacteria, while only 35% were gram-positives (Figure 21M). The results of these analyses demonstrated that approximately 37% of these microorganisms display disease-associated properties, while 44% are considered commensal, and 19% exhibit health-promoting characteristics (Figure 21N). Furthermore, these analyses uncovered the presence of particular OMdP in bEVs from health-promoting microorganisms in the bEVs of age-matched controls and early AD subjects, as determined by the identification of unique microorganism-associated peptides (Figure 21N). These microorganisms include Cronobacter sakazakii and Cutibacterium granulosum (Figure 21N). Additionally, we found specific OMdP in bEVs derived from disease-associated microorganisms in the bEVs of AD patients, which were not present in age-matched controls (Figure 21N). These AD-linked disease associated microorganisms include *Prevotella dentalis, Ralstonia picketti, Actinomyces massiliensis, Actinomyces sp. HMT 897* and *Rhizobium rhizogenes.*

### Specific OMdP levels in bEVs are differentially linked to VaD

We subsequently examined whether OMdP in bEVs of individuals with VaD exhibit consistent results and/or specific differences in comparison to the observations made during the analysis of these proteins and microorganisms in AD. The majority of OMdP in bEVs identified as differentially regulated in the previously analyzed AD stages exhibited significant differences when compared to VaD, as demonstrated in Figure 22A-L. Specifically, the bacterial protein ATP synthase subunit beta (ATPD) was exclusively identified in the bEVs of subjects with VaD (Figure 22A), while the bacterial PstS (Figure 22B) and acnA (Figure 22C) were not present in the bEVs of VaD subjects. Several OMdP in bEVs were found to be upregulated in the bEVs of VaD subjects compared to those from one or more of the analyzed AD stages. These included AHCY, the chaperones DnaK1 and DnaK4, TuF, and 2-keto-3-deoxy-L-fuconate dehydrogenase (SDR) (Figure 22D-H). Additionally, several OMdP were found to be downregulated in the bEVs of VaD compared to AD, including the chaperones DnaK2 and DnaK3, Thioredoxin (trxA), and the dehydrogenase (ACAD) (Figure 22 I-L).

Our example aimed to subsequently determine any potential relationship that may exist between the proteins identified with specific profiles linked to VaD with potential OM microorganisms and dementia stages, as depicted in Figure 22M. The results of these analyses suggested that some organisms from the OM, such as Rhodobacter capsulatus, might be specifically associated with VaD, while other organisms, including Chlamydia pneumoniae, Kytococcus sedentarius, Klebsiella pneumoniae, and Chronobacter sakazakii, among others, may be linked to both VaD and early AD. However, no clear association was established between VaD and clinical AD at the OM level, as illustrated in Figure 22M. Furthermore, several microorganisms that may be responsible for producing the OMdP in bEVs associated with VaD were identified as specific to the OM and exhibiting commensal or health-associated properties (Figure 22N).

### Presence of OMdP in whole brain proteomes is linked to their levels in bEVs

The analysis of the presence and levels of OMdP in complementary brain tissues was carried out by performing next-generation proteomics of the remaining WB tissues after isolating bEVs. The study revealed that numerous OMdP previously discovered in bEVs were also present in the examined WB excluding bEVs (Figure 23A). In particular, the OMdP in bEVs: AHCY, DnaK, ATPD, Tuf, FHS, and SDR, which were previously identified in bEVs and linked to AD and VaD, were also identified in WB (Figure 23B). Analysis of these proteins in these complementary brain tissues revealed significant differences between dementia groups (AD and VaD) involving four of these proteins, FHS, DnaK, ATPD and Tuf, (Figure 23C-F).

We subsequently undertook an extensive correlation study to determine whether any interaction existed between the levels of OMdP in bEVs and WB by analyzing the proteins commonly found in these brain vesicles and corresponding tissues, excluding bEVs. This study demonstrated that several of the dementia modulated OMdP in bEVs exhibited significantly robust negative correlations between their levels in bEVs and their levels in WB (Figure 23G-J). In particular, significantly associated levels between the brain parenchyma and bEVs were identified in clinical cases of AD involving the OMdP DnaK1, AHCY, and SDR, as illustrated in Figure 23G-I. Additionally, a significant and statistically robust negative correlation was also observed for the OMdP AHCY in cases of VaD (Figure 23J).

### OMdP display docking abilities with key AD-associated proteins

In order to assess the influence of molecular interactions involving OMdP in bEVs on the progression of AD, we examined their capacity to interact with human proteins that are associated with brain dementia. Specifically, we investigated the ability of dementia-modulated OMdP in bEVs to interact with proteins that are traditionally linked to AD neuropathology, such as amyloid precursor protein (APP), prion protein (PrP), tau protein (TAU), and presenilin 1 (PSEN1), as depicted in Figure 24A. In addition, we evaluated the capacity of dementia-modulated OMdP in bEVs to interact with specific human proteins that are related to cognitive decline, by referencing the interaction capabilities demonstrated by their corresponding human homologs. Through this analysis, we identified 30 potential protein interactors that are associated with cognitive-decline related neuropathological factors (as shown in Figure 24A), such as BDNF, Aβ42, APoE, PPIF, and ATPF2, among others. Additional analysis of this subset of proteins indicates that the majority of cognitive decline-associated proteins with homologous OMdP in bEVs are mainly connected to cellular proteostasis, comprising approximately 26% of the overall outcomes.

Moreover, a considerable number of these proteins are associated with cellular structure, accounting for about 13% of the total (Figure 24A). In silico docking simulations were conducted to specifically evaluate the interactions between OMdP in bEVs and proteins associated with AD neuropathology, as well as with cognitive decline (Figure 24B-K). Significant dockings (with confidence scores greater than 0.7)[50, 58] were identified in these experiments involving OMdP in bEVs with several dementia-related proteins, including AD-neuropathological markers such as APP, PrP, TAU, and PSEN1 (Figure 24B-K). The specific OMdP in bEVs, SDR, ATPD, trxA, and DnaK, were found to have the ability to dock with the neurotoxic AD peptide Aβ42 (Figure 24E, G, I, and K). The OMdP in bEVs trxA displayed the capacity to dock with crucial trophic factors, such as the brain-derived neurotrophic factor (BDNF) (Figure 24G). To provide a control, in silico docking simulations with the randomly selected non-interacting protein (chignolin) were also conducted.

Further investigation was carried out to pinpoint the particular areas of the AD-neuropathological markers that interact with the previously identified OMdP in bEVs (Figure 24L-O). Notably, these studies demonstrated that OMdP in bEVs possess the ability to interact with regions of human proteins that are directly involved in proteinopathy and neurotoxicity in AD neuropathology in 95% of the simulated cases involving the proteins APP, TAU, and PrP (Figure 24L-N), whereas in the 75% of the simulated cases involving PSEN1 (Figure 24O).

### OMdP in bEVs fractions associate with AD markers at peptide level

After conducting peptidome-wide correlation analyses, we aimed to determine if any significant relationships existed between OMdP and AD markers within the bEVs fractions of dementia patients. Our findings revealed the presence of notable correlation patterns, which manifested as reversed correlations, affecting both age-matched controls and preclinical AD subjects (Figure 25). The research uncovered that many peptides derived from the OMdP in bEVs DnaK1 and 2 exhibited positive correlations with the AD-associated protein APP in the bEVs of control subjects. Conversely, negative correlations were observed in early AD, as demonstrated in Figure 25A-B. Additionally, these peptides displayed similar patterns when interacting with the AD marker TAU and exhibited a similar tendency to transition from positive to negative correlations when interacting with the protein PrP, as illustrated in Figure 25C-F.

### OMdP in WB fractions associate with AD markers at peptide level

Reversed correlation patterns affecting the remaining WB fractions of subjects in the age-matched control and early AD groups were also identified involving specific peptides of the OMdP in bEVs DnaK2 and 3 and the APP-20 and -21 (Figure 26). These results suggest that the presence of these peptides in whole brain fractions and bEVs is disrupted in preclinical AD compared to controls, and that what is observed in EVs has a direct influence on the presence of these molecules in the remaining WB fractions.

### OMdP differentially circulate in blood EVs of clinical AD subjects

To evaluate and verify the circulation capabilities of the identified OMdP in bEVs in the blood of AD patients, and in order to substantiate the results obtained, we scrutinized publicly available proteomics data from alternative clinical cohorts analyzing blood EVs from AD subjects. These confirmatory experiments revealed the presence of OMdP DnaK and ATPD in the circulating blood plasma EVs of subjects diagnosed with MCI, clinical AD, and age-matched controls, as presented in Table 1. Additional analysis noteworthily uncovered that the concentrations of OMdP DnaK in blood plasma EVs were markedly elevated in individuals with MCI and particularly in those with AD as compared to age-equivalent controls (Table below).

Table. Identification of oral microbiome-derived proteins in blood plasma circulating extracellular vesicles (OMdPpEVs) from age-matched controls, subjects diagnosed with mild cognitive impairment (MCI) and clinical Alzheimer's disease (AD). Quantification of OMdPpEVs is expressed as average ± standard error of the mean (SEM). OMdP in bEVs protein identity was confirmed by the identification of unique peptides. Significant differences were assessed by one-way ANOVA with Bonferroni correction for multiple comparisons. *at MCI indicates significant differences at p ≤ 0.01 compared to Control and significant differences at p ≤ 0.05 compared to AD, ** at AD indicates significant differences at p ≤ 0.01 compared to Control and significant differences at p ≤ 0.05 compared to MCI.

| **Gene symbol** | **Protein description** | **Unique** | **Control** | **MCI** | **AD** |
|---|---|---|---|---|---|
| DnaK | Chaperone protein DnaK | Yes | 50 ± 11 | 65.74 ± 24* | 80.9 ± 17** |
| ATPD | ATP synthase subunit beta | Yes | 2.94 ± 2.1 | 2.89 ± 1.8 | 3.2 ± 1.7 |

## Claims

1. *In vitro* use of an oral microbiome-derived extracellular vesicle associated protein (OMEVP) or a combination of OMEVPs, in the diagnosis or prognosis of mixed dementias, in a biological sample obtained from a human subject selected from the group consisting of saliva, mucus, gum plaque, blood, plasma, urine, tear liquid, cerebrospinal fluid or brain tissue, wherein the OMEVP is the Chaperone protein DnaK, and wherein the Chaperone protein DnaK is selected from any one of:
a. SEQ ID NO 1 to 23 or any fragments thereof, or
b. a sequence having at least 90% identity with any one of SEQ ID NO 1 to 23 or any fragments thereof.

2. The use of an OMEVP according to claim 2, wherein the biological sample obtained from a human subject is selected from the group consisting of saliva, blood, plasma, cerebrospinal fluid or brain tissue.

3. The use of an OMEVP according to any one of claims 1 to 2, wherein the OMEVP is a protein fragment selected from any one of sequences SEQ ID NO 51 to 142 or a sequence having at least 90% identity with any one of SEQ ID NO 51 to 142.

4. The use of an OMEVP according to any one of claims 1 to 3, wherein the OMEVP is a protein fragment selected from any one of sequences SEQ ID NO 51 to 142.

5. The use of an OMEVP according to claim 4, wherein the biological sample obtained from a human subject is selected from the group consisting of saliva, blood, plasma, cerebrospinal fluid or brain tissue.

6. The use of an OMEVP according to any one claims 1 to 5, wherein the mixed dementia is selected from the list consisting of Alzheimer's disease (AD) and vascular dementia (VaD).

7. The use of an OMEVP according to any one of the precedent claims, wherein the OMEVP is combined with a further OMEVP selected from any one of the group consisting of:
a) Adenosylhomocysteinase of SEQ ID NO 24 to 28 or any fragments thereof, in particular fragments of SEQ ID NO 143 to 158 or fragments having at least 90% sequence identity with any one of SEQ ID NO 143 to 158, or a sequence having at least 90% identity with any one of SEQ ID NO 24 to 28,
b) 2-keto-3-deoxy-L-fuconate dehydrogenase of SEQ ID NO 29 to 31 or any fragments thereof, in particular fragments of SEQ ID NO 159 to 161 or fragments having at least 90% sequence identity with any one of SEQ ID NO 159 to 161, or a sequence having at least 90% identity with any one of SEQ ID NO 29 to 31,
c) Formate-tetrahydrofolate ligase of SEQ ID NO 32 or any fragments thereof, in particular fragments of SEQ ID NO 162 to 163 or fragments having at least 90% sequence identity with any one of SEQ ID NO 162 to 163, or a sequence having at least 90% identity with any one of SEQ ID NO 32,
d) Thioredoxin 1 of SEQ ID NO 33 to 39 or any fragments thereof, in particular fragments of SEQ ID NO 164 to 170 or fragments having at least 90% sequence identity with any one of SEQ ID NO 164 to 170, or a sequence having at least 90% identity with any one of SEQ ID NO 33 to 39,
e) Phosphate ABC transporter substrate-binding protein PstS of SEQ ID NO 49 or any fragments thereof, in particular fragments of SEQ ID NO 180 or fragments having at least 90% sequence identity with SEQ ID NO 180, or a sequence having at least 90% identity with SEQ ID NO 49,
f) 2-methylcitrate dehydratase of SEQ ID NO 40 or any fragments thereof, in particular fragments of SEQ ID NO 171 or fragments having at least 90% sequence identity with SEQ ID NO 171, or a sequence having at least 90% identity with SEQ ID NO 40,
g) Acyl-CoA dehydrogenase of SEQ ID NO 41 or any fragments thereof, in particular fragments of SEQ ID NO 172 or fragments having at least 90% sequence identity with SEQ ID NO 172, or a sequence having at least 90% identity with SEQ ID NO 41,
h) Elongation factor Tu of SEQ ID NO 42 to 48 or any fragments thereof, in particular fragments of SEQ ID NO 173 to 179 or fragments having at least 90% sequence identity with any one of SEQ ID NO 173 to 179, or a sequence having at least 90% identity with any one of SEQ ID NO 42 to 48,
i) ATP synthase subunit betaof SEQ ID NO 50 or any fragments thereof, in particular fragments of SEQ ID NO 181 to 186 or fragments having at least 90% sequence identity with any one of SEQ ID NO 181 to 186, or a sequence having at least 90% identity with SEQ ID NO 50, or any combinations thereof, or
any combinations thereof.

8. The use of an OMEVP according to claim 7, wherein the further OMEVP is selected from the group consisting of:
a. fragments of the Adenosylhomocysteinase of SEQ ID NO 143 to 158, or a sequence having at least 90% identity with any one of SEQ ID NO 143 to 158,
b. fragments of the 2-keto-3-deoxy-L-fuconate dehydrogenase of SEQ ID NO 159 to 161, or a sequence having at least 90% identity with any one of SEQ ID NO 159 to 161,
c. fragments of the Formate-tetrahydrofolate ligase of SEQ ID NO 162 to 163, or a sequence having at least 90% identity with any one of SEQ ID NO 162 to 163,
d. fragments of the Thioredoxin 1 of SEQ ID NO 164 to 170, or a sequence having at least 90% identity with any one of SEQ ID NO 164 to 170,
e. fragments of the Phosphate-binding protein of SEQ ID NO 180, or a sequence having at least 90% identity with SEQ ID NO 180,
f. fragments of the 2-methylcitrate dehydratase of SEQ ID NO 171, or a sequence having at least 90% identity with SEQ ID NO 171,
g. fragments of the Acyl-CoA dehydrogenase of SEQ ID NO 172, or a sequence having at least 90% identity with SEQ ID NO 172,
h. fragments of the Elongation factor Tu of SEQ ID NO 173 to 179, or a sequence having at least 90% identity with any one of SEQ ID NO 173 to 179 and
i. fragments of the ATP synthase subunit beta of SEQ ID NO 181 to 186, or a sequence having at least 90% identity with any one of SEQ ID NO 181 to 186, or any combinations thereof.

9. The use of an OMEVP according to claim 7, wherein the OMEVP of any one of claims 1 to 6 is combined with the following OMEVPs: Adenosylhomocysteinase of SEQ ID NO 24 to 28 or any fragments thereof as defined in claim 8 for this protein, or a sequence having at least 90% identity with any one of SEQ ID NO 24 to 28, Formate-tetrahydrofolate ligase of SEQ ID NO 32 or any fragments thereof as defined in claim 8 for this protein, or a sequence having at least 90% identity with SEQ ID NO 32 and Thioredoxin 1 of SEQ ID NO 33 to 39 or any fragments thereof as defined in claim 8 for this protein, or a sequence having at least 90% identity with any one of SEQ ID NO 33 to 39.

10. The use of an OMEVP according to claim 7, wherein the OMEVP of any one of claims 1 to 6 is combined with the following OMEVPs: 2-keto-3-deoxy-L-fuconate dehydrogenase of SEQ ID NO 29 to 31 or any fragments thereof as defined in claim 8 for this protein, or a sequence having at least 90% identity with any one of SEQ ID NO 29 to 31 and Formate-tetrahydrofolate ligasein of SEQ ID NO 32 or any fragments thereof as defined in claim 8 for this protein, or a sequence having at least 90% identity with SEQ ID NO 32.

11. The use of an OMEVP according to claim 7, wherein the OMEVP of any one of claims 1 to 6 is combined with the following OMEVPs: Adenosylhomocysteinase of SEQ ID NO 24 to 28 or any fragments thereof as defined in claim 8 for this protein, or a sequence having at least 90% identity with any one of SEQ ID NO 24 to 28 and Formate-tetrahydrofolate ligase of SEQ ID NO 32 or any fragments thereof as defined in claim 8 for this protein, or a sequence having at least 90% identity with SEQ ID NO 32.

12. The use of an OMEVP according to claim 7, wherein the OMEVP of any one of claims 1 to 6 is combined with the following OMEVP: 2-keto-3-deoxy-L-fuconate dehydrogenasein of SEQ ID NO 29 to 31 or any fragments thereof as defined in claim 8 for this protein, or a sequence having at least 90% identity with any one of SEQ ID NO 29 to 31.

13. The use of an OMEVP according to claim 7, wherein the OMEVP of any one of claims 1 to 6 is combined with the following OMEVP: Adenosylhomocysteinasein of SEQ ID NO 24 to 28 or any fragments thereof as defined in claim 8 for this protein, or a sequence having at least 90% identity with any one of SEQ ID NO 24 to 28.

14. The use of an OMEVP according to claim 7, wherein the OMEVP of any one of claims 1 to 6 is combined with the following OMEVPs ATP synthase subunit beta of SEQ ID NO 50 or any fragments thereof as defined in claim 8 for this protein, or a sequence having at least 90% identity with SEQ ID NO 50.
